# EUROPEAN PATENT SPECIFICATION

(11) **EP 2 841 577 B1**
(45) Date of publication and mention of the grant of the patent: **02.01.2019**
(21) Application number: 13718839.7
(22) Date of filing: 25.04.2013
(51) Int. Cl.: C12N 15/11, A61K 31/713

(54) **HIGH COMPLEXITY SIRNA POOLS**
SIRNA-POOLS VON HOHER KOMPLEXITÄT
GROUPES D'ARNSI DE HAUTE COMPLEXITÉ

(30) Priority: 26.04.2012 GB 201207291; 26.04.2012 EP 12165702; 26.10.2012 EP 12190148
(43) Date of publication of application: 04.03.2015
(73) Proprietor: Intana Bioscience GmbH, 82152 Planegg-Martinsried (DE); Universität Regensburg, 93053 Regensburg (DE)
(72) Inventor: MEISTER, Gunter, 93128 Regenstauf (DE); HANNUS, Michael, 01099 Dresden (DE)
(74) Representative: Bühler, Dirk
(86) International application number: PCT/EP2013/058603
(87) International publication number: WO 2013/160393

(56) References cited:
- WO-A1-2004/106517
- WO-A2-2004/035765
- WO-A2-2004/046320
- US-A1- 2005 260 754
- WANG SHUNQING ET AL: "Development and validation of vectors containing multiple siRNA expression cassettes for maximizing the efficiency of gene silencing", BMC BIOTECHNOLOGY, BIOMED CENTRAL LTD. LONDON, GB, vol. 6, no. 1, 22 December 2006 (2006-12-22), page 50, XP021023586, ISSN: 1472-6750, DOI: 10.1186/1472-6750-6-50
- POLISENO ET AL: "370. Multi-Copy, Multi-siRNA Vectors as Versatile Tools for Multiple Gene Knock-Down Applications", MOLECULAR THERAPY, NATURE PUBLISHING GROUP, GB, vol. 11, 15 August 2005 (2005-08-15), page 144, XP005015709, ISSN: 1525-0016

## Description

### Field of the invention

The present invention relates to a method for producing pools of siRNA molecules suitable for RNA interference. The methods rely on *in vitro* transcription and hybridization of template molecules to generate annealed RNA molecules which comprise double stranded sections defining at least part of the siRNA sequences and a single stranded loop sequence being capable of being recognised, cleaved and digested by an RNase. The present invention further relates to nucleic acid molecules and kits thereof wherein the nucleic acid molecules comprise DNA molecules which can be used in the afore described methods in accordance with the invention.

### Background

RNA interference (RNAi) is a powerful tool for performing loss of function studies in diverse organisms by transiently shutting of gene expression. Various methods have been developed to allow for efficient RNAi.

One of the common approaches is to use small inhibitory (siRNA) molecules which are typically double-stranded RNA molecules of a length of 15 to 30 nucleotides. The sequences of such siRNA molecules are selected so that they match sequences of the mRNA to be silenced by RNAi. The siRNAs are then brought into contact with the organism or cell for which gene silencing studies are to be performed. The siRNA molecules are integrated into the RISC complex a complex enzymatic machinery involving the so-called Ago proteins mediating the separation of the double-stranded siRNA molecules and effecting the hybridization of single stranded siRNA molecules with the target mRNA ultimately leading to the desired transient silencing of the respective mRNA, i.e. gene that is targeted by the siRNAs.

RNAi and siRNAs can be used in a versatile manner. For example, siRNA libraries may be designed to perform screens for loss of function studies addressing not only one, but numerous and, in principle, all genes of a particular cell or organism.

Some of the drawbacks of RNAi and siRNAs, which have been increasingly recognised in recent years, are so-called off-target-effects and efficacy as well as problems relating to the manufacturing of pools of siRNAs, which either allow for silencing of the expression of various genes at the same time, or a single gene by using a multitude of siRNAs being specific for that specific target gene.

In principle, the selectivity of RNAi can be addressed by properly selecting siRNAs. For example, one may select the sequence of an siRNA that determines which sequence of the gene to be silenced will be recognised such that a sequence is selected that should in principle be unique to the target gene and not be found in other sequences. By properly selecting such siRNA sequences, it should be possible to ensure that only the gene of interest is silenced. However, even though it should in principle be possible to select target siRNA sequences such that no other target sequences are recognised by the siRNAs with the consequence that no off-target-effects should occur, such siRNAs are not necessarily effective to the desired degree.

Effectiveness is determined *inter alia* by the fact that a target sequence may not easily be accessible in the *in vivo* situation to the siRNA due to interaction with proteins within a cell or the fact that the sequence of the target genes may adopt confirmations that render them non- or at least partially accessible to the siRNA. Due to these facts, an siRNA sequence which according to common selection procedures should not provide any off-target effects, may not prove effective or may also impact the expression of other genes. In view of the aforementioned problems, it may be necessary to design siRNAs with different sequences and to use them simultaneously in order to silence the expression of a single gene. For reasons not understood using numerous siRNA sequences against the same target gene, the danger of off-target effects may be reduced, perhaps by increasing the signal to nose ratio for specific siRNAs over non-specific or non-effective siRNAs.

Manufacturing of siRNAs by e.g. solid phase chemistry can be rather time and cost consuming. Particularly if one wants to produce complex pools of siRNAs either of siRNAs being directed to the same target gene or siRNA pools recognising different target genes can thus become prohibitive from a cost perspective.

Nevertheless such pools of siRNA sequences are of high interest because, as mentioned before, they allow efficient silencing of the expression of a single gene as then not each and every siRNA has to be tested stepwise. Rather, one can quite straigthforwardly silence a gene by using such a pool or one can even silence numerous genes at the same time.

WO 2004/035765 A2 describes double stranded RNA and DNA structures for generating siRNAs and methods of utilizing them for silencing genes.

WO 2004/106517 A1 describes a ribonucleic acid (RNA) for use as interfering RNA in gene silencing techniques to silence a target gene comprising in a 5' to 3' direction at least a first effector sequence, a second effector sequence, a sequence substantially complementary to the second effector sequence and a sequence substantially complementary to the first effector sequence, wherein the complementary sequences are capable of forming double stranded regions with their respective effector sequences and wherein at least one of these sequences is substantially identical to the predicted transcript of a region of the target gene, and a nucleic acid construct encoding such an RNA.

WO 2004/046320 A2 describes methods and compositions involving the production or generation of siRNA mixtures or pools capable of triggering RNA-mediated interference (RNAi) in a cell, wherein these mixtures and pools involve processing by Rnase III.

US 2005/0260754 A1 relates to constructs and methods for regulating the gene expression of at least two endogenous target genes by introducing, into a eukaryotic cell or a eukaryotic organism, an at least partially double-stranded ribonucleic acid molecule, the ribonucleic acid molecule comprising at least two ribonucleotide sequence segments which are homologous to various genes of the eukaryotic cell.

In the light of this background, there is thus continuing interest in methods that allow for provision of pools of siRNAs that allow silencing of either expression of a single gene and/or that allow silencing of expression of numerous genes at the same time. Furthermore, there is a continuing interest in providing new methods for efficiently producing siRNAs and in particular the aforementioned siRNA pools. It is *inter alia* these problems that the present invention addresses.

### Objectives and summary of the present invention

It is one objective of the present invention to provide efficient methods for producing siRNAs. It is in particular an objective of the present invention to provide methods that allow manufacturing of siRNA pools which can either be used to silence the expression of single genes and/or the expression of various genes at the same time. Furthermore, it is an objective of the present invention not only to provide methods but also tools in the form of nucleic acid molecules and kits that can be used for methods of manufacturing siRNAs and in particular pools of siRNAs either for the silencing of a single genes and/or simultaneous silencing of various genes. It is another objective of the present invention to provide pools of siRNAs, which can be used to selectively silence gene expression at reduced off-target effects.

These and other objectives as they will become apparent from the ensuing description are attained by the subject matter of the independent claims. The dependent claims relate to some of the preferred embodiments of the present invention.

The invention as described herein starts from the finding that a new enzymatic approach can be used to produce defined, complex pools of short interfering RNAs (siRNAs) for the gene specific inhibition of gene expression *in vitro* and *in vivo.* In the first step of the method, two partly complementary single strand RNAs are generated by in vitro transcription from custom DNA templates. Hybridization of the two single strand RNAs gives rise to a double strand RNA molecule composed of alternating base pairing and non base pairing sections. Using the single strand specific ribonuclease Rnase T1, the non base pairing loop sections are degraded, cleaving the long double strand RNA precursor into a mixture of short double strand RNA molecules corresponding to the base pairing sections of the precursor molecule.

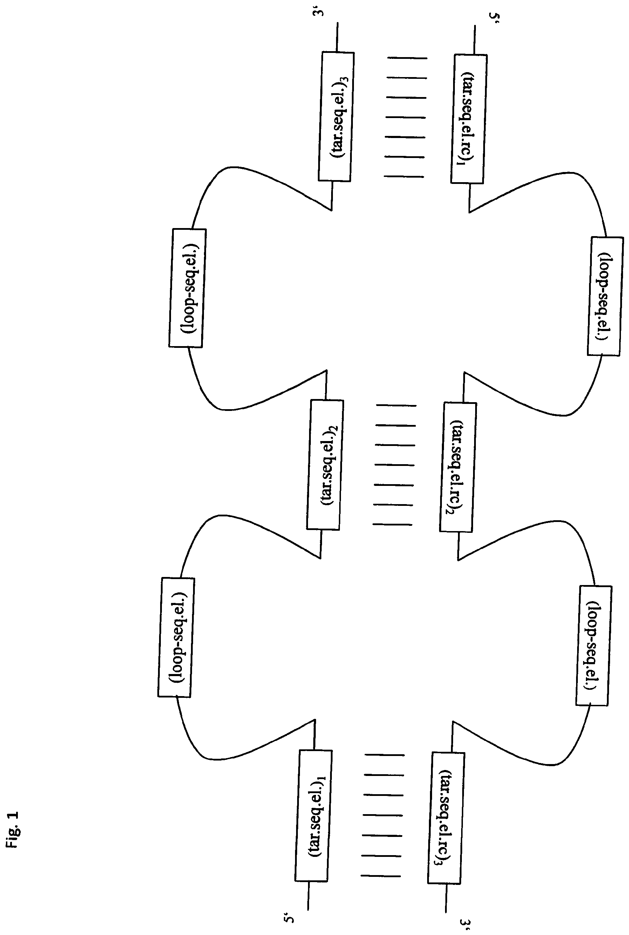

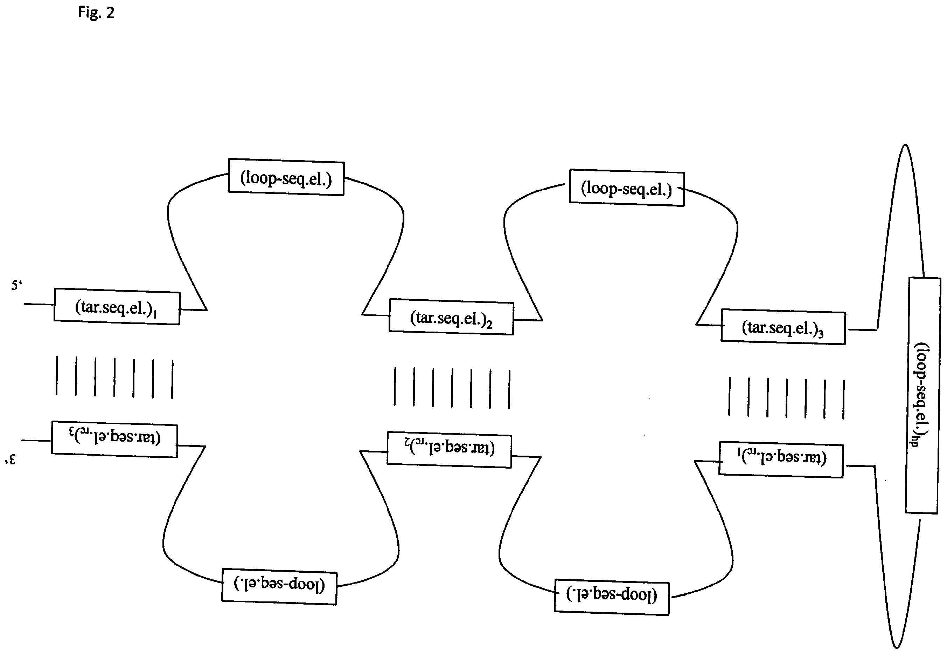

The inventors of the present invention thus have found that it is possible to produce siRNAs by designing and providing template nucleic acid molecules that upon transcription, hybridization and digestion with RNAse T1 can be used to produce the same siRNAs or pools of different siRNAs which may be directed to the same target genes and/or pools of siRNAs which are directed to different target genes. To this end, the present invention uses template molecules and preferably DNA molecules which upon transcription and hybridization, preferably *in vitro* transcription and *in vitro* hybridization, yield hybridized RNA molecules as depicted in Figures 1 and 2.

The resulting hybridized RNA molecules are characterised by sections of double-stranded RNA comprising at least part of the sequences of the final siRNA molecules and single-stranded loop sequences which can be recognised, cleaved and digested by RNAse T1 the loop-sequence-element having the sequence 5'-(X)ₘ-G-(Y)ₙ-G-3', with X being A and m being an integer of 1, with Y being A or T and n being an integer of 1, 2, or 3, and with X and Y being selected such that there is no hybridization of two molecules consisting just of the loop-sequence-element and wherein the loop-sequence element does not match a sense or antisense sequence in said at least one target gene of RNA interference. It is immediately evident to a skilled person that by manufacturing template molecules which upon transcription and hybridization will lead to RNA molecules as depicted in Figures 1 and 2, one can produce either siRNAs of the same sequence, pools of siRNAs of different sequences, which can be used to silence expression of a single target gene and/or pools of siRNA molecules of different sequences which can be used to silence the expression of numerous genes, such as e.g. genes defining pathways. The pools of siRNAs, which may de designated as siRNA pools, may also be used for genome wide screens of genes. Such high complexity pools can also be used for treating a disease in a human or animal being. They may also be used to silence the expression of numerous genes, such as e.g. genes defining pathways. The pools of siRNAs, which may de designated as siRNA pools, may also be used for genome wide screens of genes.
As the template DNA molecules may be preferably made from DNA, these template molecules can be integrated into common vector systems and thus serve as a blueprint and storage device of such template molecules. However, manufacturing of template molecules, which may preferably be made of DNA molecules, is far more economical, both in terms of cost and time than direct synthesis of specific siRNA molecules. Once such a template molecule, which may preferably be a DNA molecule has been made and cloned in a vector, it can be stored, propagated and then *in vitro* transcribed and hybridized, once there is a need for a new batch of siRNA molecules.
By selecting and adjusting the length of the sections in the template molecules which will ultimately correspond to the siRNA molecules, it is possible to produce siRNAs of a length as they are commonly used for RNAi, i.e. typically between 15 to 30 nucleotides. It is thus possible to produce siRNA molecules by selecting the length of the sequences in the template molecule which will correspond to the siRNA molecules that e.g. have already been found to be effective for certain genes in certain organisms. For the case of mammalian organisms and cells, one can thus produce siRNAs e.g. of a length of 21, 22 and 23 nucleotides.
The present invention is illustrated with respect to a template molecule where overall 14 different siRNA sequences, all of which were directed to the same target gene, namely AUKRB, were incorporated into a template molecule, for which the sequences giving rise to the single-stranded loop sequences, were selected to be cleavable and digestible by RNase T1. It is furthermore demonstrated that the resulting pool of 14 siRNA sequences, all of which had a length of 21 nucleotides and a 3' overhang of 2 nucleotides was efficient in silencing the expression of AUKRB.

The present disclosure furthermore illustrates that complex pools of 15 and 60 siRNAs, which were obtained using the methods described herein, provide better on-target effects for Scyll or PolG than established siRNAs and esiRNA pools against these genes. Further, such complex siRNA pools effectively avoid off-target effects as is shown in comparision to siRNAs against PolG and Scyll, which are known to give off-target effects for Mad2, and in comparison to so called smart pools. It seems reasonable to assume in view of the data presented that the reduced off-target effects result from low concentration of siRNAs, which may be responsible for the off-targer effects, in the pools. Interestingly, the pools were shown in a genome wide analysis to not lead to substantial off-target effects on other genes.

The person skilled in the art will of course immediately realise that this concept can be transferred to template molecules for which the siRNA sequences are selected such that different genes other than AUKRB, PolG or Scyl 1 are silenced.

The data presented hereinafter further show for RNase T1, according to which considerations loop sequences may be selected to allow for efficient cleavage by RNAse T1. In case of RNAse T1, efficient cleavage is achieved by loop sequences of 4, 5, or 6 nucleotides in length.
The present invention in a first aspect relates to a method of preparing double stranded RNA molecules as defined by claim 1, wherein each strand of said different double stranded molecules has a length of 15 to 30 nucleotides wherein said different double stranded RNA molecules are capable of target-specific RNA interference of at least one target gene, said method comprising at least the steps of:
a. Providing at least one first DNA molecule,
b. Providing at least one second DNA molecule,
c. *In vitro* transcribing said at least one first and at least one second DNA molecules using an RNA polymerase to obtain corresponding at least one first and at least one second RNA molecules,
d. Hybridizing said at least one first and at least one second RNA molecules of step c. to obtain an double stranded RNA molecule of the general structure depicted in Fig. 1,
e. Digesting the double stranded RNA molecule obtained in step d. with RNAse T1 which is capable of preferentially recognizing, cleaving and digesting the single stranded loop-sequence-elements over the hybridized double stranded sections of the double stranded RNA obtained in step d. thereby removing single stranded RNA loops,
wherein the sequence of said target-sequence-elements depicted on Fig. 1 of the at least one first DNA molecule is sense to sequences of said at least one target gene of RNA interference, wherein the sequences of said target-sequence-elementsrc of the at least one second DNA molecule are the reverse complements of the sequences of the target-sequence-elements of the at least one first DNA molecule, which they hybridize to, and wherein the loop-sequence elements of the at least one first and at least one second DNA molecules are not reverse complements of each other,
wherein the sequences of the loop-sequence-elements are selected such that double stranded RNA molecules are obtained by recognition, cleavage and digestion of the single stranded loop-sequence elements by RNase T1 in step e., wherein each strand of said resulting double stranded molecules has a length of 15 to 30 nucleotides and wherein said resulting double stranded RNA molecules are capable of target-specific RNA interference of at least one target gene.

The present invention thus relates to a method comprising at least the steps of:
a. Providing at least one first DNA molecule comprising in the 5'-3' direction as repeating units a nucleic acid sequence with the following elements:
   5' - [(target-sequence-element) - (loop- sequence-element)]ₖ-3', with k being an integer >1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
   with X being A and m being an integer of 1,
   with Y being A or T and n being an integer of 1, 2, or 3, and
b. with X and Y being selected such that there is no hybridization of two molecules consisting just of the loop-sequence-element and wherein the loop-sequence element does not match a sense or antisense sequence in said at least one target gene of RNA interference, Providing at least one second DNA molecule comprising in the 5'-3' direction in a repetitive manner a nucleic acid sequence with the following elements:
   5' - [(target-sequence-element)rc - (loop-sequence-element)]i-3',
   with 1 being an integer >1 and having the same value as k in the first DNA molecule,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference, wherein the target-sequence-elementsrc counted from the 3' end in the repeating units of said second DNA molecule are the respective reverse complement of the target-sequence-elements counted from the 5' end in the repeating units of said first DNA molecule, and
   wherein the loop-sequence-elements in the repeating units of said second DNA molecule are not reverse complements of the loop-sequence-elements in the repeating units of said first DNA molecule,
c. *in vitro* transcribing said at least one first and at least one second DNA molecules using an RNA polymerase to obtain corresponding at least one first and at least one second RNA molecules,
d. Hybridizing said at least one first and at least one second RNA molecules of step c. to obtain a double stranded RNA molecule of the general structure depicted in Fig. 1,
e. Digesting the double stranded RNA molecule obtained in step d. with RNAse T1, which is capable of preferentially recognizing, cleaving and digesting the single stranded loop-sequence-elements over the hybridized double stranded sections of the double stranded RNA obtained in step d. thereby removing single stranded RNA loops,
wherein the sequences of the loop-sequence-elements are selected such that double stranded RNA molecules are obtained by recognition, cleavage and digestion of the single stranded loop-sequence elements by an RNase in step e., wherein each strand of said resulting double stranded molecules has a length of 15 to 30 nucleotides, wherein said resulting double stranded RNA molecules are capable of target-specific RNA interference of at least one target gene.

In a second aspect the present invention relates to a method of preparing different double stranded RNA molecules according to claim 2, wherein each strand of said double stranded molecules has a length of 15 to 30 nucleotides, wherein said double stranded RNA molecules are capable of target-specific RNA interference of at least one target gene, said method comprising at least the steps of:
a. Providing at least one DNA molecule,
b. *In vitro* transcribing said at least one DNA molecules using an RNA polymerase to obtain corresponding at least one first RNA molecule, which upon hybridization provides the general structure depicted in Fig. 2,
c. Digesting the RNA molecule obtained in step b. with Rnase T1, which is capable of of preferentially recognizing, cleaving and digesting the single stranded loop-sequence-elements over the hybridized double stranded sections of the double stranded RNA obtained in step b. thereby removing single stranded RNA loops,
wherein the sequence of said target-sequence-elements depicted on Fig. 2 of the at least one first DNA molecule is sense to sequences of said at least one target gene of RNA interference, wherein the sequences of said target-sequence-elementsrc of the at least one DNA molecule are the reverse complements of the sequences of the target-sequence-elements of the at least one first DNA molecule, which they hybridize to, and wherein the loop-sequence elements of the at least one first and at least one second DNA molecules are not reverse complements of each other,
wherein the sequences of the loop-sequence-elements are selected such that double stranded RNA molecules are obtained by recognition, cleavage and digestion of the single stranded loop-sequence elements by an RNase in step c., wherein each strand of said resulting double stranded molecules has a length of 15 to 30 nucleotides and wherein said resulting double stranded RNA molecules are capable of target-specific RNA interference of at least one target gene.

The present invention thus also relates to a method comprising at least the steps of:
a. Providing at least one first DNA molecule comprising in the 5'-3' direction as repeating units a nucleic acid sequence with the following elements:
   5' - [(target-sequence-element) - (loop- sequence-element)]ₖ - (target-sequence-element) - (loop- sequence-element)ₕₚ - [(target-sequence-element)_{rc} - (loop-sequence-element)]i-3',
   with k being an integer >1,
   with 1 being an integer >1 and being the same as 1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,,
   wherein the (loop sequence element)ₕₚ is of sufficient length to allow for a hairpin structure enabling a self-hybdrization pattern depicted in Fig. 2, with the target-sequence-elementrc being a continuous sequence of 15 to 30 desoxyribonucleotides,
   wherein the target-sequence-elementsrc counted from the 3' end are the respective reverse complement of the target-sequence-elements counted from the 5' end,
   wherein the loop-sequence-elements following the (loop sequence element)ₕₚ are not reverse complements of the loop-sequence-elements preceeding the in the repeating units of said second DNA molecule,
b. *In vitro* transcribing said at least one first DNA molecules using an RNA polymerase to obtain corresponding at least one first RNA molecule, which upon hybridization provides the general structure depicted in Fig. 2,
c. Digesting the double stranded RNA molecule obtained in step b. with RNAse T1, which is capable of preferentially recognizing, cleaving and digesting the single stranded loop-sequence-elements over the hybridized double stranded sections of the double stranded RNA obtained in step b. thereby removing single stranded RNA loops,
wherein the sequences of the loop-sequence-elements are selected such that double stranded RNA molecules are obtained by recognition, cleavage and digestion of the single stranded loop-sequence elements by an RNase in step c., wherein each strand of said resulting double stranded molecules has a length of 15 to 30 nucleotides and wherein said resulting double stranded RNA molecules are capable of target-specific RNA interference of at least one target gene.

In a third aspect, the present invention relates to a combination or a kit of at least two DNA molecules, which upon in vitro transcription, hybridization and digestion with RNAse T1 by the method of claim 1 are capable of providing double stranded RNA molecules,wherein each strand of said different double stranded molecules has a length of 15 to 30 nucleotides and wherein said double stranded RNA molecules are capable of target-specific RNA interference of at least one target gene, wherein said at least two DNA molecules have the sequence elements necessary to obtain an RNA molecule of the general structure depicted in Fig. 1 after in vitro transcription and hybridization,
wherein the sequences of the loop-sequence-elements are selected such that double stranded RNA molecules can be obtained after cleavage and digestion with RNAse T1, which is capable of preferentially recognizing and cleaving the single stranded loop-sequence-elements over the hybridized double stranded sections of the double stranded RNA thereby removing single stranded RNA loops.

The present invention thus relates to a combination or a kit, obtainable by
a. at least one first DNA molecule comprising in the 5'-3' direction as repeating units a nucleic acid sequence with the following elements:
   5' - [(target-sequence-element) - (loop- sequence-element)]ₖ-3', with k being an integer >1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,
b. at least one second DNA molecule comprising in the 5'-3' direction in a repetitive manner a nucleic acid sequence with the following elements:
   5' - [(target-sequence-element)_{rc} - (loop-sequence-element)]ₗ-3',
   with l being an integer >1 and having the same value as k in the first DNA molecule,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,
   wherein the target-sequence-elementsrc counted from the 3' end in the repeating units of said second DNA molecule are the respective reverse complement of the target-sequence-elements counted from the 5' end in the repeating units of said first DNA molecule, and wherein the loop-sequence-elements in the repeating units of said second DNA molecule are not reverse complements of the loop-sequence-elements in the repeating units of said first DNA molecule,
   wherein said at least one first and second DNA molecules can be in vitro transcribed and hybridized to obtain a double stranded RNA molecule of the general structure depicted in Fig. 1,
   wherein the sequences of the loop-sequence-elements are selected such that double stranded RNA molecules can be obtained by recognition, cleavage and digestion of the single stranded loop-sequence elements by RNAse T1, which is capable of preferentially recognizing, cleaving and digesting the single stranded loop-sequence-elements over the hybridized double stranded sections of the double stranded RNA thereby removing single stranded RNA loops.

In a fourth aspect, the present invention relates to a combination or a kit of at least one DNA molecule, which upon in vitro transcription, hybridization and digestion with RNAse T1, which is capable of providing double stranded RNA molecules, wherein each strand of said double stranded molecules has a length of 15 to 30 nucleotides and wherein said different double stranded RNA molecules are capable of target-specific RNA interference of at least one target gene,
wherein said at least one DNA molecule has the sequence elements necessary to obtain an RNA molecule of the general structure depicted in Fig. 2 after in vitro transciption and hybridization,
wherein the loop-sequence-element have the sequence 5'-(X)ₘ-G-(Y)ₙ-G-3',
   with X being A and m being an integer of 1,
   with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference, such that double stranded RNA molecules are obtained after digestion with RNAse, which is capable of preferentially recognizing, cleaving and digesting the single stranded loop-sequence-elements over the hybridized double stranded sections of the double stranded RNA thereby removing single stranded RNA loops.

The present invention thus also relates to a combination or a kit of at least one DNA molecule, obtainable by:
a. Providing at least one first DNA molecule comprising in the 5'-3' direction as repeating units a nucleic acid sequence with the following elements:
   5' - [(target-sequence-element) - (loop- sequence-element)]ₖ - (target-sequence-element) - (loop- sequence-element)ₕₚ - [(target-sequence-element)_{rc} - (loop-sequence-element)]i-3',
   with k being an integer >1,
   with 1 being an integer >1 and being the same as 1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,,
   wherein the (loop sequence element)ₕₚ is of sufficient length to allow for a hairpin structure enabling a self-hybdrization pattern depicted in Fig. 2, with the target-sequence-elementrc being a continuous sequence of 15 to 30 desoxyribonucleotides,
   wherein the target-sequence-elementsrc counted from the 3' end are the respective reverse complement of the target-sequence-elements counted from the 5' end,
   wherein the loop-sequence-elements following the (loop sequence element)ₕₚ are not reverse complements of the loop-sequence-elements preceeding the in the repeating units of said second DNA molecule,
b. *In vitro* transcribing said at least one first DNA molecules using an RNA polymerase to obtain corresponding at least one first RNA molecule, which upon hybridization provides the general structure depicted in Fig. 2,
c. Digesting the double stranded RNA molecule obtained in step b. with RNAse T1, which is capable of preferentially recognizing, cleaving and digesting the single stranded loop-sequence-elements over the hybridized double stranded sections of the double stranded RNA obtained in step b. thereby removing single stranded RNA loops,
wherein the sequences of the loop-sequence-elements are selected such that double stranded RNA molecules are obtained by recognition, cleavage and digestion of the single stranded loop-sequence elements by an RNase in step c., wherein each strand of said resulting double stranded molecules has a length of 15 to 30 nucleotides and wherein said resulting double stranded RNA molecules are capable of target-specific RNA interference of at least one target.

Such combinations, kits and DNA molecules may be provided with
optionally an RNA polymerase,
optionally a buffer for in vitro transcription,
optionally a buffer for hybridization,
optionally an RNase, and
optionally written instructions.

In a fifth aspect the present invention relates to the use of any method as claimed herein, any kit as claimed herein or any template molecule as claimed for producing siRNA pools.

### Figure Legends

- Fig. 1: depicts schematically hybridized RNA molecules for use in methods in accordance with the invention, "targ.seq.el" stands for target sequence element, "loop.seq.el." stands for loop sequence element.
- Fig. 2: depicts schematically a self-hybridized RNA molecule for use in methods in accordance with the invention, "targ.seq.el" stands for target sequence element, "loop.seq.el." stands for loop sequence element. "rc" stands for reverse complement, "(loop.seq.el.)ₕₚ" stands for hairpin loop sequence element.

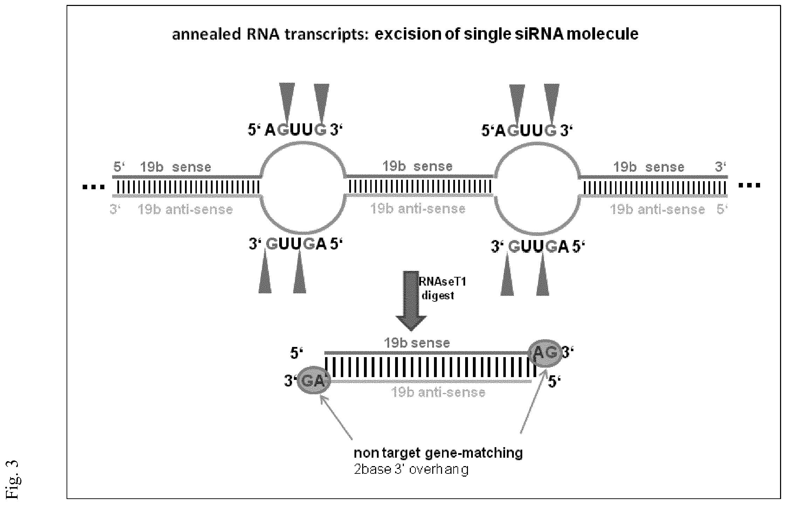

- Fig. 3: depicts schematically two annealed RNA molecules as used in Example 1 and the effects of an RNase T1 digest. (top): target gene specific, base-pairing 19base sense and antisense sequences. Base-pairing highlighted by vertical bars ("IIIIIII"). Non base-pairing, non gene-specific constant loop sequence indicated as curved line. 5 base loop sequence indicated above or below loop with Guanine positions, accessible to RNAseT1 highlighted by arrows. Points of RNAseT1 cleavage 3' to accessible Guanines indicated by arrows. (bottom): 19b sense and antisense sequence remain unaffected by RNAseT1 cleavage. Cleavage by Rnase T1 leads to a 3' overhang of AG resulting in a total of 21 base length for sense and antisense strand. The 3' overhang AG bases are constant and do not have to match the target gene sequence.

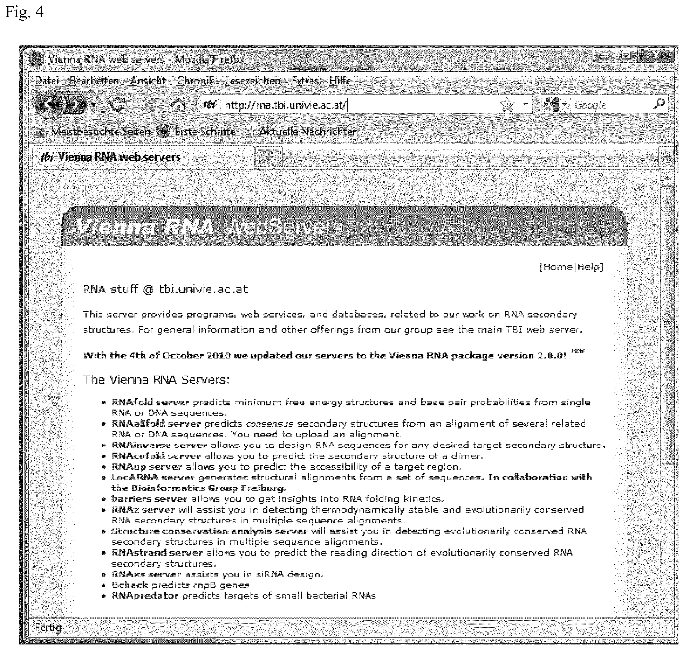

- Fig. 4: depicts the homepage and available software suites of the Vienna RNA WebServers

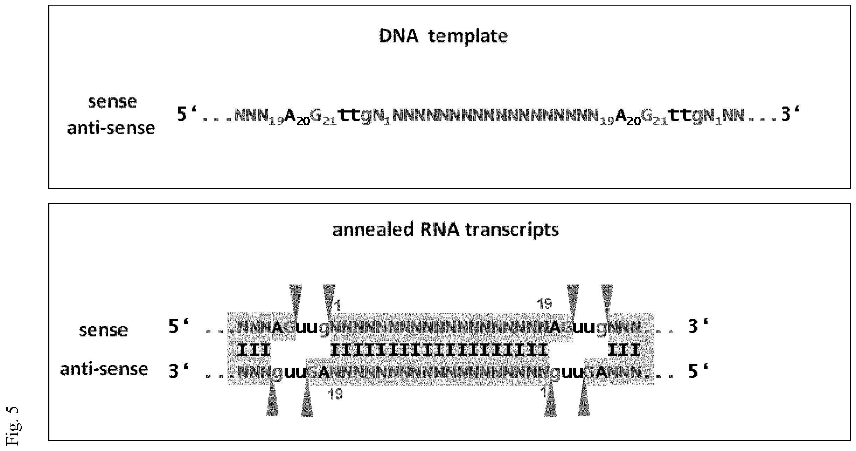

- Fig. 5: Upper box: Scheme of DNA template for both sense and antisense strand as used in example 1. 19 gene matching, base pairing nucleotides (N₁ to N₁₉) of siRNA sequence in indicated position in siRNA. 5 base loop sequence AGTTG. Excised loop sequence (ttg) in lower case. Lower box: annealing of sense and antisense strand transcribed from template as indicated in upper box. Arrowheads indicate positions of RNAseT1 cleavage, 3' of non base pairing G nucleotides. Base-pairing nucleotides indicated by "I" between sense and antisense strand. Mature siRNA after RNAseT1 digest highlighted by blue background color.

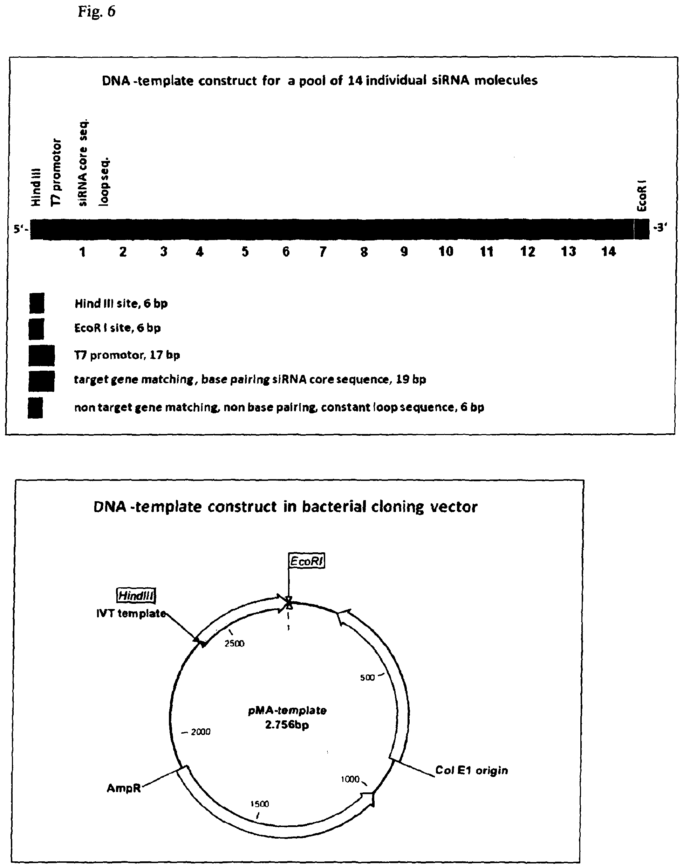

- Fig. 6: Upper panel depicts schematically overall structure of template used for producing pools of 14 siRNAs as described in Example 1 and 2. Lower panel shows template cassette inserted into pMA bacterial cloning vector by EcoRI and HindIII restriction sites as obtained from DNA template provider (e.g. Geneart)

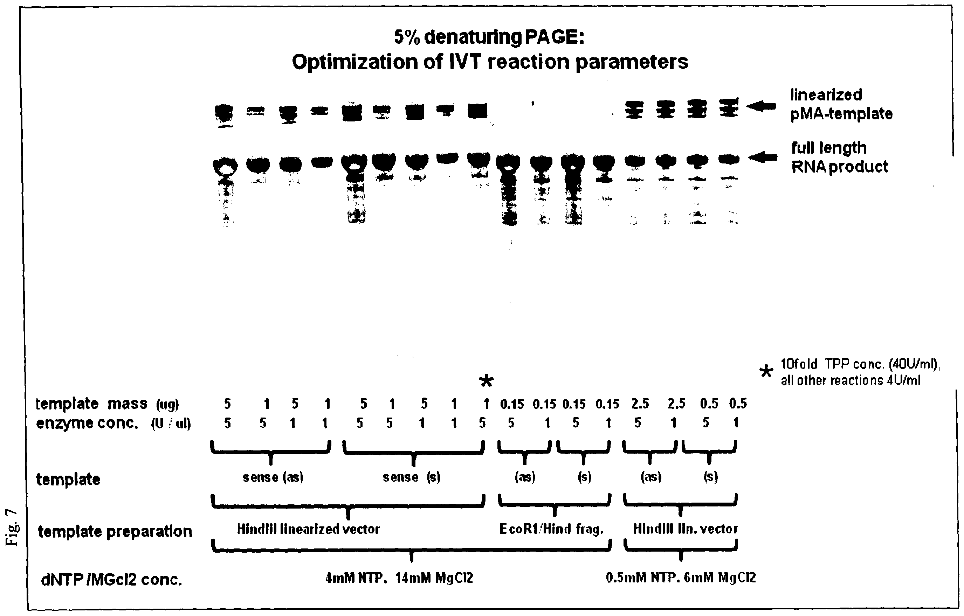

- Fig. 7: depicts optimization of in vitro transcription (IVT)of template of Example 1. Each lane of the 5% denaturing PAA gel loaded with 5 µl sample from one IVT reaction with 100 µl (lane 1-9) and 50ul (lane 10-17) total volume. Parameters varied between reactions as indicated below the gel image were: template mass (0.15-5 µg), enzyme concentration (1 or 5units RNA pol T7), template (sense or antisense), template preparation (Hind III linearized vector, HindII-EcoRI excised template fragment), dNTP (4 mM vs 0.5 mM) and MgCl₂ concentration (14 mM vs 6 mM). T7 RNA polymerase enzyme, 10x reaction buffer and thermostable pyrophosphatase from NEB.

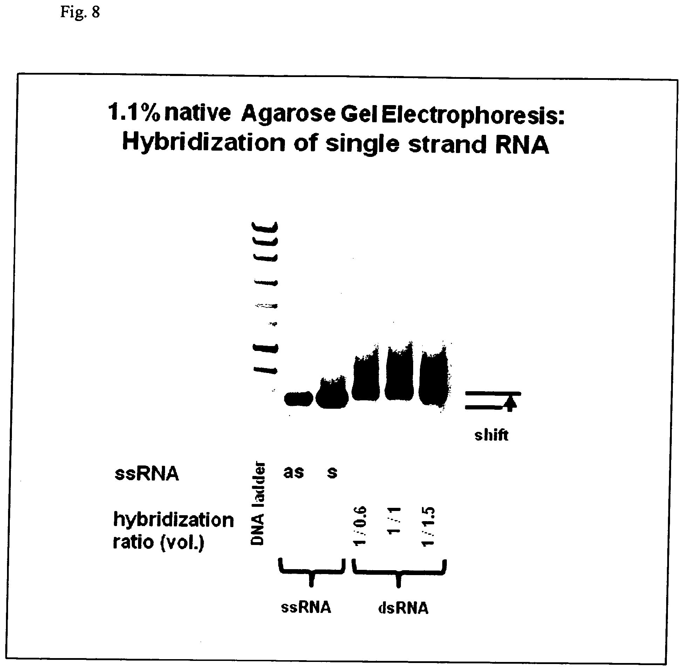

- Fig.8: depicts efficiency of annealing step of single stranded RNA of Example 1. 1 µl per lane of single strand RNA (lane 2 and 3) or hybridization reaction (lane 4 to 6) loaded on 1.1% native agarose gel. Sense (lane 3) and antisense (lane 2) single strand RNA were hybridized in 3 ratios (antisense/sense): 1/0.6, 1/1, 1/1.5. Main band of all hybridization reactions show shift to higher molecular weight as compared to single strand RNA, indicative for formation of double strand RNA.

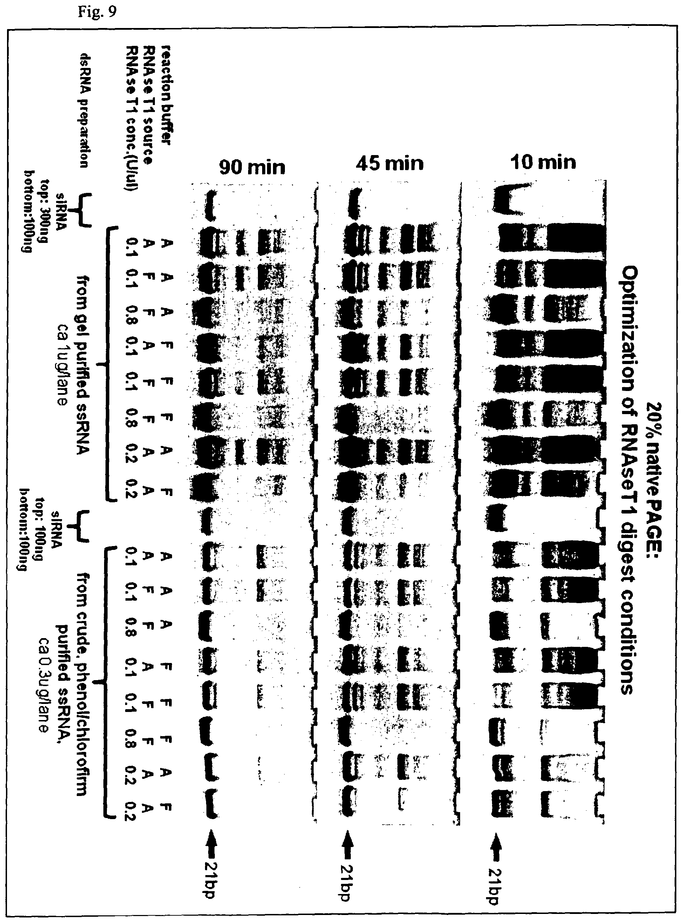

- Fig. 9: depicts optimization of RNAse T1 digest of annealed RNA molecules with ATGGT loop sequence as used for example 1. Each lane of 20% native PAA gel loaded with 5 µl sample of 20ul RNAse T1 cleavage reaction, taken after 10 min (top gel image), 45 min (center gel image) and 90 min (bottom gel image). dsRNA from hybridized gel purified single strand RNA (lane 2-9) or phenol chloroform extracted single strand RNA (lane 11-18) were cleaved under 8 different reaction conditions, varying 3 parameters: reaction buffer (A = Ambion "structure buffer": 10mM Tris/Cl pH 7.0, 100mM KCl, 10mM MgCl₂, FF = Fermentas reaction buffer: 50mM Tris/Cl ph7.4, 2mM EDTA), RNAse T1 (A = Ambion 1units/µl), F = Fementas 1000 units/µl) and RNAse T1 concentration (0.1 to 0.8 units/µl). 100 ng and 300 ng of synthetic siRNA were loaded on lane 1 and 10 as standard. Red arrows indicate the position of 21 nucleotide dsRNA fragments, identical to synthetic siRNAs, 0.8 units/µl of RNAse T1 in a MgCl₂ free buffer are sufficient to digest 1 µg of dsRNA to 21bp fragments in 45 min at 37'C.

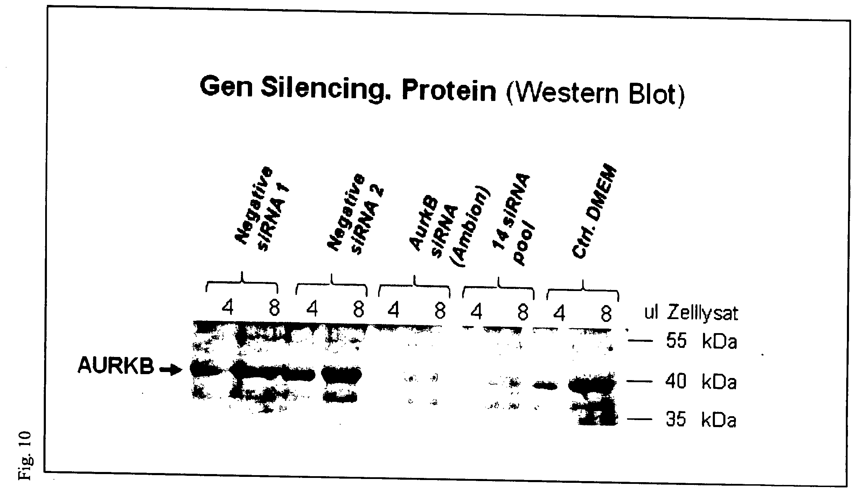

- Fig. 10: depicts Western Blot analysis of AURKB protein knock down by a 14 siRNA pool targeting human AURKB, generated from a dsRNA with AGTTG loop sequence elements. Human HeLa cells were transfected with the siRNA pool or synthetic control siRNAs in a final concentration of 10 nM. Each cell lysates, generated 48h after transfection was loaded on two lanes of a denaturing SDS PAA gel in a volume of 4 µl and 8µl. The blot was developed with an antibody specific to human AURKB. Little or no change in AURKB protein in negative control siRNA (lane 1-4) transfected cells as compared to medium control (lanes 9, 10). Identical, almost complete reduction of AURKB protein for the validated, positive control siRNA targeting AURKB (synthetic AURKB siRNA, Ambion #s495) (lane 5, 6) and the siRNA pool (lanes 7, 8).

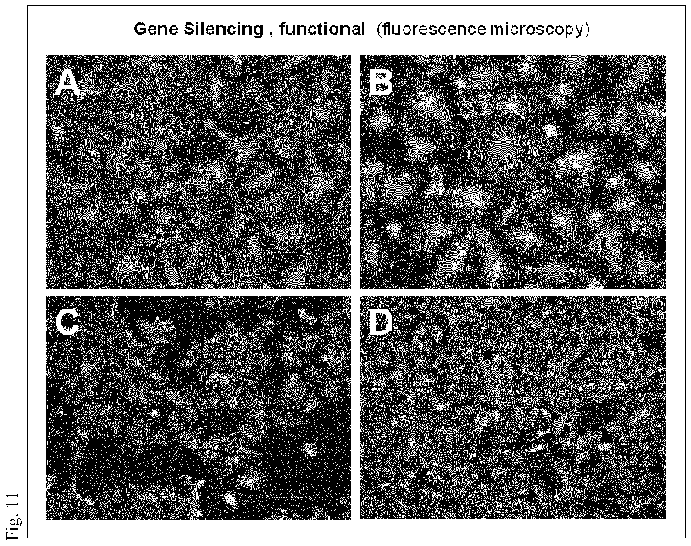

- Fig. 11: depicts the functional validation of a 14 siRNA pool targeting human AURKB, generated from a dsRNA with AGTTG loop sequence elements. Human HeLa cells were transfected with the siRNA pool or synthetic control siRNAs in a final concentration of 10 nM. 72h after transfection, cells were fixed and stained with Dapi for cell nuclei (blue) and an antibody specific to human alpha tubulin (green). Cells were imaged by confocal fluorescence microscopy using a 20x lense. The red sale bar at the lower right of each panel indicates a distance of 100uM. The 4 panels show cells transfected as follows: A: synthetic AURKB siRNA (Ambion #s495), B: 14 siRNA AURKB pool, C: negative control siRNA (Ambion), D: untransfected cells. Cells with strongly increased cell size and multiple cell nuclei are predominant in panel A and B, indicative for efficient knock down of AURKB.

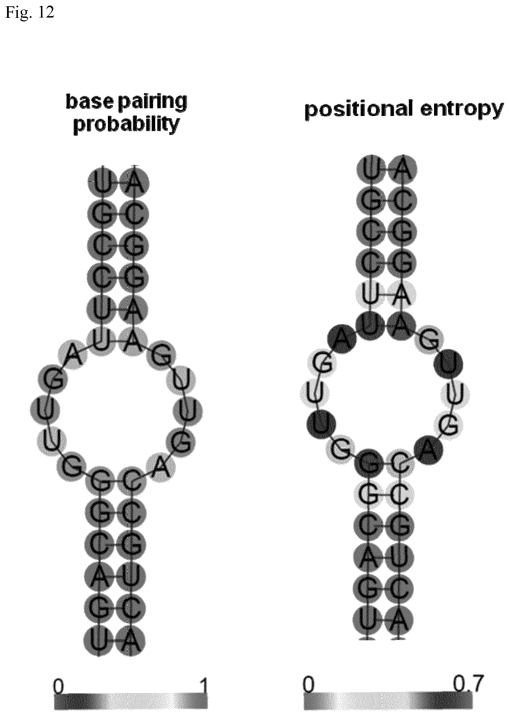

- Fig. 12: depicts minimal free energy structure of dsRNA with loop sequence AGTTG as determined by RNAfold. The image shows one of 14 single strand loops with adjacent base pairing dsRNA regions. Color code indicates base pairing probability (left) and positional enthropy (right). Base pairing probability is identical to all 14 loops. Positional enthropy is constant between the 14 base pairing segments but shows some variability between the loop sequences (not shown).

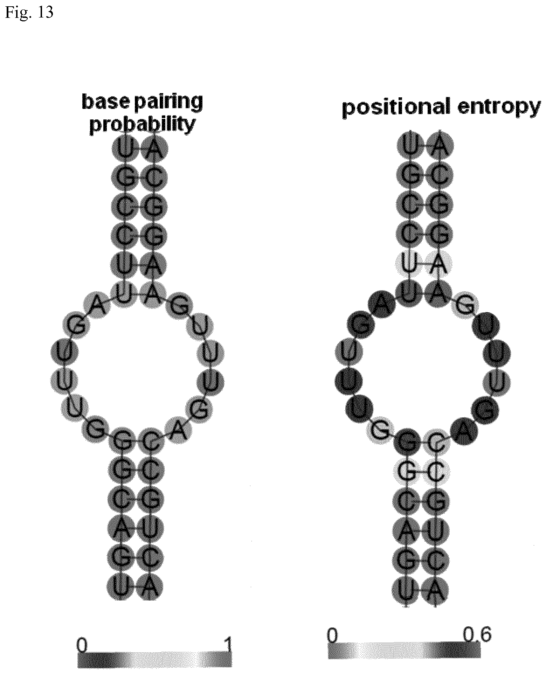

- Fig. 13: depicts minimal free energy structure of dsRNA with loop sequence AGTTTG as determined by RNAfold.The image shows one of 14 single strand loops with adjacent base pairing dsRNA regions. Color code indicates base pairing probability (left) and positional enthropy (right). Base pairing probability is identical to all 14 loops. Positional enthropy is constant between the 14 base pairing segments but shows some variability between the loop sequences (not shown).

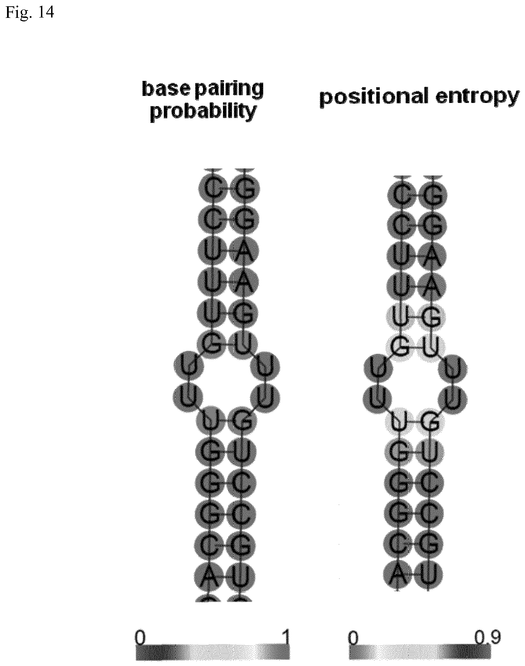

- Fig. 14: depicts minimal free energy structure of dsRNA with loop sequence TGTTTG as determined by RNAfold. The image shows one of 14 single strand loops with adjacent base pairing dsRNA regions. Color code indicates base pairing probability (left) and positional enthropy (right). Base pairing probability and positional enthropy is identical between all 14 segments of the dsRNA (not shown).

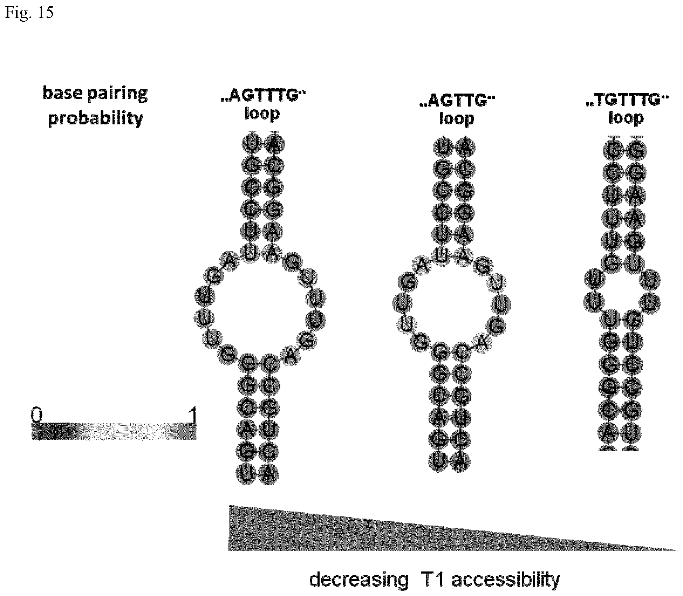

- Fig. 15: compares accessibility to RNAse T1 between dsRNAs with three loop sequences AGTTTG, AGTTG and TGTTTG based on RNAfold minimal free energy structure. The image shows one of 14 single strand loops with adjacent base pairing dsRNA regions. The color code indicates the base pairing probability.

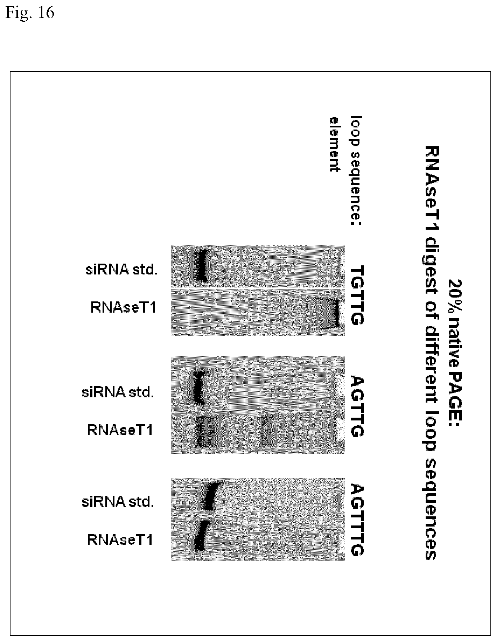

- Fig. 16: Comparison of RNAse T1 cleavage efficiency between dsRNAs with the three loop sequence elements AGTTTG, AGTTG and TGTTTG. Equal amounts of dsRNA containing the three loop sequences were digested with equal concentration of RNAse T1 for 10 minutes. Samples were analyzed by 20% native PAGE. Synthetic siRNA was loaded for comparison. DsRNA with AGTTTG loop sequence element was completely digested to 21base pair dsRNA fragments identical to the siRNA control.

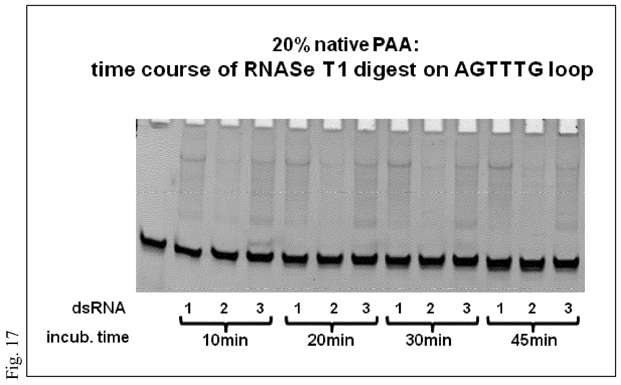

- Fig. 17: Time course of RNAse T1 cleavage of dsRNA with AGTTTG loop sequence element. 3 dsRNA preparations (ds RNA 1,2 and 3) with identical sequence purified by size exclusion chromatography (dsRNA 1 and 2) or phenol chloroform extraction and ethanol precipitation (dsRNA3) were incubated with 1 unit/µl of RNAse T1 at 37'C. Aliquots were taken after 10, 20, 30 and 45 minutes and analyzed by 20% native PAGE. A synthetic siRNA was loaded for comparison (lane 1) (synthetic AURKB siRNA, Ambion #s495). For all three dsRNA samples, completion of the digest was reached after 10 minutes. Lower purity of dsRNA 3 (lanes 4,7,10 and 13), did not affect the efficiency of the digest.

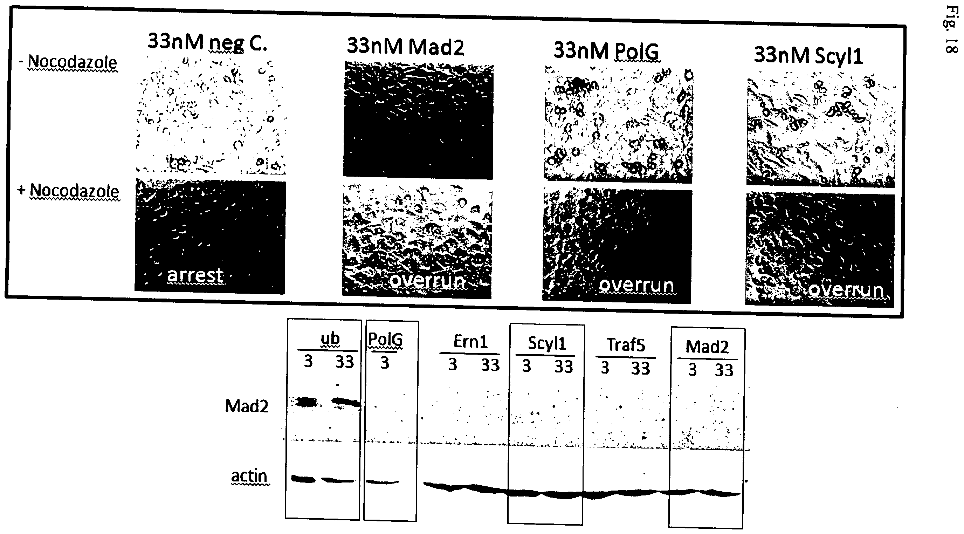

- Fig. 18: depicts the off-target effect on Mad2 expression by the siRNAs , "PolG siRNA OT" and "Scyl1 siRNA OT" as compared to a non gene targeting "negative Control" and a "Mad2 siRNA" with on-target silencing of Mad2 as positive control. Upper panel shows arrest or overrun of mitotic arrest in the absence or presence of nocodazole depending on Mad2 expression. Lower panel shows effects of siRNA transfection on Mad2 protein expression as verified by Western Blots. Details are described in Example 3.

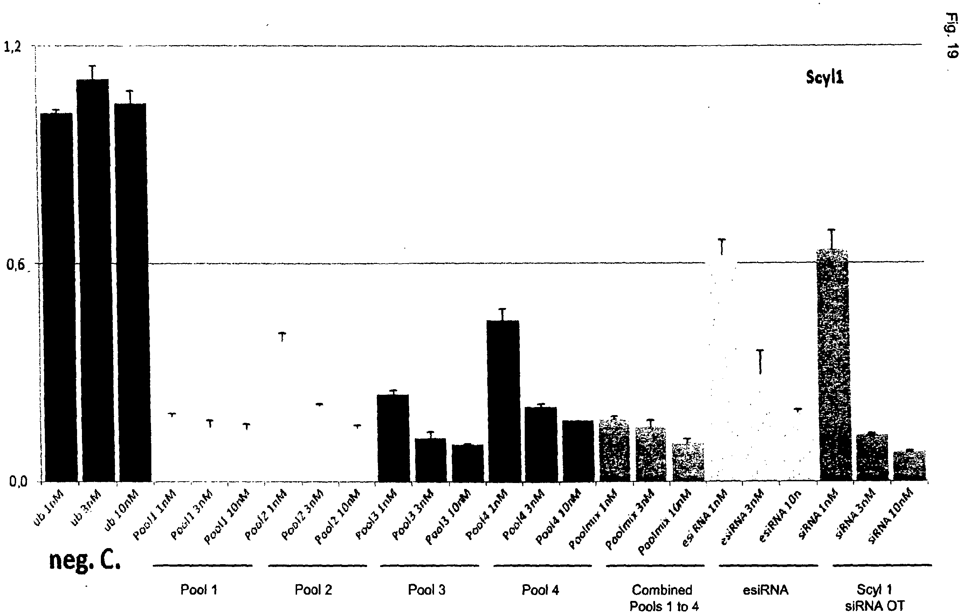

- Fig. 19: depicts the improved on-target silencing of Scyl1 by the Pools 1, 2, 3 or 4 and the combined Pools 1 to 4 for Scyl1 as compared to the esiRNA for Scyl1 and "Scyl1 siRNA OT"as determined by RT-PCR. Scyl1 expression is indicated as % remaining Scyl1 mRNA as compared to a "negative Control" treated sample. Details are described in Example 3.

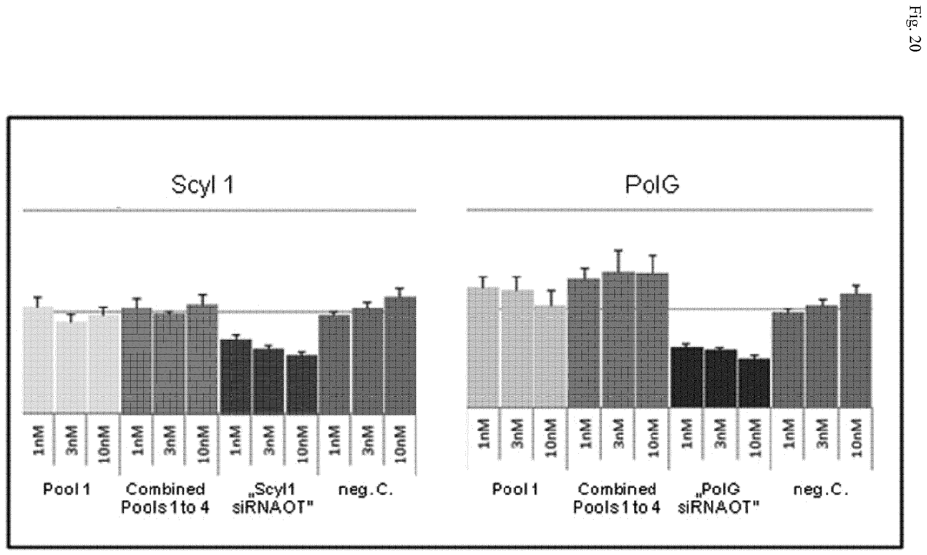

- Fig. 20: depicts the reduced off-target effect on Mad2 expression by Pool 1 and the combined Pools 1 to 4 for Scyl1 as compared to "Scyl1 siRNA OT" as positive control and the non gene targeting siRNA "negative control" (neg. C), of Pool 1 for PolG, of combined Pools 1 to 4 for PolG and of the siRNA "PolG siRNA OT" on Mad2 at InM, 3nM or 10 nM. Effects on Mad2 expression were determined by Luciferase assay. Details are described in Example 3.

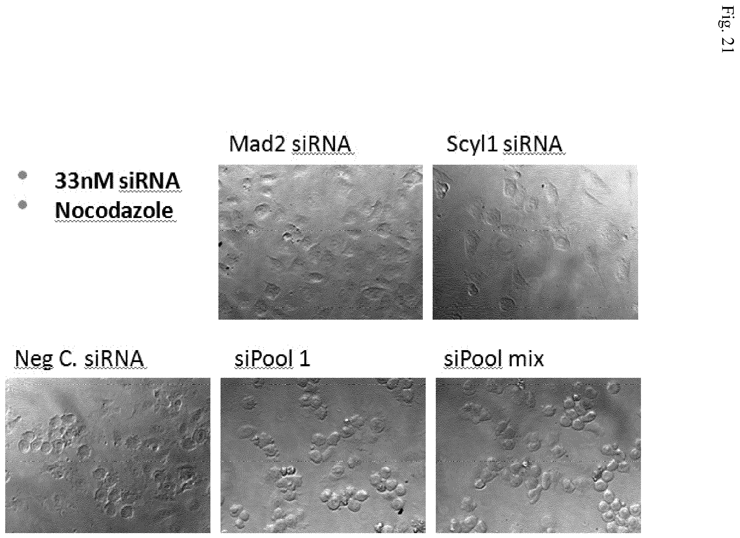

- Fig. 21: depicts the reduced off-target effects on Mad2 as determined by a cellular assay. To this end, HeLa cells were transfected with 33 nM of either "Mad2 siRNA", "negative Control siRNA", Pool 1 for Scyl1, the combined Pools 1 to 4 for Scyl1 and "Scyl1 siRNA OT". If nodocazole was added, an overrun was observed for "Mad2 siRNA" and "Scyll siRNA OT", but not for "negative Control siRNA" (neg. C. siRNA), Pool1 for Scyl1 or for the combined Pools 1 to 4 for Scyll. Details are described in Example 3.

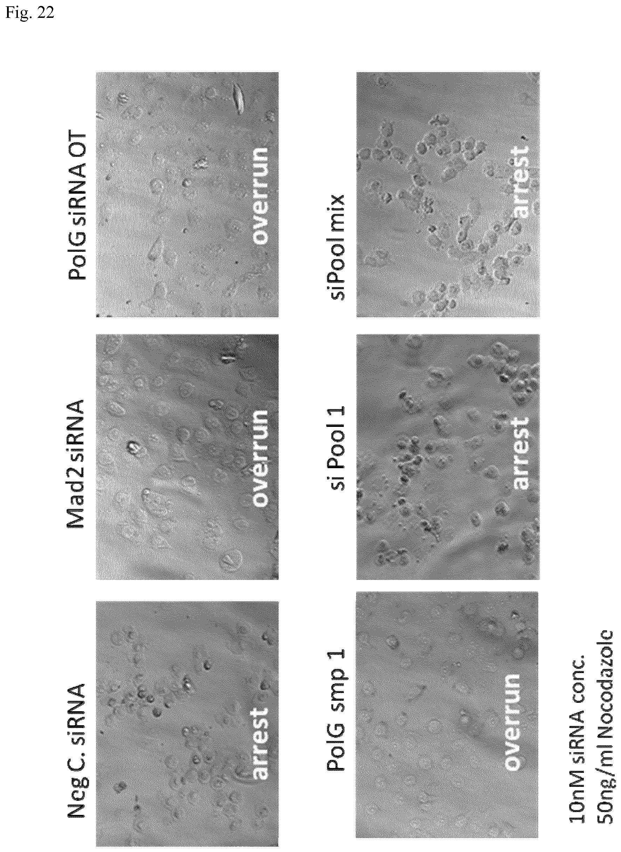

- Fig. 22: depicts off-target effects on Mad2 as determined by a cellular assay. To this end, HeLa cells were transfected with 10 nM of either "Mad2 siRNA", "negative Control siRNA", Pool 1 for PolG, the combined Pools 1 to 4 for PolG, smart pool 1 (smp 1) for PolG and "PolG siRNA OT". If nocodazol was added, an overrun was observed for "Mad2 siRNA" and "PolG siRNA OT", but not for "negative Control siRNA", Pool1 for PolG or for the combined Pools 1 to 4 for PolG. Details are described in Example 3.

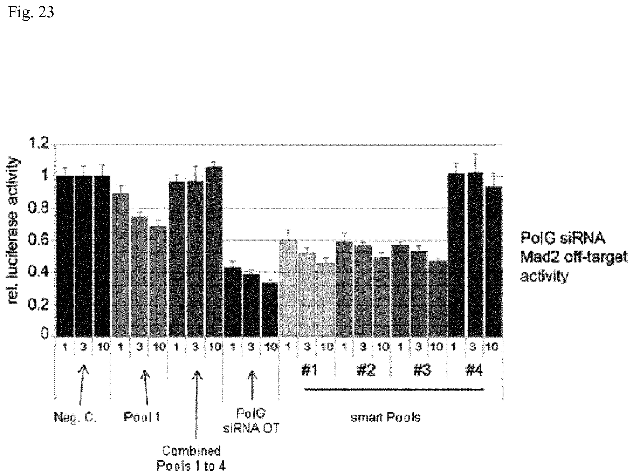

- Fig. 23: depicts off-target effects on Mad2 as determined with a luciferase assay by Pool 1 for PolG and combined Pools 1 to 4 for PolG vs. the off-target effects of "PolG siRNA OT" and smart pools #1, #2, #3 and #4 (smp 1, smp 2, smp 3 and smp 4) for PolG. Each pool or individual siRNA was transfected in 3 concentrations, 1,3 and 10nM as indicated below the bars. Details are described in Example 3.

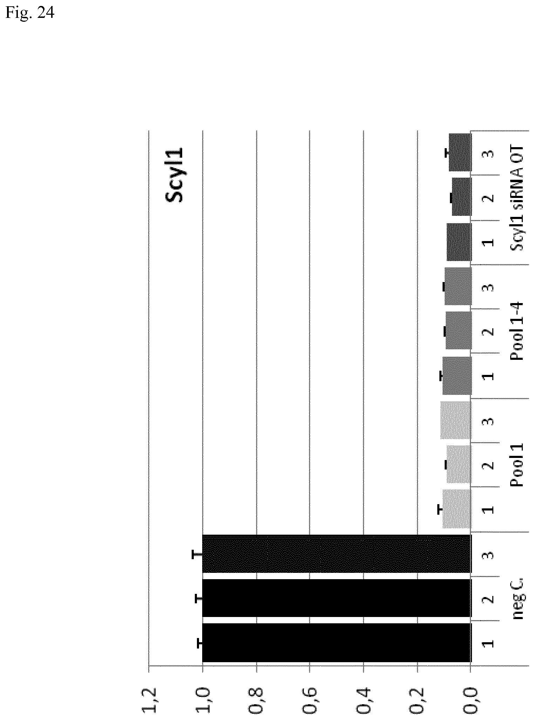

- Fig. 24: depicts on-target effects of the Pools 1, the combined Pools 1 to 4 for Scyl1 and "Scyl1 siRNA OT on Scyl1 expression as determined by RT-PCR. Details are described in Example 4.

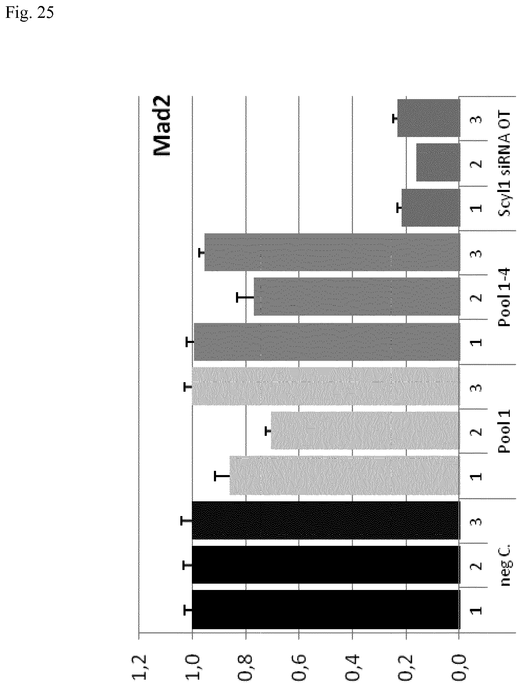

- Fig. 25: depicts the reduced off-target effect on Mad2 expression by Pool 1 (see Table 7) and the combined Pools 1 to 4 for Scyl1 as compared to "Scyl1 siRNA OT" as positive control. Details are described in Example 4.

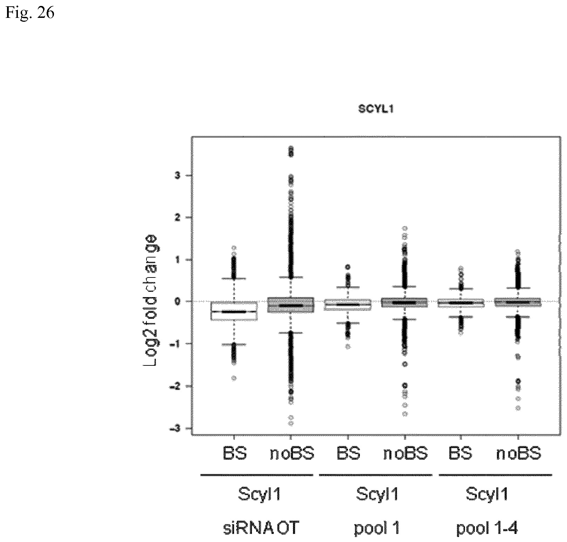

- Fig. 26: depicts the Logarithmic fold changes (base 2) of transcripts without (noBS) or with one or more potential binding site(s) for "Scyl1 siRNA OT" (BS). The two leftmost boxes show log2 fold changes of the samples treated with the single siRNA versus untransfected control samples, the central two boxes fold changes for the complex siRNA pool 1 and the two rightmost boxes for the complex siRNA pool 1 to 4. The boxes represent the interquartile range (IQR) consisting of the central 50% of the data. The whiskers indicate the maximum and minimum of the data defined as 1.5 times the interquartile range. Data values larger than the maximum and smaller than the minimum are drawn as outliers (black circles). Notches roughly indicate the 95% confidence interval of the median (+/-1.58 IQR/sqrt(n)). Details are described in Example 4.

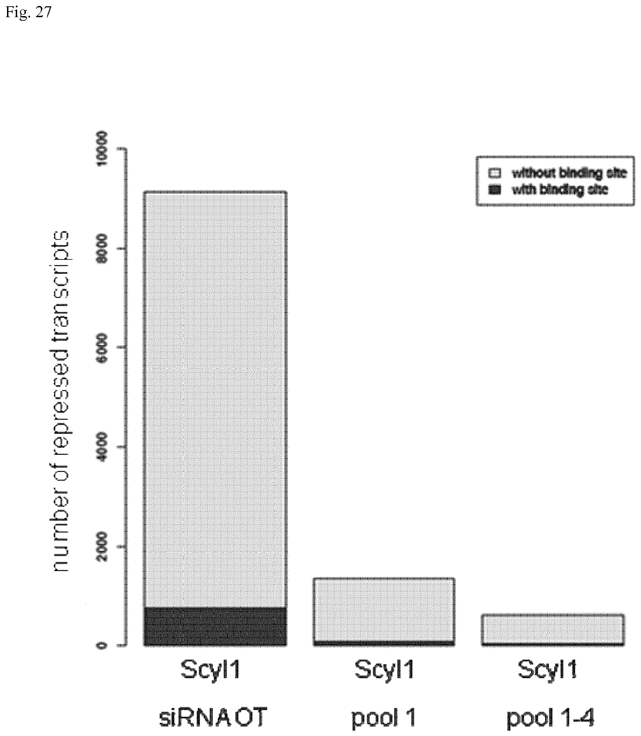

- Fig. 27: depicts the number of repressed transcripts in the individual experiments. Transcripts with a potential binding site (seed sequence) to the "Scyll siRNA-OT" are represented in the dark grey fraction of the bar, the remaining transcripts in the light grey fraction of the bar. Transcripts differentially expressed at a q-value level of 0.001 were considered. Details are described in Example 4.

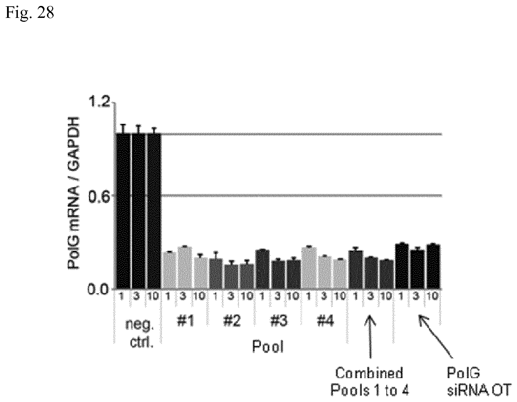

- Fig 28: depicts on-target activity of different complex siRNA pools (siPools) on PolG. Hela cells were transfected with 1, 3 or 10 nM concentrations of siPools containing 15 siRNAs (pools #1-4), a combination of all 15 siRNA-siPools resulting in a siPool containing 60 different siRNAs (pool 60) or specific siRNA "PolG si RNA OT" directed against PolG. PolG mRNA levels were measured by qPCR and normalized to GAPDH. Relative expression levels were calculated based on transfection of an unspecific control siRNA (neg. Ctrl.).

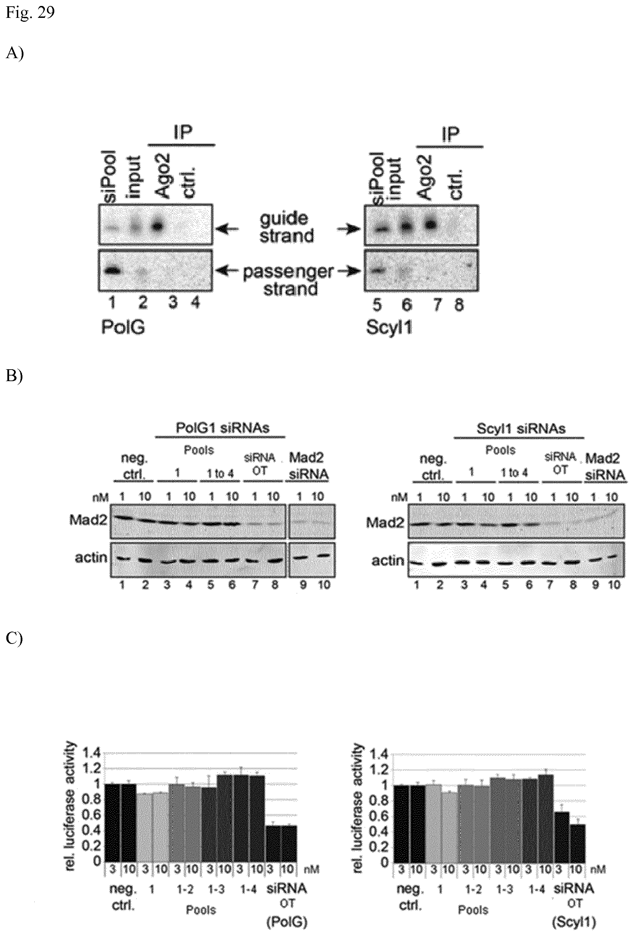

- Fig. 29: depicts off-target activity of different complex siRNA pools (siPools). (A) Hela cells were transfected with 10 nM of combined pools 1 to 4 for PolG or Scyl1. To validate that the specific off-T siRNAs are present in the pools, Ago2 was immunoprecipitated from the lysates and passenger and guide strands of PolG off-T (left) or Scyll off-T (right) siRNAs was analyzed by Northern blotting. As positive controls, 3 pmol of total siPools and 2.5% input material were used. (B) Hela cells were transfected with 1 or 10 nM Pool 1 combined Pools 1 to 4 or specific off-T siRNAs directed against PolG (PolG siRNA OT) or Scyl1 (Scyl1 siRNA OT) Mad2 protein levels were analyzed by western blotting 48 h after transfection. A specific Mad2 siRNA served as a positive control (lanes 9 and 10). Actin expression levels were used as loading controls (lower panels). (C) Hela cells were transfected with 3 or 10 nM siRNA off-T or Pools 1, 1-2, 1-3 or 1-4 different siRNAs directed against PolG or Scyll. Off-target activity was analyzed using Mad2 3'UTR controlling firefly-luciferase activity. Relative luciferase activity was calculated using the ratio of firefly/renilla luciferase and via normalization to the corresponding ratios of the empty control vector.

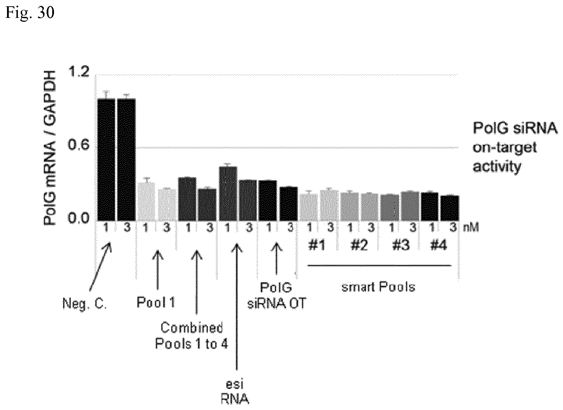

- Fig. 30: depicts comparison of complex siRNA pools (siPools) with other available RNAi reagents. qPCR analysis of on-target activities of various siRNA reagents for PolG. Hela cells were transfected with 1 or 3 nM Pool 1, combined Pools 1 to 4, PolG siRNA OT, esiRNAs and four different smart pools directed against PolG. mRNA levels were normalized to GAPDH and relative expression levels were calculated using a negative control siRNA. Smart pool #4 served as a Mad2 off-target negative control.

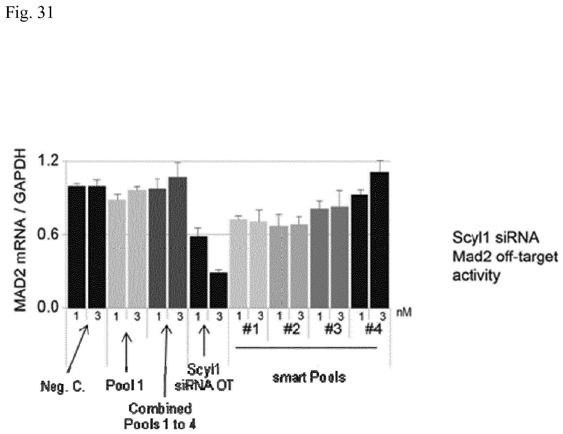

- Fig. 31: depicts comparison of complex siRNA pools (siPools) with other available RNAi reagents. qPCR analysis of off-target effect on MAD2 of various siRNA reagents for SCYL1. Hela cells were transfected with 1 or 3 nM Pool 1, combined Pools 1 to 4, Scyl1 siRNA OT, and four different smart pools directed against Scyll. MAD2 mRNA levels were normalized to GAPDH and relative expression levels were calculated using a negative control siRNA. Smart pool #4 served as a Mad2 off-target negative control.

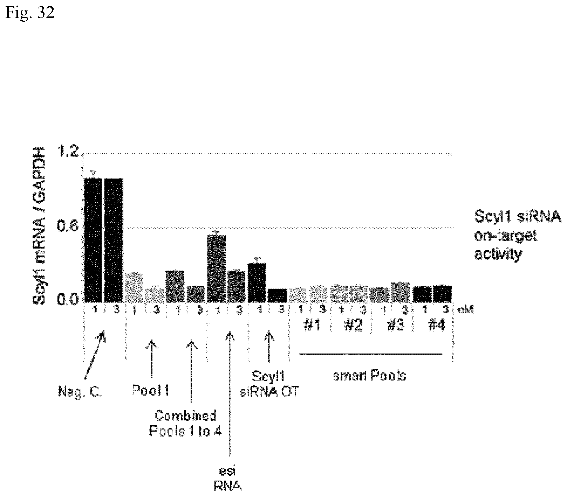

- Fig. 32: depicts comparison of complex siRNA pools (siPools) with other available RNAi reagents. qPCR analysis of on-target silencing activities of various siRNA reagents for SCYL1. Hela cells were transfected with 1 or 3 nM Pool 1, combined Pools 1 to 4, Scyl1 siRNA OT, esiRNAs and four different smart pools directed against Scyl1. mRNA levels were normalized to GAPDH and relative expression levels were calculated using a negative control siRNA. Smart pool #4 served as a Mad2 off-target negative control.

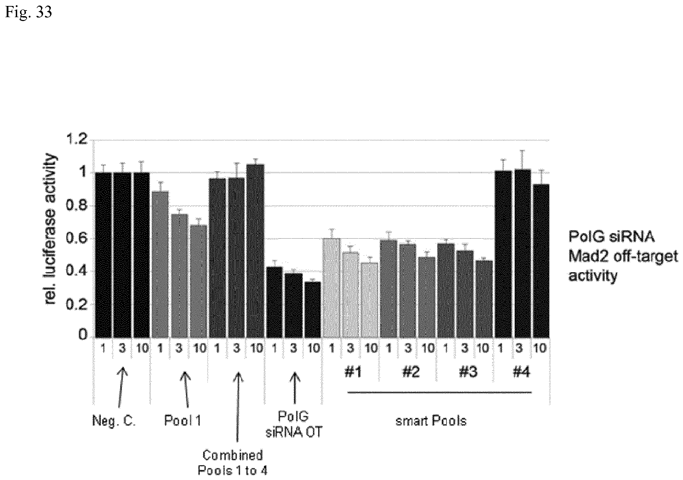

- Fig. 33: depicts comparison of complex siRNA pools (siPools) with other available RNAi reagents. Hela cells were transfected with 1 or 3 nM Pool 1, combined Pools 1 to 4, PolG siRNA OT, and four different smart pools directed against PolG. Off-target activity was analyzed using a reporter system based on firefly-luciferase activity controlled by the Mad2 3' UTR. Relative luciferase activity was calculated using the ratio of firefly/renilla luciferase and via normalization to the corresponding ratios of the empty control vector. Smart pool #4 served as a Mad2 off-target negative control.

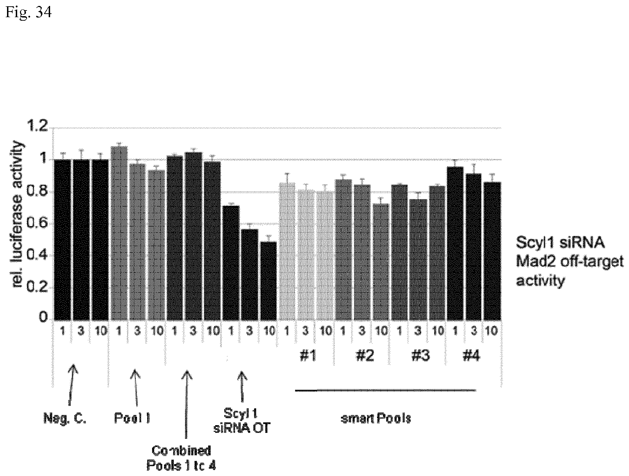

- Fig. 34: depicts comparison of complex siRNA pools (siPools) with other available RNAi reagents. Hela cells were transfected with 1 or 3 nM Pool 1, combined Pools 1 to 4, Scyl1 siRNA OT, and four different smart pools directed against Scyll. Off-target activity was analyzed using a reporter system based on firefly-luciferase activity controlled by the Mad2 3' UTR. Relative luciferase activity was calculated using the ratio of firefly/renilla luciferase and via normalization to the corresponding ratios of the empty control vector. Smart pool #4 served as a Mad2 off-target negative control.

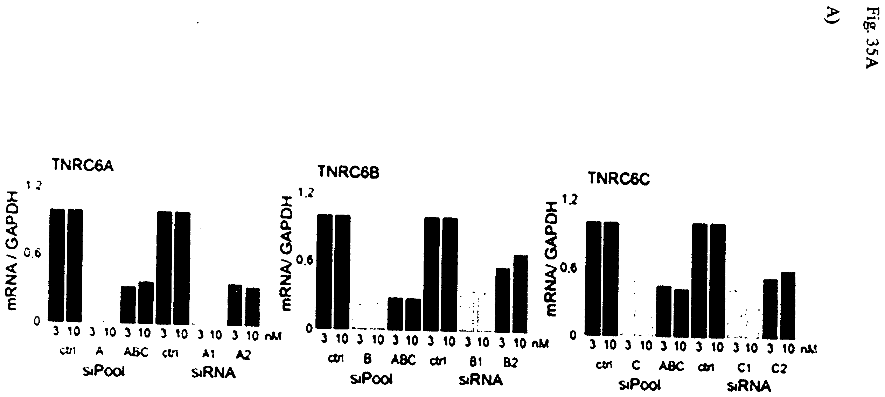

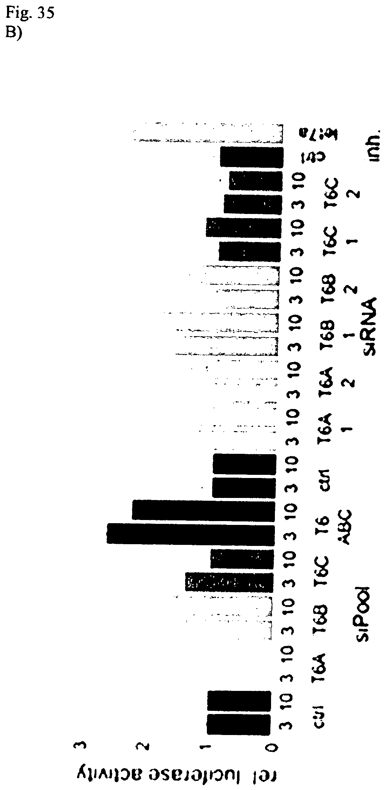

- Fig. 35: depicts silencing of the three members of the TNRC6 gene family by combinations of sipools and individual siRNAs in HeLa cells. A) TNRC6A (left), TNRC6B (center) and TNRC6C (right) were silenced by 3 and 10 nM of siPool and siRNA. SiPools were either specific to the individual TNRC6 gene (A, B or C) or a mixture of all three pools (ABC). Two individual siRNAs were tested against each gene (A1, A2, B1, B2, C1, C2). Gene silencing was quantified by RT-PCR and normalized to GAPDH mRNA and a negative control siRNA. B) Quantification of TNRC6 gene family silencing by a functional luciferase assay. Firefly luciferase was expressed from a reporter construct containing LET7 miRNA binding sites repressing Luciferase expression in Hela cells. Simultaneos silencing of all three members of the TNRC6 gene family became obvious in a de-repression of luciferase expression. Each siRNA or siRNA pool were transfected in Hela cells in concentrations of 3 and 10 nM. siRNA pools targeting individual TNRC6 genes (T6A, T6B, T6C) or all three TNRC6 genes (T6ABC) were compared to the effect of individual siRNAs (T6A1,-2, T6B1,-2, T6C1,-2). Luciferase activity was measured in a dual luciferase assay and normalized to negative control siRNA transfected samples.

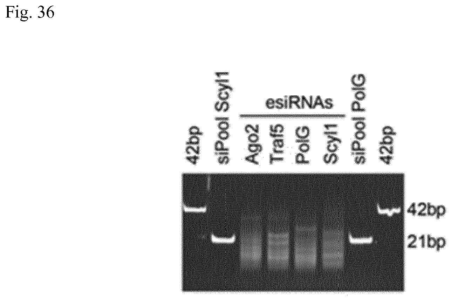

- Fig. 36: depicts .a siRNA pools and esiRNAs analyzed on a 20% native PAA TBE gel. Approximately 300ng of 42bp dsRNA markers, siPools targeting SCYL1 and POLG and esiRNAs (Sigma) targeting the genes AGO2, TRAF5, POLG and SCYL1 were loaded as indicated above the gel image.

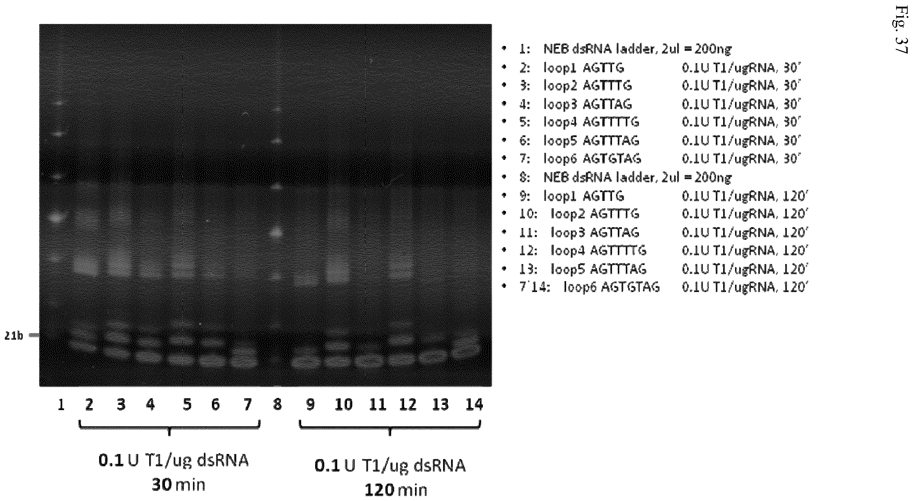

- Fig. 37: depicts.enzymatic cleavage of dsRNA with different loop sequences by RNaseT1 under limiting conditions. Long dsRNA with 15siRNAs targeting human AURKB were generated from DNA templates containing 6 different loop sequence elements: 1) AGTTG, 2) AGTTTG, 3) AGTTAG, 4) AGTTTTG, 5) AGTTTAG, 6)AGTGTAG. DsRNAs were digested with 0.1 unit of RNaseT1 / ug dsRNA for 30 (lanes 1-7) and 120 minutes (lanes 9-14) resulting in a partial digest of the dsRNA.dsRNA fragments were resolved on a native 20% PAA TBE gel. 200ng dsRNA marker (NEB) with a smallest fragment of 21 bp (red arrow) was loaded on lane 1 and 8.

### Detailed Description of the Invention

As already mentioned, the present invention is based on the concept of transcribing and hybridising template molecules which will give rise to annealed RNA molecules of the general structure depicted in Figures 1 and 2. By the steps of transcribing such template molecules, hybridising them and digesting them with RNAse T1 ultimately a multitude of siRNA molecules can be produced which for the purposes of the present invention are described as siRNA pools. The invention has been described by using such template molecules by *in vitro* transcription, hybridization and digestion with RNase T1 to produce an siRNA pool for the gene silencing of AUKRB, Scyl 1 and PolG. As has been pointed out and will be described in further detail below, the skilled person will immediately understand that the invention is not limited to the production of siRNAs for these specific target genes. Nevertheless, the invention will be explained primarily with respect to the specific constructs disclosed herein as this should facilitate an understanding of the invention.
Before the present invention is described in further detail, the following definitions are provided:
The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.
Where the term "comprising" is used in the present description and claims, it does not exclude other elements or steps. For the purposes of the present invention, the term "consisting of' is considered to be a preferred embodiment of the term "comprising of'. If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

If technical terms such as RNAi and siRNA are not defined otherwise, they are used in their common technical sense. A suitable source for the understanding of such technical terms may be Günter Kahl, The Dictionary of Gene Technology, 2nd edition, 2001, Wiley VCH.

If the term "RNase T1" is used hereinafter this should apply to all forms and variants of RNase T1, e.g. those that have been optimised by mutation, as long these forms and variants provide for the same activity and specificity as RNase T1. The RNase T1, which was used in the experiments described hereinafter, has the sequence of (SEQ ID No.: 546) and was obtained from Fermentas (Thermo).

The term "siRNA pool" as used as described herein refers to a multitude of siRNA molecules which can be produced in accordance with the methods of the present invention. These siRNA molecules may either all have the same sequence, may have different sequences being directed to the same target gene or may have different sequences being directed to different target genes.

The term siRNA molecule is used to describe double-stranded RNA molecules wherein each strand of said double-stranded RNA molecules has a length of 15 to 30 nucleotides and wherein said double-stranded RNA molecules by way of the selected sequences are capable of RNAi of at least one target gene. As will become apparent from the ensuing description, the term siRNA molecules comprise blunt ended siRNA molecules as well as siRNA molecules with a 3' overhang.

The terms "high complexity siRNA pool", "complex siRNA pools" or "siPools" are used hereinafter to refer particularly to a combination of at least 5 and preferably at least 8 siRNAs, all being targeted against at least one gene, preferably against one gene. Even more preferably, such a high complexity siRNA pool may comprise at least 10, at least 11, at least 12, at least 13 or at least 14, preferably at least 15, at least 20, at least 25, at least 30, at least 40, at least 50, at least 60, at least 70, at least 80, at least 90 or at least 100 siRNAs, all being targeted against at least one gene, preferably against one. The siRNAs of such high complextiy siRNA pools may be produced by the methods in accordance with the invention, but also by methods known in the state of the art such as by chemical synthesis. Hig complexity pools against different genes can thus be obtained by mixing high complexity pools against one gene, which according to the above considerations comprise at least at least 5, preferably at least 8 siRNAs and even more preferably at least 10, at least 11, at least 12, at least 13 or at least 14, preferably at least 15, at least 20, at least 25, at least 30, at least 40, at least 50, at least 60, at least 70, at least 80, at least 90 or at least 100 siRNAs against one gene. Such high complexity pools can be used for treating a disease in a human or animal being.

The terms DNA, RNA etc. are used in their common sense. It is to be understood that where the present invention, for example, mentions DNA molecules, this does not necessarily exclude that such DNA molecules are modified DNA molecules having e.g. modifications or unusual bases. However, such modifications have to be selected so that the properties of these DNA molecules, such as the ability to be replicated, *in vitro* transcribed and hybridized are not affected. It is preferred that DNA molecules comprise the naturally occurring bases and have a phosphate backbone.

The term siRNA molecule as pointed out above must refer to a double-stranded RNA molecule. However, as the siRNA molecules are produced by *in vitro* transcription, hybridization and digestion with an RNase, of which RNAse T1 may be preferred,, the RNA molecules can be made from nucleotides that are modified to increase protease resistance. Again, such modifications must be selected so that the *in vitro* transcribed RNA molecule can still be recognised, cleaved and digested by RNases, of which RNAse T1 may be preferred, as described herein. Even though it is understood that RNAs comprise U instead of T, the ensuing description where it refers to specific sequences may not reflect this. Thus, the person skilled in the art will understand that when a sequence is mentioned to be an RNA and where the sequence is indicated to comprise T, this actually refers to U. It is preferred to have siRNA molecules which do not comprise any non-natural modifications meaning that the siRNA molecules should use the common RNA nucleotides being A, U, C, G being connected by phosphate bonds.

As mentioned herein, the invention contemplates for loops sequences to be cleaved by RNase _{[S1]}T1 loop sequence which only consist of A and G. This will allow to make siRNAs by *in vitro* transcription, which can incorporate modified T and C. Examples of such modified nucleotides are sugar modifications as 2'-Fluoro-2'-deoxy, 2'-Amino-2'-deoxy, 2'-Azido-2'-deoxy or 2'-O-methylcytidin or uridine.

As already mentioned above, the present invention relates to a method of preparing double stranded RNA molecules, wherein each strand of said different double stranded molecules has a length of 15 to 30 nucleotides wherein said different double stranded RNA molecules are capable of target-specific RNA interference of at least one target gene, said method comprising at least the steps of:
a. Providing at least one first DNA molecule,
b. Providing at least one second DNA molecule,
c. *In vitro* transcribing said at least one first and at least one second DNA molecules using an RNA polymerase to obtain corresponding at least one first and at least one second RNA molecules,
d. Hybridizing said at least one first and at least one second RNA molecules of step c. to obtain an double stranded RNA molecule of the general structure depicted in Fig. 1,
e. Digesting the double stranded RNA molecule obtained in step d. with RNAse T1, which is capable of preferentially recognizing, cleaving and digesting the single stranded loop-sequence-elements over the hybridized double stranded sections of the double stranded RNA obtained in step d. thereby removing single stranded RNA loops,
wherein the sequence of said target-sequence-elements depicted on Fig. 1 of the at least one first DNA molecule is sense to sequences of said at least one target gene of RNA interference, wherein the sequences of said target-sequence-elementsrc of the at least one second DNA molecule are the reverse complements of the sequences of the target-sequence-elements of the at least one first DNA molecule, which they hybridize to, and wherein the loop-sequence elements of the at least one first and at least one second DNA molecules are not reverse complements of each other,
wherein the sequences of the loop-sequence-elements are selected such that double stranded RNA molecules are obtained by recognition, cleavage and digestion of the single stranded loop-sequence elements by RNase T1 in step e., wherein each strand of said resulting double stranded molecules has a length of 15 to 30 nucleotides and wherein said resulting double stranded RNA molecules are capable of target-specific RNA interference of at least one target gene.

The present invention thus relates to a method comprising at least the steps of:
a. Providing at least one first DNA molecule comprising in the 5'-3' direction as repeating units a nucleic acid sequence with the following elements:
   5' - [(target-sequence-element) - (loop- sequence-element)]ₖ-3', with k being an integer >1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
   with X being A and m being an integer of 1,
   with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,
b. Providing at least one second DNA molecule comprising in the 5'-3' direction in a repetitive manner a nucleic acid sequence with the following elements:
   5' - [(target-sequence-element)_{rc} - (loop-sequence-element)]ₗ-3', with l being an integer >1 and having the same value as k in the first DNA molecule,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
   with X being A and m being an integer of 1,
   with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference, wherein the target-sequence-elementsrc counted from the 3' end in the repeating units of said second DNA molecule are the respective reverse complement of the target-sequence-elements counted from the 5' end in the repeating units of said first DNA molecule, and wherein the loop-sequence-elements in the repeating units of said second DNA molecule are not reverse complements of the loop-sequence-elements in the repeating units of said first DNA molecule,
c. *In vitro* transcribing said at least one first and at least one second DNA molecules using an RNA polymerase to obtain corresponding at least one first and at least one second RNA molecules,
d. Hybridizing said at least one first and at least one second RNA molecules of step c. to obtain a double stranded RNA molecule of the general structure depicted in Fig. 1,
e. Digesting the double stranded RNA molecule obtained in step d. with RNAse T1, which is capable of preferentially recognizing, cleaving and digesting the single stranded loop-sequence-elements over the hybridized double stranded sections of the double stranded RNA obtained in step d. thereby removing single stranded RNA loops,
wherein the sequences of the loop-sequence-elements are selected such that double stranded RNA molecules are obtained by recognition, cleavage and digestion of the single stranded loop-sequence elements by RNAse T1 in step e., wherein each strand of said resulting double stranded molecules has a length of 15 to 30 nucleotides, wherein said resulting double stranded RNA molecules are capable of target-specific RNA interference of at least one target gene.

Figure 1 shows a hybridized RNA molecule that is obtained upon *in vitro* transcription and hybridization of the at least one first DNA and at least one second DNA molecule as mentioned above. As one can see, the first and the second DNA molecules consist of target sequence elements as well as loop sequence elements.

The target sequence elements are selected such that they comprise at least a substantial part of the sequence of the siRNA molecules to be produced. Thus, the target sequence elements comprise a sequence that matches part of a sequence of the target gene(s) that is (are) to be silenced by the siRNA molecules. For the sake of nomenclature, the target sequence element in the at least one first DNA molecule should correspond to the sense sequence of the target gene to be ultimately targeted by the siRNA molecules. This follows from the understanding that one strand of the siRNA corresponds to the sequence of a cDNA of a gene which is understood to refer to the sense sequence. The sequence of at least one first DNA molecule corresponds to the cDNA sequence and thus to the sense sequence. As a consequence, the target sequence elements of the second DNA molecule will comprise a sequence corresponding to the antisense sequence of the target gene. Given that the at least one first and at least one second DNA molecule upon transcription and hybridization should form double-stranded RNA section of the target sequence elements, the target sequence elements of the at least one first and the at least one second molecule will have to be selected both in terms of sequence and order such that the first target sequence element of the first DNA sequence counted from the 5' end can hybridize to the sequence of the first target sequence element of the second DNA molecule counted from the 3' end. Similarly, the second target sequence element of the first DNA molecule counted from the 5' end must be selected so that it can hybridize with the target sequence element for the second target sequence element of the second DNA molecule counted from the 3' end. As a consequence, the first target sequence element of the second DNA molecule counted from the 3' end will be the reverse complement of the first target sequence element of the first DNA molecule counted from the 5' end. Similarly, the second target sequence element counted from the 3' end of the second DNA molecule will be the reverse complement of the second target sequence element of the first DNA molecule counted from the 5' end, etc. This relationship is depicted in Figure 1.

Further, the various target sequence elements of the first and the second DNA molecule which upon transcription and hybridization of these DNA molecules form double-stranded RNA sections are intermitted by single-stranded loop sequence elements. These loop sequence elements must be selected such that they upon *in vitro* transcription and hybridization of the two DNA molecules do not form double-stranded sections, but instead single-stranded loop sections such that they can be recognised, cleaved and digested by an RNase over double-stranded target sequence elements.

Even though this is not depicted in Fig. 1, the invention also envisages embodiments where the first target sequence element, as counted from the 5' end, is preceded by a loop sequence element and where the last target sequence element, as counted from the 5' end, is followed by a sequence element.

As is immediately evident from Figure 1, the concept laid out for the first aspect of the invention cannot only be realised by the use of two different DNA molecules, which upon *in vitro* transcription and hybridization form the general structure depicted in Figure 1, but can also be realised by single DNA molecules that in addition to the target sequence elements and the loop sequence elements comprise an additional loop sequence element, which is designated for the purposes of the present invention as a hairpin loop sequence element that allows upon *in vitro* transcription of such a DNA molecule the 3' end to fall back to the 5'end and to form the general structure depicted in Figure 2. However, the considerations to be applied for the selection of the target sequence elements and the loop sequence element as pointed out above for the first aspect of the invention equally apply. Thus, it must be ensured that the first target sequence element counted from the 3' end is the reverse complement of the first target sequence element counted from the 5' end. Further, the second target sequence element counted from the 3' end must be the reverse complement of the second target sequence element counted from the 5' end, etc. Again the sequences of the loop sequence elements shall be selected to be not reverse complements of each other.

In a second aspect the present invention thus also relates to a method of preparing different double stranded RNA molecules, wherein each strand of said double stranded molecules has a length of 15 to 30 nucleotides, wherein said double stranded RNA molecules are capable of target-specific RNA interference of at least one target gene, said method comprising at least the steps of:
a. Providing at least one DNA molecule,
b. *In vitro* transcribing said at least one DNA molecules using an RNA polymerase to obtain corresponding at least one first RNA molecule, which upon hybridization provides the general structure depicted in Fig. 2,
c. Digesting the RNA molecule obtained in step b. with RNAse T1, which is capable of of preferentially recognizing, cleaving and digesting the single stranded loop-sequence-elements over the hybridized double stranded sections of the double stranded RNA obtained in step b. thereby removing single stranded RNA loops,
wherein the sequence of said target-sequence-elements depicted on Fig. 2 of the at least one first DNA molecule is sense to sequences of said at least one target gene of RNA interference, wherein the sequences of said target-sequence-elementsrc of the at least one DNA molecule are the reverse complements of the sequences of the target-sequence-elements of the at least one first DNA molecule, which they hybridize to, and wherein the loop-sequence elements of the at least one first and at least one second DNA molecules are not reverse complements of each other,
wherein the sequences of the loop-sequence-elements are selected such that double stranded RNA molecules are obtained by recognition, cleavage and digestion of the single stranded loop-sequence elements by RNAse T1 in step c., wherein each strand of said resulting double stranded molecules has a length of 15 to 30 nucleotides and wherein said rsulting double stranded RNA molecules are capable of target-specific RNA interference of at least one target gene.

In a preferred embodiment of this second aspect, the present invention relates to a method comprising at least the steps of:
a. Providing at least one first DNA molecule comprising in the 5'-3' direction as repeating units a nucleic acid sequence with the following elements:
   5' - [(target-sequence-element) - (loop- sequence-element)]ₖ - (target-sequence-element) - (loop- sequence-element)ₕₚ - [(target-sequence-element)_{rc} - (loop-sequence-element)]ₗ-3',
   with k being an integer >1,
   with l being an integer >1 and being the same as 1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,
   wherein the (loop sequence element)ₕₚ is of sufficient length to allow for a hairpin structure enabling a self-hybdrization pattern depicted in Fig. 2, with the target-sequence-elementrc being a continuous sequence of 15 to 30 desoxyribonucleotides,
   wherein the target-sequence-elementsrc counted from the 3' end are the respective reverse complement of the target-sequence-elements counted from the 5' end,
   wherein the loop-sequence-elements following the the (loop sequence element)ₕₚ are not reverse complements of the loop-sequence-elements preceeding the in the repeating units of said second DNA molecule,
b. *In vitro* transcribing said at least one first DNA molecules using an RNA polymerase to obtain corresponding at least one first RNA molecule, which upon hybridization provides the general structure depicted in Fig. 2,
c. Digesting the double stranded RNA molecule obtained in step b. with RNAse T1, which is capable of preferentially recognizing, cleaving and digesting the single stranded loop-sequence-elements over the hybridized double stranded sections of the double stranded RNA obtained in step b. thereby removing single stranded RNA loops,
wherein the sequences of the loop-sequence-elements are selected such that double stranded RNA molecules are obtained by recognition, cleavage and digestion of the single stranded loop-sequence elements by RNAse T1 in step c., wherein each strand of said resulting double stranded molecules has a length of 15 to 30 nucleotides and wherein said resulting double stranded RNA molecules are capable of target-specific RNA interference of at least one target gene.

As for the first aspect of the invention and even though this is not depicted in Fig. 2, the invention for the second aspect also envisages embodiments where the first target sequence element, as counted from the 5' end, is preceded by a loop sequence element and where the last target sequence element, as counted from the 5' end, is followed by a sequence element.

As regards the hairpin loop sequence element of Figure 2, the only restriction is that the sequence must be of sufficient length to allow a self-hybridization as depicted in Figure 2.

For both, the first and second aspect of the invention, the number of target sequence elements which define the number of an siRNAs that will be produced by methods in accordance with either the first or the second aspect of the invention can vary. Thus, the number of target sequence elements ultimately giving rise to a number of siRNA molecules may be at least 2, at least 3, at least 4, at least 5, at least 6, at least 7, at least 8, at least 9, at least 10, at least 11, at least 12, at least 13, at least 14, at least 15, at least 20, at least 30, at least 40, at least 50, at least 60, at least 70, at least 80, at least 90 or at least 100. However, in view of the overall length of the resulting hybridized RNA molecules as depicted in Figure 1 or Figure 2, it seems reasonable that the number of target sequence elements should not exceed more than 100 target sequence elements with an overall number of up to 50 to 60 target sequence elements being reasonable.

Consequently, the integer of k and 1 for both the first and second aspect of the invention can be at least 2, at least 3, at least 4, at least 5, at least 6, at least 7, at least 8, at least 9, at least 10, at least 11, at least 12, at least 13, at least 14, at least 15, at least 20, at least 30, at least 40, at least 50, at least 60, at least 70, at least 80, at least 90 or at least 100. However, in view of the overall length of the resulting hybridized RNA molecules as depicted in Figure 1 or Figure 2, it seems reasonable that the integer of k and 1 for both the first and second aspect of the invention should not exceed more than 100 target with values of up to 50 to 60 being preferred upper limits.

In order to obtain high complexity siRNA pools, the number of target sequence elements may be at least 5, 6, 7, preferably at least 8, 9, 10, 11, 12, 13, 14, more preferably at least 15, 16, 17, 18, 19, 20, 25, 30, 40, 50, 60, 70, 90 or at least 100 sequence elements. It seems that efficient gene silencing and reduced off-target effects as described below can be achieved with a high complexity siRNA pool having at least 8 to 10 such as 11, 12, 13, 14, or 15 siRNAs and thus at least 8 to 10 target such as 11, 12, 13, 14, or 15 sequence elements (if the high complexity siRNA pool is produced by methods in accordance with the invention). A high complexity siRNA pool having at least about 11 to at least about 15 siRNAs and thus at least about 11 to at least about 15 target sequence elements (if the high complexity siRNA pool is produced by methods in accordance with the invention) seems to give substantially no off-target effect. A high complexity siRNA pool having at least about 40 to at least about 60 siRNAs and thus at least about 40 to at least about 60 target sequence elements (if the high complexity siRNA pool is produced by methods in accordance with the invention) seems to give no off-target effect at all.

As has been mentioned above, methods in accordance with the first and second aspect of the present invention and their preferred embodiments can be used to produce different pools of siRNA molecules.

If the sequences of the target sequence elements are the same, the resulting pool with the methods in accordance with the first and second aspect of the present invention and their preferred embodiments will provide an efficient means of producing a pool comprising the same siRNAs. However, if the sequences of the target sequence elements are not the same, two different scenarios can be envisaged.

If the sequences of the target sequence elements are not the same, but are selected such that the resulting siRNAs are all directed to the same target sequence, the methods in accordance with the present invention will allow production of pools of siRNAs all of which are directed to the same target gene. The advantages of such pools and embodiments of the present invention where the target sequence elements are not the same but are directed to the same target genes include that one can efficiently produce a plethora of siRNA sequences. This pool of sequences can then be used to silence the expression of a gene and it can be assumed that at least some of the siRNAs will be effective. As has been noted above, complex siRNA pools, even where all siRNAs are directed to the same target gene can show reduced off-target effects as it seems that those siRNAs which work well with respect to a particular target gene seem to suppress off-target effects by other siRNAs being directed against the same gene.

High complexity siRNA pools as described herein comprise at least 5, preferably at least 8 siRNAs, all being targeted against at least one gene of interest. The siRNAs of such high complexity siRNA pools may be produced by the methods in accordance with the invention, but also by methods known in the state of the art such as by chemical synthesis. The present disclsoure considers to combine different high complexity siRNA pools, with each pool being directed against on target gene of interest to achieve a combination which can be used to simultaneously silence multiple genes and by simultaneously reducing off-target effects.

In a second scenario, the sequences of the target sequence elements are not the same and can be moreover directed to different target genes. The resulting siRNA pool will in this respect be an siRNA pool allowing gene silencing of various target genes. This approach can be used to effectively provide combinations of siRNAs which can be used to e.g. silence gene expression of various homologues of a gene family and/or to e.g. silence gene expression of various genes all which belong to cellular pathways such signal transduction pathways. In this second scenario, one can also create high complexity siRNA pools for silencing e.g. gene homologues or e.g. members of a signal transduction pathway by selecting at least 8 target sequence elements for one homologue or member of the signal transduction pathway, and selecting further at least 8 additional target sequence elements against another homologue or member of the signal transduction pathway, etc.. Depending on how many siRNAs for each separate gene will be present in the resulting pool, high complexity pools against different target genes may be obtained with such high complexity pooly comprising at least five, preferably at least 8 to 10 such as at least 11, 12, 13, 14, or at least 15 siRNAs against each single gene.

It is thus to be understood that the aforementioned number of target sequence elements, which can be present may not only relate to the number of target sequence element of identical sequence being present but also to target sequence elements of different sequences being present. In one particular aspect the aforementioned number may thus relate to the number of different target sequence elements all of which are being directed to a single target gene or all of which are directed to different target genes.

As will become apparent from the ensuing description, the loop sequence elements are chosen such that the resulting siRNA molecules upon recognition, cleavage and digestion (after transcription and hybridization of the template molecules) by RNase T1 have a 3' overhang. Thus, the length of the target sequence of the resulting siRNAs depending on the specific scenario may be determined by the target sequence elements plus nucleotides of the loop sequence elements, namely those that precede the cleavage site of the RNAs. Therefore, no strict limitations are given on the length of the target sequence element.

However, the length of the target sequence elements should be selected such that taking the additional bases that are added from the loop sequence element into account, the resulting siRNA molecules have a length of 15 to 30 nucleotides. Preferably, the resulting siRNA molecules have a length of 17 to 25, 18 to 24, 19 to 23, or 20, 21, or 22 nucleotides with siRNA molecules having a length of 21 nucleotides being preferred. Where the siRNA molecules should have a 3' overhang, the target sequence elements and the loop sequence elements may be designed to give an overhang of 2 nucleotides.

Depending on the number of nucleotides resulting from the loop sequence elements, the length of the target sequence element will thus typically have a continuous sequence of 17 to 23, 17 to 22, 17 to 21, 18, 19 or 20 nucleotides.

In all of the embodiments discussed herein it can be particularly preferred that siRNAs have a length of 21 to 23 nucleaotides including a 3' overhang of 2nucleotide.

The loop sequence elements can be the same throughout the hybridized molecules as depicted in Figure 1 and Figure 2. However, the loop sequence elements is selected such that they are all recognised by RNAse T1
Using these different embodiments, the methods in accordance with the invention, as well as the kits and DNA molecules as described below, can be optimised to provide efficient means for producing siRNAs that have been identified to be particularly suitable for silencing either of single or numerous target genes. Thus, if e.g. certain siRNAs are known to efficiently silence the expression of a particular gene and if these siRNAs differ e.g. by their length and overhangs, these siRNAs can be implemented into the DNA molecules being used according to the first and second aspects of the invention and their preferred embodiments by e.g. modifying the loop sequences accordingly.

As pointed out, the loop sequences are selected such that upon transcription and hybridization these sequence elements form single-stranded, unpaired RNA loops which can preferentially be recognised, cleaved and digested over double-stranded target sequence element sections by Rnase T1.

If it is stated in the context of the loop sequences for RNase T1 that X and Y being selected such that there is no hybridization of two molecules consisting just of the loop-sequence-element, this is to be understood that hybridization does not occur to an extent that double stranded or other secondary structure elements form which would prevent RNase T1 from acting on the loop sequences. Further, the loop sequences must be selected such that the loop sequences of the sense and antisense strand molecules as described hereinafter do not hybridize to an extent extent that double stranded or other secondary structure elements form, which would prevent Rnase T1 from acting on the loop sequences.

Next to the specific RNase T1 loop sequences discussed in the following, the experiments described hereinafter allow the following conclusion for loop sequences being cleavable by RNase T1. It seems that the optimal mimimal length of a loop sequence being cleavably by RNase T1 is 5 to 6 nucleotides_{[S2]}. The integer of m in the below mentioned examples of loop sequences for RNase T1 is 1 and the integer of n is 1, 2, or 3.

Further, it seems that the loop sequence element should have optimally a sequence, which minimizes the occurence of unusual G-T base pairs in the loop sequence elements upon hybridization of the sense and antisense strand. Thus a loop sequence comprising A and G only may be most preferred. This would also allow to incorporate modified T and C in the siRNA target sequences.

The loop-sequence-element has the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element.

In order to determine an optimized loop length and sequence, the skilled person will understand that this sequences should be non-gene specific. Even if gene specific sequences are used, the loop will usually not give rise to siRNAs as it is single stranded and cut at all e.g. G-positions in case of RNase T1. However, even for unusual situations (e.g. comparetively long loop sequences) one can avoid that the loop sequence give themselves rise to undesired siRNAs by properly considering the sepcific sequences. For optimization, one can refer e.g. to established databases and software programmes which allow structure prediction of various loop sequence structures. For example, the Vienna RNA WebServer at http://RNA.tbi.univie.ac.at offers various programes which *inter alia* allow prediction of minimum free energy structures, base pair probabilities and secondary structure predictions from single RNA or DNA sequences or allow prediction of the secondary structure of double stranded RNAs. This web service also provides programmes for assisting in siRNA design. In this respect, reference is made to the software suits RNAfold server, RNAcofold server and RNAxs server (see also Figure 4). Other software programs for designing siRNAs are available e.g. at http://www.dharmacon.com/designcenter/DesignCenterPage.aspx, or https://ecom.mwgdna.com/register/index.tcl?return_url=%2fservices%2fwebeist%2f sirna_design%3fuser_id%3d740967. These latter software programs have been used for designing siRNAs of the Examples.

It has further been found that the minimum free energy structure prediction provided by the RNAfold server provides a suitable guiding parameter for determining both the exact sequence identity as well as the sequence length of the loop sequence elements being recognised, cleaved and digested by RNase T1_{[S3]}. For example, the minimum free energy structure, calculated by RNAfold (see above) for loop sequence structures AGTTG and AGTTTG predicts a low base pairing probability for both loops, suggesting a better accessibility of the desired G-nucleotides to RNAse T1 for the larger, 6 nucleotide loop sequence, AGTTTG than for the smaller, 5 nucleotide sequence AGTTG. For the 6 nucleotide TGTTTG loop sequence structure however, RNAfold predicts a high base pairing probability due to 4 non canonical GU base pairing positions, reducing the effective single strand loop region to the two, central T nucleotides. These data indicate that, considering the length of the effective single strand loop region a measure for the accessibility of the desired G nucleotides to RNAseT1, the AGTTTG loop sequence structure should be optimized for RNAse T1 digest, followed by AGTTG and, worst of all 3, TGTTTG. This is moreover confirmed by the experimental data set out hereinafter in Example 2.

The afore-mentioned software programmes can also be used to determine which hairpin loop sequence element as depicted Fig. 2 are suitable to allow self-annealing.

As mentioned above, the present invention in a third aspect relates to a combination or a kit of at least two DNA molecules, which upon in vitro transcription, hybridization and digestion with RNase T1 according to claim 1 are capable of providing double stranded RNA molecules,wherein each strand of said different double stranded molecules has a length of 15 to 30 nucleotides and wherein said double stranded RNA molecules are capable of target-specific RNA interference of at least one target gene, wherein said at least two DNA molecules have the sequence elements necessary to obtain an RNA molecule of the general structure depicted in Fig. 1 after in vitro transcription and hybridization,
wherein the loop-sequence-element have the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference, such that double stranded RNA molecules can be obtained after cleavage and digestion with RNase T1.

The present invention thus also relates to a combination, obtainable by:
a. Providing at least one first DNA molecule comprising in the 5'-3' direction as repeating units a nucleic acid sequence with the following elements:
   5' - [(target-sequence-element) - (loop- sequence-element)]ₖ-3', with k being an integer >1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,
b. Providing at least one second DNA molecule comprising in the 5'-3' direction in a repetitive manner a nucleic acid sequence with the following elements:
   5' - [(target-sequence-element)_{rc} - (loop-sequence-element)]ₗ-3', with l being an integer >1 and having the same value as k in the first DNA molecule,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,
   wherein the target-sequence-elementsrc counted from the 3' end in the repeating units of said second DNA molecule are the respective reverse complement of the target-sequence-elements counted from the 5' end in the repeating units of said first DNA molecule, and wherein the loop-sequence-elements in the repeating units of said second DNA molecule are not reverse complements of the loop-sequence-elements in the repeating units of said first DNA molecule,
c. *In vitro* transcribing said at least one first and at least one second DNA molecules using an RNA polymerase to obtain corresponding at least one first and at least one second RNA molecules,
d. Hybridizing said at least one first and at least one second RNA molecules of step c. to obtain a double stranded RNA molecule of the general structure depicted in Fig. 1,
e. Digesting the double stranded RNA molecule obtained in step d. with RNase T1, which is capable of preferentially recognizing and cleaving the single stranded loop-sequence-elements over the hybridized double stranded sections of the double stranded RNA obtained in step d. thereby removing single stranded RNA loops,
wherein the sequences of the loop-sequence-elements are selected such that double stranded RNA molecules are obtained by recognition, cleavage and digestion of the single stranded loop-sequence elements by RNase T1 in step e. thereby removing single stranded RNA loops, wherein each strand of said resulting double stranded molecules has a length of 15 to 30 nucleotides, wherein said resulting double stranded RNA molecules are capable of target-specific RNA interference of at least one target gene RNA.

In a fourth aspect the present invention relates to a combination or a kit of at least one DNA molecule, which upon in vitro transcription, hybridization and digestion with RNase T1 according to claim 1 is capable of providing double stranded RNA molecules, wherein each strand of said double stranded molecules has a length of 15 to 30 nucleotides and wherein said different double stranded RNA molecules are capable of target-specific RNA interference of at least one target gene, wherein said at least one DNA molecule has the sequence elements necessary to obtain an RNA molecule of the general structure depicted in Fig. 2 after in vitro transcription and hybridization,
wherein the loop-sequence-element have the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference, such that double stranded RNA molecules are obtained after digestion with RNase T1, which is capable of preferentially recognizing, cleaving and digesting the single stranded loop-sequence-elements over the hybridized double stranded sections of the double stranded RNA thereby removing single stranded RNA loops.

The present invention thus relates to at least one DNA molecule, obtainable by:
a. Providing at least one first DNA molecule comprising in the 5'-3' direction as repeating units a nucleic acid sequence with the following elements:
   5' - [(target-sequence-element) - (loop- sequence-element)]ₖ - (target-sequence-element) - (loop- sequence-element)ₕₚ - [(target-sequence-element)_{rc} - (loop-sequence-element)]ₗ-3',
   with k being an integer >1,
   with l being an integer >1 and being the same as 1,
   with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
      with X being A and m being an integer of 1,
      with Y being A or T and n being an integer of 1, 2, or 3, and
   with X and Y being selected such that there is no hybridization of two molecules consisting just of the loop-sequence-element and wherein the loop-sequence element does not match a sense or antisense sequence in said at least one target gene of RNA interference,,
   wherein the (loop sequence element)ₕₚ is of sufficient length to allow for a hairpin structure enabling a self-hybdrization pattern depicted in Fig. 2, with the target-sequence-element_{rc} being a continuous sequence of 15 to 30 desoxyribonucleotides,
   wherein the target-sequence-elementsrc counted from the 3' end are the respective reverse complement of the target-sequence-elements counted from the 5' end,
   wherein the loop-sequence-elements following the (loop sequence element)ₕₚ are not reverse complements of the loop-sequence-elements preceeding the in the repeating units of said second DNA molecule,
b. *In vitro* transcribing said at least one first DNA molecules using an RNA polymerase to obtain corresponding at least one first RNA molecule, which upon hybridization provides the general structure depicted in Fig. 2,
c. Digesting the double stranded RNA molecule obtained in step b. with RNAse T1, which is capable of preferentially recognizing, cleaving and digesting the single stranded loop-sequence-elements over the hybridized double stranded sections of the double stranded RNA obtained in step b. thereby removing single stranded RNA loops,
wherein the sequences of the loop-sequence-elements are selected such that double stranded RNA molecules are obtained by recognition, cleavage and digestion of the single stranded loop-sequence elements by an RNase in step c., wherein each strand of said resulting double stranded molecules has a length of 15 to 30 nucleotides and wherein said resulting double stranded RNA molecules are capable of target-specific RNA interference of at least one target.

As regards the structure and elements of the DNA molecules to be used for the third and fourth aspects as well as their preferred embodiments, reference is made to the same considerations as laid out above for the methods forming the first and second aspects as well as the preferred embodiments thereof. Thus the considerations mentioned for the number of targest sequence elements, the number of loop sequence elements, their positioning, etc.

It is to be understood that such combinations, kits and DNA molecules may be provided in a form where they additionally comprise components that will allow performing the methods in accordance with the invention. These components include inter alis an RNA polymerase, a buffer for in vitro transcription, nucleotides for in vitro transcription, means for purifying and isolating the in vitro transcribed RNA molecules, a buffer for hybridization, Rnase T1, means for purifying and isolating the obtained siRNAs and written instructions for performing methods in accordance with the invention.

In a fifth aspect the present disclosure _{[S4]}relates to the use of any method as described herein, any kit as described herein or any template molecule for producing siRNA pools.

It is to be understood that the various steps of the methods, kits etc. in accordance with the invention such as in vitro transcription, hybdridization, RNase digest etc. can be performed as it is know to the skilled person from standard textbooks. The skilled person thus will be able to readily determine how to clone the template molecule in vectors for propagation, which promoter and termination sequences have to be used, and how hybridization can be performed. The same applies to purification steps of the in vitro transcribed RNAs or the siRNAs after the RNase digest.

As regards the specific steps that need to be undertaken for performing the present invention, some general exemplary teaching is provide in the following. Specific exemplary embodiments are then mentioned in the example section.

### Design of templates for in vitro transcription

DNA templates for in vitro transcription can be generated by gene synthesis as offered by multiple commercial providers (i.e. Geneart). For each siRNA pool, which is produced according to the first or third aspect of the invention at least two template DNA constructs are required: one for the sense RNA strand and one for the antisense RNA strand. If siRNA pools are produced according to the second or fourth aspect of the invention at least one template DNA constructs is required, which however has to also implement the sequences for the sense and antisense RNA strand. Such a single DNA template will in addition comprise a hairpin look sequence element ((loop-seq.el)ₕₚ of Fig. 2). The invention will however be discussed for the first or third aspect of the invention. The considerations mentioned in this context aplly *mutatis mutandis* to the second and fourth aspect.

The templates are composed of the following, minimal parts (see Fig. 1 and 3): 1. A minimal RNA polymerase promoter sequence (for instance the viral 19base T7 promotor sequence) at the 5' end of the template construct. 2. variable, target gene specific sequence fragments reverse complementary between the sense and antisense template constructs (target sequence elements corresponding to (tar.seq.el.) and (targ.seq.el_{rc}) of Fig. 1). 3. Non gene specific loop sequences, non reverse complementary between the sense and antisense template constructs (corresponding to (loop-seq-el.) of Fig.1). Variable, gene specific, reverse complementary and non gene specific, non reverse complementary loop sections are alternating, which may start and end with a loop section. For convenient and cost efficient production of the template DNA, the minimal template described above can be cloned into a suitable bacterial high cloning plasmids using e.g. two different, terminal restriction sites for linearization or excision from the plasmid backbone. The plasmid should not contain the RNA promoter used in template.

The variable, gene specific, reverse complementary sequence sections can be selected from the cDNA sequence of the targeted gene applying any of the published siRNA selection algorithms or custom selection criteria (see e.g. Vienna RNA WebServer at http://rna.tbi.unvie.ac.at (see. Fig. 4), particularly the RNAxs Server; other tools include the siDesign Center (Dharmacon/Thermo: http://www.dharmacon.com/designcenter/DesignCenterPage.aspx) and the online siMAX™ Design Tool (Eurofins MWG: http://www.eurofinsdna.com/products-services/sirna/sima-design.html). To trigger an RNAi response, they should have a length between 19 and 28 base pairs even though the synthesis method would also allow the use of shorter and longer sequence sections.

Non gene specific, non reverse complementary loop sections must be optimized for minimal base pairing between the product sense and antisense RNA strands to allow the recognition and cleavage by RNAseT1. Loop sections as short as 5 base pairs containing guanine nucleotide residues at specific positions were found to be sufficient for efficient and specific cleavage by RNAseT1. Longer loop sections are functional and possibly superior for RNAseT1_{[S5]}. For example one may use RNAfold Server and RNAcofold Server of the Vienna RNA WebServer (see. Fig.4) to identify optimal loop sequence elements based on their minimal free energy and base pairing propensity. Reference is made to the above explanations in this context.

The use of RNAseT1which shows a high specificity for cleaving 3' of guanine ribonucleotide residues allows the generation of siRNA fragments with 3' overhangs as described in Fig. 5. To that end, guanine residues must be placed at specific positions in the loop section. Blunt ends can be generated by placing guanine residues at the 3' and 5' terminal positions of the loop sections.

### DNA template preparation

DNA templates for in vitro transcription designed as described above can be generated by gene synthesis as offered by multiple commercial providers (i.e. Geneart). DNA templates, cloned in plasmid vectors must be either linearized by restriction digest at the 3' end of the template construct (opposite end of the T7 promotor). Alternatively, the template can be excised by restriction digest and purified by preparative agarose gel electrophoresis and gel extraction.

### In vitro transcription

The transcription of sense and antisense RNA strands in separate in vitro transcription reactions allows an individual quality control, purification and quantification of both strands for a precise use of equal amounts of both strands in the downstream annealing reaction (see below). Alternatively, both strands could be generated in one single in vitro transcription reaction using a mixture of sense and antisense DNA template.

Suitable viral RNA polymerases as RNAs polymerase T3 and T7 are offered by multiple commercial reagent providers or can be generated from bacterial expression clones and protein purification by established protocols. Optimal reaction conditions are described in multiple publications. High yields of RNA, exceeding concentrations of 1 µg/µl can be obtained from comparably cheap reagents, making in vitro transcription an extremely cost effective way of RNA synthesis.
To remove residual non incorporated nucleotides and buffer components, in transcriptions reactions can be purified by size exclusion chromatography, using gel filtration resins of suitable pore size (G25 or50, S200-400). The obtained purified RNA product can be quantified by spectro-photometry and visualized by denaturing polyacrylamide gel electrophoresis for quality control. For efficient downstream single strand RNA annealing, the full length RNA transcript should be the main product of the reaction (> 90% of total RNA).

### Hybridization/Single strand RNA annealing

Equal amounts of both single strand RNAs are hybridized in 1x annealing buffer (30mM Hepes pH 7.4, 100mM KAc, 2mM MgAc) by a melting and annealing program in a conventional PCR cycler. For efficient and precise annealing of both strands, the reaction is first heated for 2 min to 98'C and then slowly cooled to 4'C. As RNA is succeptible to hydrolysis at high temperatures, the cooling process can be started with a faster cooling step from 98'C to 80'C (1'C/s), followed by a slow cooling step in the range from 80'C to 55'C (0.1'C/s) for optimal hybridization. The mixture can then be rapidly cooled to 4'C (1'C/s) and kept on ice for downstream use. As both strands share perfect base pair match over the 19b coding sections, single strand annealing is a robust process with multiple possible annealing programs.

### Ribonuclease digest

As RNAsT1 has a high specificity for guanosine residues in single stranded RNA, a range of enzyme and substrate concentrations as well as multiple buffer constitutions are possible. The concentration of RNAseT1, required for a complete digest of the long dsRNA substrate is dependent on the size and sequence of the single strand loops, which determine the accessibility of the guanosine residues. 3 loop sequences were tested (5' to 3' direction): 1): AGTTG, 2): AGTTTG, 3): TGTTTG. For loop 1) with 5 nucleotides, 12ug of dsRNA were completely digested to 21mer dsRNA fragments by 40 units of RNAsT1 in 45 minutes. For the 6 nucleotide loop 2) the same amount of dsRNA was completely digested to 21mer-dsRNA fragments under identical conditions in 10 minutes. The loop sequence 3), where 4 G-T base pairing reduces the single strand region to 2 nucleotides and masks the guanosine residue, no satisfactory conditions were found (see for detail below), the results well matching the base probability prediction of the RNAcofold Server programe of the Vienna RNA WebServer and thus accessibility by RNase T1. The length of the fragments, generated by RNAseT1 from the above described long dsRNA precursor was identical to commercial 21-mer siRNAs as determined by 20% PAA electrophoresis.

The increased length are due to the 2 5'-overangs, left from the loop section (see Fig. 3). Very high concentrations of RNAseT1(> 10fold of conditions described above) also cause a digest of the double strand RNA. However, there seems to be a sufficiently large window of suitable saturating enzyme concentrations that yield the same, complete digest with little or no detectable smaller degradation products (< 21b).

### siRNA purification

Even under saturating enzyme conditions as described above, there are small amounts of larger dsRNA fagments only detectable by PAA gel electrophoresis (20%) and sensitive detection methods. Those larger fragments however can trigger an interferon response in higher vertebrate cells (as human, mouse, or other cell) leading to apoptosis and cell death. It is therefore advisable, that all dsRNA fragments larger 30 bp are completely removed. For low throughput and proof of concept experiments as described below, this can be achieved by preparative PAA gel electrophoresis, excision of the respective dsRNA bands followed by elution and precipitation of the 21mer dsRNA fragments and optionally size exclusion chromatography. For high throughput experiments the same effect can be achieved by ion pair reverse phase chromatography.

### siRNA pool transfection and demonstration of functionality

Standard human tissue culture cells (HeLa, A549 or other cells) can be seeded in multititer plates at suitable, subconfluent densities. For Hela or A549, 1000cells/well are e.g. suitable for 384well format. RNAseT1 generated siRNA pools as well as standard, commercial siRNAs as controls are transfected in a final concentration of 10nM using a standard commercial transfection reagent as Oligofectamine RNAiMax (Invitrogen). For a 384well format with cells seeded in 30 µl of complete medium, 0.06 µl/well of Oligofectamine RNAiMax should show efficient transfection. The complex of dsRNA and reagent, formed according to the instructions of the reagent provider, can then be added to the cell suspension at the time point of cell seeding or on the adherent cells 24h post seeding. The gene silencing effect of the tested dsRNA can be assessed by phenotypic analysis, RT-PCR measuring specific mRNA concentration or western blotting, measuring specific protein concentration. In all cases, the gene specific effect of a gene targeting high complexity pool or siRNA is compared to a negative control siRNA. As proof of concept, a RNAseT1 generated, complex siRNA pool of 14siRNAs targeting AURKB was compared to an experimentally validated, highly active commercial siRNA (AMbion/life technology) against AURKB (see below). The gene specifc, phenotypic effect of AURKB knock down as assessed by phenotypic analysis was most clear and pronounced in the cells, transfected by the AURKB complex siRNA pool.

### Cell culture and transfections

Hela cells were cultivated in Dulbeco's modified eagles medium substituted with 10% FCS and Penicillin/Streptomycin. siRNA transfections were done using Lipofectamine RNAiMax (Life Technologies) according to the manufacturer's protcol. Cells were harvested 24h or 48h after transfection.

### qPCR and Western blot

RNA was isolated 24h after transfection followed by cDNA synthesis and qPCR. The following Primers were used:
PolG forward: 5'-TTCCAGGACCTGATGCAGTA-3' (SEQ ID No.: 344)
PolG reverse: 5'- ACAGGCAGGTAGGAGACACC-3'(SEQ ID No.:345)
Scyll forward: 5'-CTGGAGGAAGTGGAGAAGGA-3' (SEQ ID No.:333)
Scyll reverse: 5'-TCAGCTTGGAGGTGAGTGAG-3'(SEQ ID No.:334)
Mad2 forward: 5'-AGATGACAGTGCACCCAGAG-3'(SEQ ID No.:338)
Mad2 reverse: 5'-TCCAACAGTGGCAGAAATGT-3' (SEQ ID No.:339)
GAPDH forward: 5'-ATGGGTGTGAACCATGAGAA-3'(SEQ ID No.:335)
GAPDH reverse: 5'-GTGCTAAGCAGTTGGTGGTG-3'.(SEQ ID No.: 336)

For Western blot analysis, cells were harvested and lysed in NET buffer (50 mM Tris pH 7.4, 150 mM NaCl, 5 mM EDTA, 0.5% NP40, 10% glycerol) 48h after transfection. Proteins were separated by SDS-PAGE followed by semi-dry electro blotting. The following antibodies were used: polyclonal anti-Mad2 (Bethyl Laboratories) at a dilution of 1:5000 and a monoclonal mouse anti beta-actin antibody (clone AC15 from Abcam) at a dilution of 1:5000 in TBS-Tween with 5% milk-powder. Fluorescently labeled IRDye 800 CW antibodies were used as secondary antibodies (Li-COR). Western blots were imaged with an Odyssey Fluorescence scanner (Li-COR).

### Dual luciferase assay

To generate the off-target reporter construct, a modified pMIR dual luciferase reporter plasmid (Beitzinger et al., (2007), RNA Biol, 4 was used. The 3'UTR of Mad2 was amplified by PCR and cloned into the corresponding SacI and PmeI sites of pMIR. The following primers were used:
Mad2-forward: 5'-GATCGAGCTCGGATGACATGAGGAAAATAA-3' (SEQ ID No.:346)
Mad2-reverse: 5'-GATCGTTTAAACAAGACAAATTTAAAACAAACTTA-3' (SEQ ID No.:347)

Hela cells were transfected in 96 well plates with 1, 3 or 10 nM siRNA concentrations and 20 ng pMIR Mad2 3'UTR plasmid using Lipofectamine 2000 (Life Technologies). Cells were harvested and lysed in passive lysis buffer (Promega) 24h after transfection. Firefly/renilla luminescence ratios were normalized to corresponding ratios of the empty pMIR plasmid.

### Co-immunoprecipitation and Northern blotting

Hela cells were transfected with 10 nM siPools and lysed in NET buffer (50 mM Tris pH 7.4, 150 mM NaCl, 5 mM EDTA, 0,5% NP40, 10% glycerol) 48h after transfection. Lysates were used for Ago2-siRNA co-immunoprecipitation. Protein-G sepharose beads (GE) were pre-incubated with monoclonal anti-Ago2 (11A9) antibody (Rudel et al., (2008), RNA, 14, 1244-1253). Lysates were incubated with the Ago2 antibody-coupled beads for 4 h at 4°C. Immunoprecipitations were subsequently washed with NET buffer followed by proteinase K digestion and phenol/chloroform extraction of bound RNAs. Northern blot was performed as described earlier (Pall et al., Nat Protoc (2008), 3, 1077-1084). As probes for siRNA detection, antisense DNA oligos for the corresponding off-T siRNAs were used:
PolG Pool #1 siRNA off-T guide: 5'-GGGTGAAGCGCTGGATATT-3' (SEQ ID No.:348)
PolG Pool #1 siRNA off-T passenger: 5'-AATATCCAGCGCTTCACCC-3' (SEQ ID No.:349)
Scyll Pool #1 siRNA off-T guide: 5'-GCCTCATCCACAACAATGT-3' (SEQ ID No.:350)
Scyll Pool #1 siRNA off-T passenger: 5'-ACATTGTTGTGGATGAGGC-3' (SEQ ID No.:351)

### Examples

### Example 1 - preparation of siRNA pool comprising 14 siRNAs against AURKB

### Template design

21 base pair siRNA sequences targeting human AURKB were obtained from different commercial siRNA providers. AUKRB exists as a long and short isoform. The nucleic acid sequence of the long isoform has SEQ ID No. 57 (Genbank accesion no. NM_004217.3), the amino acid sequence of the long isoform has SEQ ID No. 58 (Genbank accesion no. NP_004208.2). The nucleic acid sequence of the short isoform has SEQ ID No. 59 (Genbank accesion no. NM_001256834.1), the amino acid sequence of the short isoform has SEQ ID No. 60 (Genbank accesion no. NP 001243763). The following 14 sequences, all which target both isoforms, were chosen:

**--Table 1:**

| | Complete sequence | | | |
|---|---|---|---|---|
| siRN A# | sense | SEQ ID No.: | Reverse complement antisense | SEQ ID No.: |
| 1 | AAGGCAAGUUUGGAAACGUTT | 1 | ACGUUUCCAAACUUGCCUUTG | 15 |
| 2 | GAUGCUCUAAUGUACUGCCTT | 2 | GGCAGUACAUUAGAGCAUCTG | 16 |
| 3 | GAAGAGCUGCACAUUUGACTT | 3 | GUCAAAUGUGCAGCUCUUCTG | 17 |
| 4 | UCUUAACGCGGCACUUCACTT | 4 | GUGAAGUGCCGCGUUAAGATG | 18 |
| 5 | UCGUCAAGGUGGACCUAAATT | 5 | UUUAGGUCCACCUUGACGATG | 19 |
| 6 | CCAAACUGCUCAGGCAUAATT | 6 | UUAUGCCUGAGCAGUUUGGAG | 20 |
| 7 | GGUGAUGGAGAAUAGCAGUTT | 7 | ACUGCUAUUCUCCAUCACCTT | 21 |
| 8 | CCUGCGUCUCUACAACUAUTT | 8 | AUAGUUGUAGAGACGCAGGAT | 22 |
| 9 | GUCCCAGAUAGAGAAGGAGTT | 9 | CUCCUUCUCUAUCUGGGACTT | 23 |
| 10 | GGUCCUCUUCAAGUCCCAGTT | 10 | CUGGGACUUGAAGAGGACCTT | 24 |
| 11 | CCAACAUCCUGCGUCUCUATT | 11 | UAGAGACGCAGGAUGUUGGGA | 25 |
| 12 | GACAAUGUGUGGCACCCUGTT | 12 | CAGGGUGCCACACAUUGUCTT | 26 |
| 13 | GCAGAGAGAUCGAAAUCCATT | 13 | UGGAUUUCGAUCUCUCUGCGC | 27 |
| 14 | GCCAGAAAAUCUGCUCUUATT | 14 | UAAGAGCAGAUUUUCUGGCTT | 28 |

For each of those sequences, the two 3' overhang nucleotides were removed to obtain the following 19b core sequences resulting in the target sequence elements ((tar.seq.el.) in Fig. 1) for SEQ ID Nos.: 29 to 42 and the reverse complement target sequence elements ((tar.seq.el._{rc}) in Fig. 1) for SEQ ID Nos.: 43 to 56.

**Table 2:**

| | 19 bp core sequence without 3' overhang | | | |
|---|---|---|---|---|
| siRN A# | sense | SEQ ID No.: | Reverse complement antisense | SEQ ID No.: |
| 1 | AAGGCAAGUUUGGAAACGU | 29 | ACGUUUCCAAACUUGCCUU | 43 |
| 2 | GAUGCUCUAAUGUACUGCC | 30 | GGCAGUACAUUAGAGCAUC | 44 |
| 3 | GAAGAGCUGCACAUUUGAC | 31 | GUCAAAUGUGCAGCUCUUC | 45 |
| 4 | UCUUAACGCGGCACUUCAC | 32 | GUGAAGUGCCGCGUUAAGA | 46 |
| 5 | UCGUCAAGGUGGACCUAAA | 33 | UUUAGGUCCACCUUGACGA | 47 |
| 6 | CCAAACUGCUCAGGCAUAA | 34 | UUAUGCCUGAGCAGUUUGG | 48 |
| 7 | GGUGAUGGAGAAUAGCAGU | 35 | ACUGCUAUUCUCCAUCACC | 49 |
| 8 | CCUGCGUCUCUACAACUAU | 36 | AUAGUUGUAGAGACGCAGG | 50 |
| 9 | GUCCCAGAUAGAGAAGGAG | 37 | CUCCUUCUCUAUCUGGGAC | 51 |
| 10 | GGUCCUCUUCAAGUCCCAG | 38 | CUGGGACUUGAAGAGGACC | 52 |
| 11 | CCAACAUCCUGCGUCUCUA | 39 | UAGAGACGCAGGAUGUUGG | 53 |
| 12 | GACAAUGUGUGGCACCCUG | 40 | CAGGGUGCCACACAUUGUC | 54 |
| 13 | GCAGAGAGAUCGAAAUCCA | 41 | UGGAUUUCGAUCUCUCUGC | 55 |
| 14 | GCCAGAAAAUCUGCUCUUA | 42 | UAAGAGCAGAUUUUCUGGC | 56 |

Sense and antisense core sequences of all 14 constructs of Table 2 were concatenated to two continuous sequences, in which each siRNA sequence was separated from adjacent sequences by an identical loop sequence of 5 bases (Figure 3).
- The 5 base loop sequence 5'-AGTTG-3' was selected for the following features :
   Complete mismatch with its own reverse complement sequence, showing minimal binding free energy in RNA folding prediction (Vienna RNAfold server, http://rna.tbi.univie.ac.at/cgi-bin/RNAfold.cgi)
- Guanin base ("G") at position 2 and 5 for RNAseT1 cleavage 3' of second loop nucleotide and 5' of first siRNA nucleotide.

Fig. 5 shows the general sequence structure of the DNA template (upper box) and the in vitro transcribed RNAs (lower box) for a single siRNA target sequence element flanked by non base pairing loop sequence elements. Positions giving rise to final siRNA product are in capital letters, excised loop sequence (tttg) in lower case. Arrowheads indicate positions of RNAseT1 cleavage, 3' of non base pairing G nucleotides. Base-pairing nucleotides indicated by "I" between sense and antisense strand. Mature siRNA after RNAseT1 digest is highlighted by blue background color.

For in vitro transcription of the construct, the minimal T7 RNA polymerase promoter sequence 5'-TAATACGACTCACTATAGG-3' was placed 5' of the concatenated siRNA-loop sequence for both sense and antisense constructs. For cloning into suitable vector sequences, a HindIII restriction site (5'-AAGCTT-3') was placed 5' of the T7 RNA polymerase promotor and a EcoRI site (5'-GAATTC-3') 3' of the terminal siRNA sequence (Fig. 3). The resulting template is shown in Fig. 6.

### Template preparation

The final template sequences, submitted for Gene Synthesis, were as follows:
Sense Template (SEQ ID No. 61):
Antisense Template (SEQ ID No. 62):

Sense and antisense RNA templates were then obtained as plasmid DNA, cloned into Genearts standard pMA cloning vector using the HindIII (5') and EcoRI(3')restriction site. In detail, 100 µl of template vector preparation were subjected to a HindIII/EcoRI double digest under standard conditions indicated by the reagent provider (New England Biolabs). The 400base insert fragments were separated from the vector by agarose gel electrophoresis and recovered from the gel using a commercial Gel Extraction kit (Qiagen). Purified template DNA was eluted in nuclease free water.

### In Vitro Transcription and RNA purification

Sense and antisense RNA strands were transcribed from the corresponding purified DNA templates by a commercial T7 RNA polymerase according to the instructions of the provider (NEB). The enzyme concentration was set to 5U/ul (10 ul of NEB enzyme in 100 ul reaction) which is presumably close to saturation. This concentration was used to transcribe 50ng/µl linearized pMA vector (3kb) or 3µg/ul excised template (400bp). These conditions may not be saturating. For maximal RNA yield, nucleotide concentrations was elevated to 4mM (per nucleotide). The transcription reaction was terminated by the addition of RNAse free DNAse (Ambion/life technology)) to remove the DNA template. For downstream processing steps, ssRNA from reactions with identical template were pooled. The obtained full length single strand RNA was separated from non-incorporated nucleotides and short RNA fragments by preparative denaturing 6% PAGE. The bands containing the full length single strand RNA were excised from the gel, eluted over night at 4'C in 1x annealing buffer with 0.1mM EDTA and precipitated with 300mM NaAc pH 5.4 and 2.5 volumes of cold 100% EtOH. After washing with 70% EtOH, the single strand RNAs were resuspended in 50ul 1x annealing buffer. Concentrations of the purified single strand RNAs as determined by spectrophotometry were 0.7 µg/µl for the sensen strand and 1.1ug/ul for the antisense strand. For analytical purpose, RNA obtained from IVT reactions was also purified from buffer components by phenol chloroform extraction and/or gel filtration chromatographie using S300HR spin columns (Amersham/GE). Quality and concentration of the purified RNA was assessed by polyacrylamide gel-electrophoresis on a denaturing 5% TBE gel with 8M urea. Some of the results of the in vitro transcription are depicted in Fig 7.

During preliminary optimization it was found that in vitro transcription for 4h at 37'C gave reasonable high yield (∼1 µg/µ RNA with >90% full length RNA) land purity. It was observed that long incubation (as over night) increase yield but also degradation products. It seems that an incubation between 4 or 8 hours may be optimal.

### Hybridization/single strand annealing RNAse T1 digest

Equal amounts of sense and antisense RNA strands were annealed in a final, total concentration of 0.65ug/ul in 1x annealing buffer (30mM Hepes pH 7.4, 100mM K Ac, 2mM MgAc)). For efficient and reproducible hybridization of the RNA strands, the annealing reaction was performed in a thermocycler applying a custom program. Briefly, after an initial 2 minute melting step at 98'C, samples were slowly cooled to 4'C using a faster ramp speed of -1'C/s for the range from 98 to 80'C to protect RNA integrity and a slow ramp speed of -0.1'C /s between 80 and 55'C for optimal hybridization.

For analytical purpose, different ratios of sense and antisense single strand RNA were hybridized under conditions described above and analyzed by 1.1% agarose gel electrophoresis. For all ratios, the hybridization gave rise to a major band with a shift up in molecular weight as compared to the bands of the single strand RNAs, indicating that at least a large fraction of the single strand RNA had successfully hybridized to their corresponding double strand molecules. Some results of the annealing step are depicted in Fig. 8.

### RNAse T1 digest and siRNA purification

Annealed double strands RNA of 341 base pair length were digested to a pool of 14 different 21base pair siRNAs by use of RNAseT1, a ribonuclease cleaving single strand RNA 3' of Guanosine ribonucleotide residues. Commercial RNAseT1 (Fermentas/Thermo) was applied in a concentration of 0.5 units enzyme/ul to digest 13ug of RNA in a volume of 80ul . Reaction buffer conditions were chosen as suggested by the enzyme provider (50 mMTris/HCL pH 7.5, 2mM EDTA.). The reaction was incubated for 45min at 37'C and directly loaded on a preparative 20% PAA gel to separate the siRNAs from residual longer dsRNA species. The band, corresponding to the 21mer siRNA fragments was visualized by UV-shadowing, cut out of the gel and eluted over night in RNA gel elution buffer (1x annealing buffer , 0.1mM EDTA). To obtain a 10uM siRNA solution, the eluted RNA was precipitated in 2.5 volumes of ethanol and re-dissolved in the corresponding volume of 1x annealing buffer.

For analytical purpose, 4 µg of dsRNAs purified by different methods as described above were digested with RNAseT1 enzyme preparations from two providers ( highly purified enzyme from Ambion /Life Technologies, 1unit/µl; recombinant enzyme from Fermentas/Thermo 1000 units/µl) in concentrations ranging from 0.1 to 0.8 units/µl using two different buffer systems (Ambions "structure buffer": 10mM Tris/Cl pH 7.0, 100mM KCl, 10mM MgCl²; Fermentas reaction buffer: 50mM Tril/Cl pH 7.5, 2mM EDTA). Of the 20 µl reactions, 6 µl aliquots were taken after 10, 45 and 90 minutes of incubation at 37'C and analyzed by 20% native PAGE. The results, some of which are shown in figure 9, indicated that neither the source of the enzyme nor the purity of the dsRNA substrate affected the quality and efficiency of the digest. Critical parameters were the concentration of the enzyme and dsRNA substrate, the buffer conditions and the duration of the reaction. Of the two buffers compared, the "structure buffer" from Ambion showed a reproducibly reduced enzyme processivity, presumably due to the presence of 10mM MgCl₂ which had been shown to inhibit RNAseT1 activity. 0.8 units/ul of RNAseT1 in absence of MgCl₂ cleaved 1 µg gel purified dsRNA substrate in 45 minutes to 21base pair dsRNA fragments with only trace amounts of dsRNA longer or shorter than 21 base pairs. None of the tested conditions lead to complete digest of the long dsRNA substrate to 21base pair fragments within 10 minutes. For an incubation time of 90 minutes, a concentration of 0.2 units/µl of RNAseT1 in absence MgCl₂ was sufficient to cleave 1 µg of substrate RNA to 21base pair fragments.

### Transfection and demonstration of functionality

HeLa cells were seeded in 384 well multi-titer plates in 30ul/well of DMEM supplemented with 10%FCS and a cell seeding density of 1000 cells/well. 24h post seeding, cells were transfected with the enzymatically produced pool of 14 different siRNAs targeting human AURKB, a standard, commercial siRNA targeting human AURKB (Ambion/life technology, siRNA ID s495) and two negative control siRNAs targeting no human gene. The sense sequence of siRNA ID s495 has SEQ ID No. 63, the antisense sequence of siRNA ID s495 has SEQ ID No. No. 64. The sense sequence of the first negative control siRNA has SEQ ID No. 65, the antisense sequence of the first negative control siRNA has SEQ ID No. 66. The sense sequence of the second negative control siRNA has SEQ ID No. 67, the antisense sequence of the second negative control siRNA has SEQ ID No. 68. As transfection reagent, Lipofectamine RNAiMax (Invitrogen/life technology) was used in a concentration of 0.06 µl/well following the instructions of the provider. Final siRNA concentration in the cell culture medium was 10nM.

Gene specific inhibition of gene expression was demonstrated by western blotting against human AURKB using standard methods (see Fig. 10) The 14 siRNA pool targeting AURKB showed equal, almost complete degradation of AURKB protein as the validated AURKB siRNA. Both negative control siRNAs showed no reduction of AURKB protein as compared to the non transfected medium control. Phenotypic response was analyzed by light microscopy (see Fig. 11. The 14 siRNA pool targeting AURKB showed an at least equal or even stronger AURKB phenotype than the validated AURKB siRNA, obvious in a strongly decreased cell number and dramatically increased cell size with multiple cell nuclei. Cells transfected with a negative control siRNA showed reduced cell number but no increased cell size as compared to non transfected cells.

### Example 2 - testing different loop sequence elements for RNaseT1 cleavage

The same siRNA pool was then generated, however with an AGTTTG or TGTTTG loop sequence element instead of the AGTTG loop sequence element of Example 1. The miminal free energy (MFE) structure prediction as performed by RNAfold indicates that the AGTTTG loop sequence element should havee the largest single strand RNA region granting best RNAseT1 accessibility to both guanosine ribonucleotide residues within the loop sequence element (figure 12 and 15). The AGTTG loop sequence element is predicted to have the second best RNAseT1 accessibility followed by the TGTTTG loop sequences, which, due to guanosine-thymidine base bairing shows the shortest stretch of accessible single strand RNA(see Fig. 13 , 14 and 15).

As shown in Fig. 16, the cleavage efficiency of the three different loop sequence elements shows good correlation with the length of the predicted single strand RNA region within the loop sequence: For 3 different dsRNA preparation with different purification methods applied, the AGTTTG loop sequence with 6 non base pairing ribonucleotides reaches complete cleavage by RNAseT1 to 21mer dsRNA fragment within an incubation time of 10 minutes at 37'C in absence of MgCl₂ (Fig 16 and 17).

Under identical reaction conditions, an equal amount of long dsRNA substrate with AGTTG loop sequence elements is only partially cleaved to a range of different dsRNA fragment sizes (Fig.16). An optimization of the reaction conditions for the cleavage of the AGTTG loop sequence elements is shown in Fig. 9.
The dsRNA substrate with TGTTTG loop sequence elements, predicted to have only 2 non baise pairing ribonucleotide residues is largely resistant to RNAseT1 cleavage under identical reaction conditions (Fig. 16).

### Example 3 - testing complex siRNA pools for off-target effects

The following experiment describes the improvement of using complex siRNA pools on off-target effects. In order to determine off-target effects of siRNAs, it was crucial to identify siRNAs, which in addition to silencing an on-target gene are known to give an off-target effect on an identified off target gene. Sigoillot et al., Nat. Methods, 9(4), 363-366 (2012) describe a genome wide RNAi screen for new factors of the spindle assembly checkpoint in which they identify multiple siRNAs by off target effect based phenotypes. Amongst others, siRNAs targeting the genes Scyll, PolG, Ern1 and Traf5. were shown to suppress the expression of the gene Mad2 by an off-target effect.

In order to determine the efficiency of complex siRNA pools in achieving optimal on-target gene silencing with minimal off target effects , complex siRNA pools were generated for PolG and Scyl1. For each gene, four complex siRNA pools (labeled as Pool 1, Pool 2, Pool 3 and Pool 4) were generated with each Pool comprising 15 siRNAs. For both PolG and Scyl1, Pool 1 comprised as one of the 15 siRNAs an siRNA which is known from Sigoillot et al., *vide supra* to give an off-target effect on Mad2. Further, for both PolG and Scyll, Pools 1 to 4 were combined to give a Pool of 60 siRNAs.

For both PolG and Scyl1, the effects of Pools 1 and 4 and of the combined Pool comprising all 60 siRNAs were determined vs. the siRNAs for PolG and Scyl1 being known to give an off-target effect for Mad2. In addition, Pools 1 and 4 and the combined Pool comprising all 60 siRNAs were determined vs.pools comprising 4 siRNAs against PolG with one of the four siRNAs being the known siRNA for PolG or Scyl1 which is known to give an off-target effect for Mad2. These pools of four siRNAs are designated herein as "smart pools" and are described in more detail hereinafter.

The off-target effects on Mad2 were either determined by a Luciferase assay or by a cellular assay which are also described in more detail hereinafter.

### Materials and Methods

### Generation of complex siRNA pools for PolG

The complex siRNA pools for PolG were generated as described above in
Example 1. Thus, DNA ctemplates were prepared comprising the 15 target sequence elements for the ultimate siRNAs being interrupted by the loop sequence element 5'-AGTTTG-3' giving rise to a construct as schematically depicted in Fig. 5 and 6.

These DNA templates were cloned into the pMA cloning vector using the HindIII (5') and EcoRI (3') site, in vitro transcribed from the T7 RNA polymerase promoter, digested with RNAse T1 and the resulting complex siRNA was purified as described above.

The sequence for the siRNAs were chosen using the on-line siRNA design platform provided by Thermo "siDesign-Center". SiRNAs were designed against the coding sequence as well as the 3'UTR of POLG. For each pool of 15 siRNAs, siRNA sequences were selected to have a maximum of 7 nucleotide overlap.

The chosen target sequence elements were as follows:

**Table 3 - PolG Pool 1**

| | 19 bp core sequence without 3' overhang for PolG Pool 1 | | | |
|---|---|---|---|---|
| siRN A# | sense | SEQ ID No.: | Reverse complement antisense | SEQ ID No.: |
| 1 | CGAGCAAATCTTCGGGCAA | 69 | TTGCCCGAAGATTTGCTCG | 84 |
| 2 | GCGTCGAGCACCTGCAGAA | 70 | TTCTGCAGGTGCTCGACGC | 85 |
| 3 | GCCAGAAGTCCCAGAGGAA | 71 | TTCCTCTGGGACTTCTGGC | 86 |
| 4 | CTAAGAAGGTGAAGAAGGA | 72 | TCCTTCTTCACCTTCTTAG | 87 |
| 5 | AGGAGGAGTTTCAACAAGA | 73 | TCTTGTTGAAACTCCTCCT | 88 |
| 6 | CCACAGAGCTCCTGCCCAA | 74 | TTGGGCAGGAGCTCTGTGG | 89 |
| 7 | GCTTACTAATGCAGTTTAA | 75 | TTAAACTGCATTAGTAAGC | 90 |
| 8 | CAGGAAGAGTTTATGACCA | 76 | TGGTCATAAACTCTTCCTG | 91 |
| 9 | GATAATTGAACTCACCAAA | 77 | TTTGGTGAGTTCAATTATC | 92 |
| 10 | GGTGTGGACTACAGGACAA | 78 | TTGTCCTGTAGTCCACACC | 93 |
| 11 | CATTGTTGCTTGTTGGGTA | 79 | TACCCAACAAGCAACAATG | 94 |
| 12 | GGGTGAAGCGCTGGATATT | 80 | AATATCCAGCGCTTCACCC | 95 |
| 13 | CTGATGCAGTGCCCTAGAA | 81 | TTCTAGGGCACTGCATCAG | 96 |
| 14 | GGAAAGAATTAATGCTCTA | 82 | TAGAGCATTAATTCTTTCC | 97 |
| 15 | GCCCCAAAGTTCACATTAA | 83 | TTAATGTGAACTTTGGGGC | 98 |

The above 19b core sequences result in the target sequence elements ((tar.seq.el.) in Fig. 1) for SEQ ID Nos.: 69 to 83 and the reverse complement target sequence elements ((tar.seq.el._{rc}) in Fig. 1) for SEQ ID Nos.: 84 to 98.

SEQ ID Nos.: 80 and 95 refer to the siRNA sequence described in Sigoillot et al., *vide supra* as being specific for the target PolG and giving an off-target effect for Mad2.

**Table 4 - PolG Pool 2**

| | 19 bp core sequence without 3' overhang for PolG Pool 2 | | | |
|---|---|---|---|---|
| siRN A# | sense | SEQ ID No.: | Reverse complement antisense | SEQ ID No.: |
| 1 | CGGCACAACCCATTGGACA | 99 | TGTCCAATGGGTTGTGCCG | 114 |
| 2 | CCACAAAGCAAGGCCAGAA | 100 | TTCTGGCCTTGCTTTGTGG | 115 |
| 3 | GAGTCAGAAATGTTCAATA | 101 | TATTGAACATTTCTGACTC | 116 |
| 4 | CCATGAAGGACATTCGTGA | 102 | TCACGAATGTCCTTCATGG | 117 |
| 5 | GAGAGAGGTACAAAGAAGA | 103 | TCTTCTTTGTACCTCTCTC | 118 |
| 6 | GAAGAAGGAACCAGCCACA | 104 | TGTGGCTGGTTCCTTCTTC | 119 |
| 7 | CCATATGGCAAACGGTAGA | 105 | TCTACCGTTTGCCATATGG | 120 |
| 8 | CGGTAGAAGAACTGGATTA | 106 | TAATCCAGTTCTTCTACCG | 121 |
| 9 | CAAGGAAGTCACAGTGGAA | 107 | TTCCACTGTGACTTCCTTG | 122 |
| 10 | AAGATTCCTTCTAACTGAA | 108 | TTCAGTTAGAAGGAATCTT | 123 |
| 11 | GAATTCAGTGGGTTCAGAA | 109 | TTCTGAACCCACTGAATTC | 124 |
| 12 | GCAGAAGCCCCAAAGTTCA | 110 | TGAACTTTGGGGCTTCTGC | 125 |
| 13 | GCTCTGATGCAGTGCCCTA | 111 | TAGGGCACTGCATCAGAGC | 126 |
| 14 | AATTAATGCTCTAACGTGA | 112 | TCACGTTAGAGCATTAATT | 127 |
| 15 | CGTGATAAACCTGCTCCAA | 113 | TTGGAGCAGGTTTATCACG | 128 |

The above 19b core sequences result in the target sequence elements ((tar.seq.el.) in Fig. 1) for SEQ ID Nos.: 99 to 113 and the reverse complement target sequence elements ((tar.seq.el._{rc}) in Fig. 1) for SEQ ID Nos.: 114 to 128.

**Table 5 - PolG Pool 3**

| | 19 bp core sequence without 3' overhang for PolG Pool 3 | | | |
|---|---|---|---|---|
| siRN A# | sense | SEQ ID No.: | Reverse complement antisense | SEQ ID No.: |
| 1 | GCAGAGGTGCACAGACTTT | 129 | AAAGTCTGTGCACCTCTGC | 144 |
| 2 | GTGAGAACTTCCAGGACCT | 130 | AGGTCCTGGAAGTTCTCAC | 145 |
| 3 | GAGATGAAGAAGTCGTTGA | 131 | TCAACGACTTCTTCATCTC | 146 |
| 4 | CAGGAGAGAGGTACAAAGA | 132 | TCTTTGTACCTCTCTCCTG | 147 |
| 5 | AAGCTAAGAAGGTGAAGAA | 133 | TTCTTCACCTTCTTAGCTT | 148 |
| 6 | GCAGTGAGGAGGAGGAGTT | 134 | AACTCCTCCTCCTCACTGC | 149 |
| 7 | TAGAAGAACTGGATTACTT | 135 | AAGTAATCCAGTTCTTCTA | 150 |
| 8 | GGTAATAGCTGTAATGTGG | 136 | CCACATTACAGCTATTACC | 151 |
| 9 | GGGCATCAGCCGTGAGCAT | 137 | ATGCTCACGGCTGATGCCC | 152 |
| 10 | TGCGCAAGGTCCAGAGAGA | 138 | TCTCTCTGGACCTTGCGCA | 153 |
| 11 | AGAGAGAAACTGCAAGGAA | 139 | TTCCTTGCAGTTTCTCTCT | 154 |
| 12 | GCAGTTGAATTCAGTGGGT | 140 | ACCCACTGAATTCAACTGC | 155 |
| 13 | GACTACAGGACAAGGGGCA | 141 | TGCCCCTTGTCCTGTAGTC | 156 |
| 14 | GTTCACATTAACTCAGGCA | 142 | TGCCTGAGTTAATGTGAAC | 157 |
| 15 | AATGCTCTAACGTGATAAA | 143 | TTTATCACGTTAGAGCATT | 158 |

The above 19b core sequences result in the target sequence elements ((tar.seq.el.) in Fig. 1) for SEQ ID Nos.: 129 to 143 and the reverse complement target sequence elements ((tar.seq.el._{rc}) in Fig. 1) for SEQ ID Nos.: 144 to 158.

**Table 6 - PolG Pool 4**

| | 19 bp core sequence without 3' overhang for PolG Pool 4 | | | |
|---|---|---|---|---|
| siRN A# | sense | SEQ ID No.: | Reverse complement antisense | SEQ ID No.: |
| 1 | GAGAAGGAGCCTCGAGAAC | 159 | GTTCTCGAGGCTCCTTCTC | 174 |
| 2 | TGAAGAAGTCGTTGATGGA | 160 | TCCATCAACGACTTCTTCA | 175 |
| 3 | AAGAAAGCTAAGAAGGTGA | 161 | TCACCTTCTTAGCTTTCTT | 176 |
| 4 | GTGAGGAGGAGGAGTTTCA | 162 | TGAAACTCCTCCTCCTCAC | 177 |
| 5 | ATGGCAAACGGTAGAAGAA | 163 | TTCTTCTACCGTTTGCCAT | 178 |
| 6 | CTTACAACGACGTGGACAT | 164 | ATGTCCACGTCGTTGTAAG | 179 |
| 7 | CTGAGAAGGCCCAGCAGAT | 165 | ATCTGCTGGGCCTTCTCAG | 180 |
| 8 | CGCAAGGTCCAGAGAGAAA | 166 | TTTCTCTCTGGACCTTGCG | 181 |
| 9 | AGGAAGTCACAGTGGAAGA | 167 | TCTTCCACTGTGACTTCCT | 182 |
| 10 | GGAAGAAGTGGGAGGTGGT | 168 | ACCACCTCCCACTTCTTCC | 183 |
| 11 | GCTCCCAAACTCAGGCTTT | 169 | AAAGCCTGAGTTTGGGAGC | 184 |
| 12 | GGGCATTGTTGCTTGTTGG | 170 | CCAACAAGCAACAATGCCC | 185 |
| 13 | CATTAACTCAGGCATTTCA | 171 | TGAAATGCCTGAGTTAATG | 186 |
| 14 | CTAGAAGGGGAAAGAATTA | 172 | TAATTCTTTCCCCTTCTAG | 187 |
| 15 | TTAATGCTCTAACGTGATA | 173 | TATCACGTTAGAGCATTAA | 188 |

The above 19b core sequences result in the target sequence elements ((tar.seq.el.) in Fig. 1) for SEQ ID Nos.: 159 to 173 and the reverse complement target sequence elements ((tar.seq.el._{rc}) in Fig. 1) for SEQ ID Nos.: 174 to 188.

Sense and antisense core sequences of all 15 constructs of Table 3 to 6 were concatenated to two continuous sequences, in which each siRNA sequence was separated from adjacent sequences by an identical loop sequence of 5 bases (see Figure 3).
- The 5 base loop sequence 5'-AGTTTG-3' was selected for the following features :
   Complete mismatch with its own reverse complement sequence, showing minimal binding free energy in RNA folding prediction (Vienna RNAfold server, http://rna.tbi.univie.ac.at/cgi-bin/RNAfold.cgi)
- Guanin base ("G") at position 2 and 5 for RNAseT1 cleavage 3' of second loop nucleotide and 5' of first siRNA nucleotide.

For in vitro transcription of the construct, the minimal T7 RNA polymerase promoter sequence 5'-TAATACGACTCACTATAGG-3' was placed 5' of the concatenated siRNA-loop sequence for both sense and antisense constructs. For cloning into suitable vector sequences, a HindIII restriction site (5'-AAGCTT-3') was placed 5' of the T7 RNA polymerase promotor and a EcoRI site (5'-GAATTC-3') 3' of the terminal siRNA sequence (Fig. 3). DNA templates were then synthesized, cloned, in vitro transribed, digested with RNAse I and purified as described above in Example 1.

### Generation of complex siRNA pools for Scyl1

The complex siRNA pools for Scyll were generated as described above for PolG. Thus, DNA templates were prepared comprising the 15 target sequence elements for the ultimate siRNAs being interrupted by the loop sequence element 5'-AGTTTG-3' giving rise to a construct as schematically depicted in Fig. 5 and 6. These DNA templates were cloned into the pMA cloning vector using the HindIII (5') and EcoRI (3') site, in vitro transcribed from the T7 RNA polymerase promoter, digested with RNAse T1 and the resulting complex siRNA was purified as described above.

The sequence for the siRNAs were chosen using the on-line siRNA design platform provided by Thermo "siDesign-Center". SiRNAs were designed against the coding sequence as well as the 3'UTR of SCYL1. For each pool of 15 siRNAs, siRNA sequences were selected to have a maximum of 7 nucleotide overlap.

The chosen target sequence elements were as follows:

**Table 7 - Scyl1 Pool 1**

| | 19 bp core sequence without 3' overhang for Scyl1 Pool 1 | | | |
|---|---|---|---|---|
| siRN A# | Sense | SEQ ID No.: | Reverse complement antisense | SEQ ID No.: |
| 1 | CGTTGGGAATATACCTCAA | 189 | TTGAGGTATATTCCCAACG | 204 |
| 2 | GCAGAGTGGTCAGAGAGAA | 190 | TTCTCTCTGACCACTCTGC | 205 |
| 3 | GCAAGAGCCTGGACGCATT | 191 | AATGCGTCCAGGCTCTTGC | 206 |
| 4 | GAGGATTTCTGTCGGCACA | 192 | TGTGCCGACAGAAATCCTC | 207 |
| 5 | GAGTATCAGCAGAAGATCA | 193 | TGATCTTCTGCTGATACTC | 208 |
| 6 | GTACATGGCTTCCTGGACA | 194 | TGTCCAGGAAGCCATGTAC | 209 |
| 7 | GGCTACAGGCCAAGGATGA | 195 | TCATCCTTGGCCTGTAGCC | 210 |
| 8 | GCTCTGCGGTCTCACTGTA | 196 | TACAGTGAGACCGCAGAGC | 211 |
| 9 | GGAGCTTCCTGTCCAAATT | 197 | AATTTGGACAGGAAGCTCC | 212 |
| 10 | GGAGAAGGATGTCCATGCA | 198 | TGCATGGACATCCTTCTCC | 213 |
| 11 | GACCACAAATCCTCCAAAT | 199 | ATTTGGAGGATTTGTGGTC | 214 |
| 12 | GCCTCATCCACAACAATGT | 200 | ACATTGTTGTGGATGAGGC | 215 |
| 13 | GCCATCTCACGTGTACATA | 201 | TATGTACACGTGAGATGGC | 216 |
| 14 | GAGCCACAATAAATTCTAT | 202 | ATAGAATTTATTGTGGCTC | 217 |
| 15 | GTCGACAGGTCAAGGCTGA | 203 | TCAGCCTTGACCTGTCGAC | 218 |

The above 19b core sequences result in the target sequence elements ((tar.seq.el.) in Fig. 1) for SEQ ID Nos.: 189 to 203 and the reverse complement target sequence elements ((tar.seq.el._{rc}) in Fig. 1) for SEQ ID Nos.: 204 to 218.

SEQ ID Nos.: 200 and 215 refer to the siRNA sequence described in Sigoillot et al., *vide supra* as being specific for the target Scyl1 and giving an off-target effect for Mad2.

**Table 8 - Scyl1 Pool 2**

| | 19 bp core sequence without 3' overhang for Scyl 1 Pool 2 | | | |
|---|---|---|---|---|
| siRN A# | sense | SEQ ID No.: | Reverse complement antisense | SEQ ID No.: |
| 1 | TCGATGGACTGGAGACAGA | 219 | TCTGTCTCCAGTCCATCGA | 234 |
| 2 | GGCAGAGTGGTCAGAGAGA | 220 | TCTCTCTGACCACTCTGCC | 235 |
| 3 | CAGCAGACATGTGGCGCTT | 221 | AAGCGCCACATGTCTGCTG | 236 |
| 4 | GTGAGCTGGTGGGAGCAAA | 222 | TTTGCTCCCACCAGCTCAC | 237 |
| 5 | CAGCCCGCTTCCTGCAGAA | 223 | TTCTGCAGGAAGCGGGCTG | 238 |
| 6 | GAGGAGTATCAGCAGAAGA | 224 | TCTTCTGCTGATACTCCTC | 239 |
| 7 | CAAAGCTGAACGAGGCCAA | 225 | TTGGCCTCGTTCAGCTTTG | 240 |
| 8 | TTGCACGGCTACAGGCCAA | 226 | TTGGCCTGTAGCCGTGCAA | 241 |
| 9 | CACTGTAGATCCTGAGAAA | 227 | TTTCTCAGGATCTACAGTG | 242 |
| 10 | TGGAGGAAGTGGAGAAGGA | 228 | TCCTTCTCCACTTCCTCCA | 243 |
| 11 | AGACGCAGGAGGAGGACAA | 229 | TTGTCCTCCTCCTGCGTCT | 244 |
| 12 | CGACTGGAGCAGCTGGGAA | 230 | TTCCCAGCTGCTCCAGTCG | 245 |
| 13 | CCGAGAGGAAGGTGGCCAA | 231 | TTGGCCACCTTCCTCTCGG | 246 |
| 14 | CATCTCACGTGTACATAAT | 232 | ATTATGTACACGTGAGATG | 247 |
| 15 | CATAATCAGAGCCACAATA | 233 | TATTGTGGCTCTGATTATG | 248 |

The above 19b core sequences result in the target sequence elements ((tar.seq.el.) in Fig. 1) for SEQ ID Nos.: 219 to 233 and the reverse complement target sequence elements ((tar.seq.el._{rc}) in Fig. 1) for SEQ ID Nos.: 234 to 248.

**Table 9 - Scyl1 Pool 3**

| | 19 bp core sequence without 3' overhang for Scyl1 Pool 3 | | | |
|---|---|---|---|---|
| siRN A# | sense | SEQ ID No.: | Reverse complement antisense | SEQ ID No.: |
| 1 | CCGTGTCCATCTTCGTCTA | 249 | TAGACGAAGATGGACACGG | 264 |
| 2 | CTTCAAAACTCTACGGCAC | 250 | GTGCCGTAGAGTTTTGAAG | 265 |
| 3 | TGGCTTACATCGATGGACT | 251 | AGTCCATCGATGTAAGCCA | 266 |
| 4 | CCCTCAGCTTCCTGGTCAA | 252 | TTGACCAGGAAGCTGAGGG | 267 |
| 5 | GTGGCAGAGTGGTCAGAGA | 253 | TCTCTGACCACTCTGCCAC | 268 |
| 6 | TCAAAGAGCCAGCCGAGAA | 254 | TTCTCGGCTGGCTCTTTGA | 269 |
| 7 | AGGAGTATCAGCAGAAGAT | 255 | ATCTTCTGCTGATACTCCT | 270 |
| 8 | CTGTGGTGGTCAAGATGTT | 256 | AACATCTTGACCACCACAG | 271 |
| 9 | TCAATGTGGAGCTGATGAA | 257 | TTCATCAGCTCCACATTGA | 272 |
| 10 | CTGAGAAATCCGTGCGAGA | 258 | TCTCGCACGGATTTCTCAG | 273 |
| 11 | CAGGAGGAGGACAAGGACA | 259 | TGTCCTTGTCCTCCTCCTG | 274 |
| 12 | TGACAGATGGGACGACGAA | 260 | TTCGTCGTCCCATCTGTCA | 275 |
| 13 | CCAAGTGAGCCGTGCTAGT | 261 | ACTAGCACGGCTCACTTGG | 276 |
| 14 | CCAGGCCATCTCACGTGTA | 262 | TACACGTGAGATGGCCTGG | 277 |
| 15 | GTACATAATCAGAGCCACA | 263 | TGTGGCTCTGATTATGTAC | 278 |

The above 19b core sequences result in the target sequence elements ((tar.seq.el.) in Fig. 1) for SEQ ID Nos.: 249 to 263 and the reverse complement target sequence elements ((tar.seq.el._{rc}) in Fig. 1) for SEQ ID Nos.: 264 to 278.

**Table 10 - Scyl1 Pool 4**

| | 19 bp core sequence without 3' overhang for Scyl1 Pool 4 | | | |
|---|---|---|---|---|
| siRN A# | sense | SEQ ID No.: | Reverse complement antisense | SEQ ID No.: |
| 1 | CATCGATGGACTGGAGACA | 279 | TGTCTCCAGTCCATCGATG | 294 |
| 2 | TGAAGGAGCTGGAGATCTC | 280 | GAGATCTCCAGCTCCTTCA | 295 |
| 3 | GCTACACCAGATCGTGAAA | 281 | TTTCACGATCTGGTGTAGC | 296 |
| 4 | GCAGCCTCATCCACAACAA | 282 | TTGTTGTGGATGAGGCTGC | 297 |
| 5 | CTGGTGGCTTCATGAGCAA | 283 | TTGCTCATGAAGCCACCAG | 298 |
| 6 | ACGCATTCCCTGAGGATTT | 284 | AAATCCTCAGGGAATGCGT | 299 |
| 7 | AGTATCAGCAGAAGATCAT | 285 | ATGATCTTCTGCTGATACT | 300 |
| 8 | GGCTCCTACCTCAGTGCTA | 286 | TAGCACTGAGGTAGGAGCC | 301 |
| 9 | CTGTAGATCCTGAGAAATC | 287 | GATTTCTCAGGATCTACAG | 302 |
| 10 | AGGAAGTGGAGAAGGATGT | 288 | ACATCCTTCTCCACTTCCT | 303 |
| 11 | GGACAAGGACACAGCAGAG | 289 | CTCTGCTGTGTCCTTGTCC | 304 |
| 12 | ACAGATGGGACGACGAAGA | 290 | TCTTCGTCGTCCCATCTGT | 305 |
| 13 | GCCCCACAGATGTATTTAT | 291 | ATAAATACATCTGTGGGGC | 306 |
| 14 | AGGCCATCTCACGTGTACA | 292 | TGTACACGTGAGATGGCCT | 307 |
| 15 | TAATCAGAGCCACAATAAA | 293 | TTTATTGTGGCTCTGATTA | 308 |

The above 19b core sequences result in the target sequence elements ((tar.seq.el.) in Fig. 1) for SEQ ID Nos.: 279 to 293 and the reverse complement target sequence elements ((tar.seq.el._{rc}) in Fig. 1) for SEQ ID Nos.: 294 to 308.

Sense and antisense core sequences of all 15 constructs of Table 7 to 10 were concatenated to two continuous sequences, in which each siRNA sequence was separated from adjacent sequences by an identical loop sequence of 5 bases (see Figure 3).
- The 5 base loop sequence 5'-AGTTTG-3' (SEQ ID No.: 309) was selected for the following features :
   Complete mismatch with its own reverse complement sequence, showing minimal binding free energy in RNA folding prediction (Vienna RNAfold server, http://rna.tbi.univie.ac.at/cgi-bin/RNAfold.cgi)
- Guanin base ("G") at position 2 and 5 for RNAseT1 cleavage 3' of second loop nucleotide and 5' of first siRNA nucleotide.

For in vitro transcription of the construct, the minimal T7 RNA polymerase promoter sequence 5'-TAATACGACTCACTATAGG-3' (SEQ ID No.: 310) was placed 5' of the concatenated siRNA-loop sequence for both sense and antisense constructs. For cloning into suitable vector sequences, a HindIII restriction site (5'-AAGCTT-3') (SEQ ID No.: 311) was placed 5' of the T7 RNA polymerase promotor and a EcoRI site (5'-GAATTC-3') (SEQ ID No.: 312) 3' of the terminal siRNA sequence (Fig. 3). DNA templates were then synthesized, cloned, in vitro transribed, digested with RNAse TI and purified as described above in Example 1.

### Generation of control siRNAs

The siRNA desccribed in Sigoillot et al., *vide supra* as having PolG as a target and giving off-target effects on Mad2 had the following sense sequence 5'-GGGUGAAGCGCUGGAUAUUTT (SEQ ID No.: 313) and the following reverse complement antisense-sequence : 5'-AAUAUCCAGCGCUUCACCCTT (SEQ ID No.: 314). This siRNA was labeled "PolG siRNA OT". This siRNA was obtained from Eurogentec.

The siRNA described in Sigoillot et al., *vide supra* as having Scyl1 as a target and giving off-target effects on Mad2 had the following sense -sequence 5'-GCCUCAUCCACAACAAUGUTT (SEQ ID No.: 315) and the following reverse complement antisense-sequence : 5'-ACAUUGUUGUGGAUGAGGCTT (SEQ ID No.: 316). This siRNA was labeled "Scyl1 siRNA OT". This siRNA was obtained from Eurogentec.

Further, a negative control siRNA was designed, which should have no effect on PolG, Scyl1 or Mad2. This siRNA had the following sense-sequence: 5'-UUGUCUUGCAUUCGACUAATT (SEQ ID No.: 317) and the following reverse complement antisense-sequence 5'-UUAGUCGAAUGCAAGACAATT (SEQ ID No.: 318). This siRNA was labeled "negative Control" (neg.C.).

Further, an siRNA wich should have Mad2 as a target and which should have no effect on PolG or Scyl1. This siRNA had the following sense-sequence: 5'-GGAACAACUGAAAGAUUGGTT (SEQ ID No.: 319) and the following reverse complement antisense-sequence: 5'-CCAAUCUUUCAGUUGUUCCTT (SEQ ID No.: 320). This siRNA was labeled "Mad2 siRNAl".

### Generation of smart pool siRNAs

Smart pool siRNAs are merchandized as providing better on-target vs. off-target effects by the vendor Thermo Fisher. Smart pools consist of 4 different siRNAs for one target gene. To allow for comparison of complex siRNA pools in accordance with the inventions vs. the smart pool approach, the 4 siRNAs comprising the smart pool for POLG were purchased as individual siRNAs from Thermo Fisher. Of these 4 siRNAs 3 were combined in all possible combinations with the siRNA being known has having PolG as a target and giving an off-target effect on Mad2 This resulted in four different smart pools for PolG comprising four siRNAs.

The first siRNA of the smart pools for PolG had the following sense-sequence: 5'-GGUAUCGGCUGUCGGAUGA (SEQ ID No.: 321) and the following reverse complement antisense-sequence 5'-UCAUCCGACAGCCGAUACC (SEQ ID No.: 322). The second siRNA of the smart pools for PolG had the following sense-sequence: 5'-AGUGGGACCUGCAAGAAUU (SEQ ID No.: 323) and the following reverse complement antisense-sequence 5'-AAUUCUUGCAGGUCCCACU (SEQ ID No.: 324). The third siRNA of the smart pools for PolG had the following sense-sequence: 5'-UCACAAGGAUGGUAAUAGC (SEQ ID No.: 325) and the following reverse complement antisense-sequence 5'-GCUAUUACCAUCCUUGUGA (SEQ ID No.: 326). The fourth siRNA of the smart pools for PolG had the following sense-sequence: 5'-GCUUACUAAUGCAGUUUAA (SEQ ID No.: 327) and the following reverse complement antisense-sequence 5'-UUAAACUGCAUUAGUAAGC (SEQ ID No.: 328). The siRNAs were obtained fromThermo Fisher. These four siRNAs were mixed in all combinations with the siRNA being known has having PolG as a target and giving an off-target effect on Mad2 (SEQ ID No.: 313 and 314, see above) except for Smart Pool 4 giving rise to Smart Pools 1 to 4 (smp1 to 4) for PolG each smart pool comprising four siRNAs.

The first siRNA of the smart pools for Scyl1 had the following sense-sequence: 5'-UUUCUCAGGAUCUACAGUGAG-3' (SEQ ID No.: 340). The second siRNA of the smart pools for Scyl1 had the following sense-sequence: 5'-UUGAGGUAUAUUCCCAACGGG-3' (SEQ ID No.: 341). The third siRNA of the smart pools for Scyl1 had the following sense-sequence: 5'-UUGGUUUCUACAAAGCGGUUG-3' (SEQ ID No.:342). The fourth siRNA of the smart pools for Scyl1 had the following sense-sequence: 5'-UUGUACAAUAAAUACAUCUGU-3' (SEQ ID No.:343). The siRNAs were obtained fromThermo Fisher. These four siRNAs were mixed in all combinations with the siRNA being known has having Scyl1 as a target and giving an off-target effect on Mad2 (SEQ ID No.: 315 and 316, see below) except for Smart Pool 4 giving rise to Smart Pools 1 to 4 (smp1 to 4) for Scyl1 each smart pool comprising four siRNAs.

### Generation of esiRNAs

EsiRNAs were obtained from Sigma. This esiRNA represents the RNAseIII digested dsRNA of a fragment of the human POLG gene with the following sequence:

This SCYL1 esiRNA represents the RNAseIII digested dsRNA of a fragment of the human SCYL1 gene with the following sequence:

### Determination of on-target gene silencing activity of siRNAs

The on-target silencing activity of complex siRNA pools, smart pools, esiRNAs and control siRNAs on PolG or Scyl1 were determined using Reverse-Transcription PCR (RT-PCR). Hela cells were seeded and cultivated in DMEM substituted with 10% FCS and Penicillin/Streptomycine (PenStrep). SiRNAs, complex siRNA pools,esiRNAs and smart pools were transfected in concentrations of 1, 3 or as indicated using LipofectamineRNAiMAX (Life Technologies). Cells were harvested for RNA extraction and RT-PCR 48h after transfection. Gene knock down was calculated using the delta CT method with GAPDH serving as house keeper gene. The following primer pairs were applied:
POLG forward: TTCCAGGACCTGATGCAGTA (SEQ ID No.: 331),
POLG reverse: ACAGGCAGGTAGGAGACACC (SEQ ID No.: 332)
SCYL1 forward: CTGGAGGAAGTGGAGAAGGA (SEQ ID No.: 333)
SCYL1 reverse: TCAGCTTGGAGGTGAGTGAG (SEQ ID No.: 334)
GAPDH forward: ATGGGTGTGAACCATGAGAA (SEQ ID No.: 335)
GAPDH reverse: GTGCTAAGCAGTTGGTGGTG (SEQ ID No.: 336)

### Determination of off-target effect on Mad2 by Luciferase assay

Off-targets effects on Mad2 were quantified with a dual luciferase approach using the vector pmir-RL-TK (Beitzinger et al, 2007). To that end,the complete 3'UTR of MAD2 with the sequence
GGTGACATGAGGAAAATAATGTAATTGTAATTTTGAAATGTGGTTTTCCTGAAATCAAGTCATCTAT AGTTGATATGTTTTATTTCATTGGTTAATTTTTACATGGAGAAACCAAAATGATACTTACTGAACTG TGTGTAATTGTTCCTTTTATTTTTTTGGTATTTGACTTACCATGGAGTTAACATCATGAATTTA TTGCACATTGTTCAAAGGAACCAGGAGGAGGTTTTTTTGTCAACATTGTGATGTATATTCCTTTGAAGAT AGTAACTGTAGATGGAAAAACTTGTGCTATAAAGCTAGATGCTTTCCTAAATCAGATGTTTTGGTCAA GTAGTTTGACTCAGTATAGGTAGGGAGATATTTAAGTATAAAATACAACAAGGAAGTCTATAGCTCC AGAATCTTTGTTAAGGTCCTGAAAGTAACTCATAATCTATAAACAATGAAATATTGCTGTATAGCTCC TTTTGACCTTCATTTCATGTATAGTTTTCCCTATTGAATCAGTTTCCAATTATTTGACTTTAATTTAT GTAACTTGAACCTATGAAGCAATGGATATTTGTACTGTTTAATGTTCTGTGATACAGAACTCTTAAA ATGTTTTTTCATGTGTTTTATAAAATCAAGTTTTAAGTGAAAGTGAGGAAATAAAGTTAAGTTTGTTT TAAATTTGTCTT (SEQ ID No.: 337) was cloned in the 3' end of the firely luciferase gene. A Renilla luciferase gene was present in the dual luciferase vector (Beitzinger et al., RNA Biol. (2007), 4(2):76-84) for internal normalization. 20ng of dual luciferase vector along with different concentrations of siRNA were transfected in Hela cells, seeded in 96-well dishes in DMEM with 10% FCS and PenStrep. Firefly and Renilla Luciferase activity were measured 24h post transfection

### Determination of off-target effect on MAD2 by cellular assay

Mad2 is an integral part of mitotic spindle check points. Wrongly assembled chromosomes inhibit mitosis on the metaphase level to give the cell time correctly distribute the chromosomes on daughter cells. The mitotic interruption can be induced by spindle poisons such as nodocazole. However, Mad2 is essential for this interruption induced by spindle poisons. If Mad2 expression is down-regulated by e.g. RNAi, mitosis will continue even in the presence of spindle poisons such as nodocazole. Interruption of mitosis can be determined visually by rounded and slightly elevated cells. Thus, if Mad2 expression is downregulated, cells will continue to proliferate even in the presence of nodocazole which is labeled herein as "overrun".

Hela cells were seeded in DMEM with 10% FCS and PenStrep on glass cover slips and transfected with 10nM siRNA. 30h after transfection, 50ng/ml Nocodazol was added to arrest cells in mitosis. 48h post transfection, cells were fixed with 1% paraformaldehyde in PBS and imaged by phase contrast microscopy.

### Determination of off-target effect on MAD2 by western blotting

Hela cells were seeded and grown in DMEM substituted with 10%FCS and PenStrep on 6-well dishes. SiRNAs were transfected in a final concentration of 3 and 33nM using Lipofectamine RNAiMax (Life Technologies) as transfection reagent. 48h after transfection, cells were harvested and lysed. The denatured proteins were resolved on 10% SDS PAGE and transferred to a ECL Hybond membrane (GE Healthcare) on a semi-dry blotting device. The blot membrane was incubated over night at 4'C with an affinity purified primary rabbit anti human MAD2 antibody (Bethyl Laboratories Inc.) at a dilution of 1:5000 and a monoclonal mouse anti beta actin antibody (clone AC15 from Abcam) at a dilution of 1:5000in TBS-Tween with 5% milk-powder. As secondary antibodies fluorescently labeled polyclonal goat anti rabbit or goat anti mouse antibodies (anti-rabbit IgG (H+L) IRDye 800 CW, antimouse IgG (H+L) IRDye 800 CW, Li-COR) were incubated for 2h at room temperature in a dilution of 1 to 10000 in TBS-Tween with 5% milk powder. The blot was imaged in two wavelength with a Odyssey Fluorescence scanner (Li-COR)

### Results

### Off- target effects on Mad2 by siRNAs for Scyl1 and PolG

HeLa cell were transfected with the siRNAs "negative Control", "PolG siRNA OT", "Scyll siRNA OT" or "Mad2 siRNA". Expression of Mad 2 and actin as a control was determined by Western Blotting. The siRNAs "PolG siRNA OT", "Scyl1 siRNA" and "Mad2 siRNA" led to decreased Mad2 expression (see Fig. 18, lower panel). In addition, cell density, size and morphology were visually inspected either in the absence or presence of 50ng/ml nocodazole. For cells transfected with the siRNAs "PolG siRNA OT", "Scyl1 siRNA OT" and "Mad2 siRNA", an overrun of the mitotic arrest was observed in the presence of nocodazole. For the negative control, a uniform mitotic arrest was observed (see Fig. 18, upper panel). This data confirms that the siRNAs"PolG siRNA OT" and "Scyl1 siRNA OT" have a strong off-target effect on Mad2 expression.

### On-target effects on Scyl1 by complex siRNA pools for Scyl1

HeLa cell were transfected with siRNA "negative control", Pools 1 to 4 for Scyl1 (see Tables 7 to 10) separately as well as all combined pools 1 to 4 for Scyll, esiRNA for Scyl1 and the siRNA "Scyl1 siRNA OT" at 1nM, 3nM or 10 nM. Effects on Scyll expression were determined by qPCR as described above (see Figure 19).

### On-target effects by high complexity siRNA Pools for PolG

HeLa cell were transfected with siRNA "negative control", Pools 1 to 4 for PolG (see Tables 3 to 6) separately as well as all combined pools 1 to 4 for PolG and the siRNA "PolG siRNA OT" at 1nM, 3nM or 10 nM. Effects on PolG expression were determined by RT-PCR as described above in Experiment 3 (see Figure 28).

### Off-target effects on Mad2 by complex siRNA pools for Scyl1 or PolG

HeLa cells were transfected with siRNA "negative control", with Pool 1 for Scyl1 (see Table 7) separately as well as with combined Pools 1 to 4 for Scyl1, with Pool 1 for PolG (see Table 3) separately as well as with combined Pools 1 to 4 for PolG and with the siRNA "Scyl1 siRNA OT" or with the siRNA "PolG siRNA OT) at 1nM, 3nM or 10 nM. Effects on Mad2 expression were determined by Luciferase assay as described above (see Figure 20).

Both, the complex siRNA Pool 1 and the combined complex siRNA Pools 1 to 4 for Scyl1 and PolG give strongly reduced off-target effects on Mad2 even though these pools comprise "Scyl1 siRNA OT" and "PolG siRNA OT", respectively. Pool 1 for Scyl 1 comprised as one of the 15 siRNAs the "Scyl1 siRNA OT" (see siRNA #12 (SEQ ID No.:200) of Table 7 and sequence of "Scyl1 siRNA OT" (SEQ ID NO:316). Pool 1 for PolG comprised as one of the 15 siRNAs the "PolG siRNA OT" (see siRNA #12 (SEQ ID No.:80) of Table 3 and sequence of "PolG siRNA OT" (SEQ ID NO:313).

The off-target effects were also determined by the cellular assay. To this end, HeLa cells were transfected with 33 nM of either "Mad2 siRNA", "negative Control siRNA", Pool 1 for Scyll, the combined Pools 1 to 4 for Scyl1 and "Scyl1 siRNA OT". If 50ng/ml nodocazole was added, an overrun of the mitotic arrest was observed for "Mad2 siRNA" and "Scyl1 siRNA OT", but not for "negative Control siRNA", Pool1 for Scyl1 or for the combined Pools 1 to 4 for Scyl1 (see Fig. 21).

The same was observed for PolG. Thus, HeLa cells were transfected with 33 nM of either "Mad2 siRNA", "negative Control siRNA", Pool 1 for PolG, the combined Pools 1 to 4 for PolG and "PolG siRNA OT". If 50ng/ml nodocazole was added, an overrun of the mitotic arrest was observed for "Mad2 siRNA" and "PolG siRNA OT", but not for "negative Control siRNA", Pool 1 for PolG or for the combined Pools 1 to 4 for PolG (see Fig. 22).

### Off-target effects on Mad2 by complex siRNA pools for PolG vs. smart Pools

The off-target effects on Mad2 by Pool 1 for PolG and combined Pools 1 to 4 for PolG was compared with the off-target effects of "PolG siRNA OT" and Smart Pools 1, 2, 3 and 4 (smp 1, smp 2, smp 3 and smp 4) for PolG. See above for construction of smart pools for PolG. Off-target effects were determined by the RT-PCR or Luciferase assay and by a phenotypic assay. Results are depicted in Fig. 23 for the PCR assay, in Fig. 33 for Luciferase assay and in Fig. 22 for the phenotypic assay. The on-target effects of the same pools as well as of esiRNA of an independent experiment are depicted in Fig.30 as determined by PCR .

### Off-target effects on Mad2 by complex siRNA pools for Scyl1 vs. smart Pools

The off-target effects on Mad2 by Pool 1 for Scyl1 and combined Pools 1 to 4 for Scyl1 were compared with the off-target effects of "Scyl1 siRNA OT" and Smart Pools 1, 2, 3 and 4 (smp 1, smp 2, smp 3 and smp 4) for Scyl1. See above for construction of smart pools for Scyl1. Off-target effects were determined by the RT-PCR, the Luciferase assay and by a phenotypic assay. Results are depicted in Fig. 31 for the PCR assay and in Fig. 34 for the Luciferase assay. The on-target effects of the same pools as well as of esiRNA of an independent experiment are depicted in Fig. 32 as determined by RT-PCR.

Taken together, the data demonstrate that while on-target silencing of complex siRNA pools matches at least the efficiency of other available RNAi reagents, only complex siRNA pools eliminated off-target effects.

### Experiment 4 - Global off-target effects by complex siRNA pools for Scyl1

Next, the off-target by complex siRNA pools for Scyl1 and a control siRNA on Scyll were determined on a global basis by gene expression array analysis.

### Materials and Methods

### Determination of expression data

Hela cells were seeded and cultivated in DMEM substituted with 10% FCS and Penicillin/Streptomycine (PenStrep). SiRNAs, and complex siRNA pools were transfected in concentrations of 3 nM using Lipofectamine RNAiMAX (Life Technologies). Each transfection was performed in triplicates to allow statistical analysis of the results. Untransfected cells served as a control. Cells were harvested for RNA extraction 48h after transfection. Global RNA expression was analyzed on a Human Gene 1.0 ST array from Affymetrix.

Normalization of raw intensity values from CEL files was performed using variance stabilization (VSN, Huber, 2002) and the median polish was used to summarize individual probes to an expression level per gene or transcript. Genes were defined using a custom chip description file based on ensembl gene identifiers and transcripts were defined with a custom chip description file based on ensembl transcript identifiers (Dai et al., Nucleic Acids Res. (2005), 10(33), 175)). The normalized data on the gene level was used for plotting the gene expression levels of Scyl1 and Mad2, for all other analyses, the normalized data on transcript level was used, since this data allows for distinguishing between transcripts with different 3'-UTRs of the same gene.

Non- and low expressed transcripts were filtered out before testing for differential expression by requiring at least one expression value of the 12 samples to be above the 40th percentile of all expression values. In addition, the 20% of transcripts with lowest interquartile-range, representing constantly expressed genes, were removed. This procedure resulted in 68,580 transcripts for differential expression testing and log2 fold change estimation. Differential gene expression between cells treated with one or more siRNAs and untreated cells was estimated using limma (Smyth GK. Stat Appl Genet Mol Biol. (2004) 3, Article 3)). Because a large number of tests were performed for differential expression, false positive findings were controlled with the false discovery rate (FDR) (Benjamini et al., Journal of the Royal Statistical Society, Series B (Methodological) (1995), 57(1), 289-300).

Instead of multiple testing adjusted p-values, so-called q-values are reported which indicate the largest FDR at which the gene/transcript could be considered significant. Genes/transcripts with a q-value below 0.001 were considered significant differentially expressed. All log2 fold changes reported are in the form of siRNA experiment versus control. Analyses were performed within the statistical programming environment R (R development core team (2011), R: A Language and Environment for Statistical Computing, Vienna, Austria, R: Foundation for Statistical Computing) and using Bioconductor (Gentleman et al., Genome Biol. (2004), 5(10), R80) packages.

### Sequence analysis

Human 3'-UTR sequences were retrieved from ensemble version 68 for the transcripts represented on the microarray. The siRNA seed sequences (nucleotides 2 to 8 of the siRNA) was searched for in the 3'-UTRs of the transcripts and matches were reported for the individual seed sequences.

The on-target silencing activity of complex siRNA pools and control siRNAs on Scyll and the off-target activity on Mad2 were determined using Reverse-Transcription PCR (RT-PCR). Gene knock down was calculated using the delta CT method with GAPDH serving as house keeper gene.

The following primer pairs were applied:
SCYL1 forward: CTGGAGGAAGTGGAGAAGGA (SEQ ID No.: 333)
SCYL1 reverse: TCAGCTTGGAGGTGAGTGAG (SEQ ID No.: 334)
GAPDH forward: ATGGGTGTGAACCATGAGAA (SEQ ID No.: 335)
GAPDH reverse: GTGCTAAGCAGTTGGTGGTG (SEQ ID No.: 336)
Mad2 forward: AGATGACAGTGCACCCAGAG (SEQ ID No.: 338)
Mad2 reverse: TCCAACAGTGGCAGAAATGT (SEQ ID No.: 339)

### Results

### Off-target effects on Mad2 1 by complex siRNA pools for Scyl1 or PolG

Hela cells were transfected with Pool 1 for Scyl1 (see Table 7) separately as well as with combined Pools 1 to 4 for Scyl1 and with the siRNA "Scyl1 siRNA OT" each at 3nM. On-target effects on Scyl1 (see Figure 24) and off-target effects on Mad2 (see Figure 25) were verified by qRT-PCR. The transfection of complex siRNA Pools as well as "Scyl1 siRNA OT" shows efficient on target effects on Scyl1 expression (Figure 24). But only "Scyl1 siRNA OT" results in strong off-target effects on Mad2 expression (Figure 25).

Reduced off-target effects of complex siRNA were further determined by global gene expression analysis. Both the complex siRNA Pool 1 as well as the combined Pools 1 to 4 showed lower amounts of regulated transcripts in comparison to the single "Scyl1 siRNA OT" (Figure 26). The number of regulated transcripts both with (BS) or without a binding site (noBS) for "Scyl1 siRNA OT" reveals that the use of complex siRNA pools results in a reduced number or regulated transcripts which could be considered as reduced off-target activity. This is shown by the reduced spreading of the regulated transcripts (Figure 26). In addition to that the reduced off-target activity of the complex siRNA pools is depicted by the increased amounts of repressed transcripts with (BS) and without (noBS) one or more seed sequence matches for "Scyl1 siRNA OT" after transfection of the single siRNA "Scyl1 siRNA OT" compared the transfection of complex siRNA pools (Figure 26). The reduced number of repressed transcripts is in addition to that also shown by the down shift of the boxes representing the interquartile range (IQR) consisting of the central 50% of the data.

### Experiment 5 - Off-target effects by high complexity siRNA Pools for PolG

Further experiments were conducted to confirm the off-target effects observed for complex siRNA pools for PolG and Scyl1 (see Experiment 3). To ensure that the off-target siRNAs had indeed entered the RNAi mechanism, the 60 siRNA-containing pools (Pools 1 to 4) against PolG or Scyl1 (see Experiment 3) were transfected into HeLa cells and Ago2 was immunoprecipitated from the cell lysates (Figure 29A). The off target siRNAs were analyzed by Northern blotting using probes against the guide (upper panel) or the passenger strand (lower panel). The guide strand was readily detectable in Ago2 complexes indicating that siRNAs are efficiently processed and loaded by Ago2.

To further solidify the results of Experiment 3, Mad2 protein reduction by PolG and Scyll siRNA off target effects was analyzed (Figure 29B). HeLa cells were transfected with siRNAs against PolG (left panel, Pool 1, combined Pools 1 to 4, neg. C., and single "PolG siRNA OT") or Scyl1 (right panel, Pool 1, combined Pools 1 to 4, neg. C., and single "Scyl1 siRNA OT"). Cells were lyzed and protein extracts were analyzed by western blotting against Mad2. In accordance with the results on Mad2 mRNA levels, it was found that both off target siRNAs as well as the control siRNA directed against Mad2 strongly reduce Mad2 protein levels (left and right panels, lanes 7-10). However, when the same off-target siRNAs are placed into complex siRNA pools, the Mad2 protein depletion is strongly reduced (left and right panels, lanes 3-6).

Finally, we generated luciferase reporters containing miRNA-like binding sites for the PolG off-target siRNA or the Scyl1 off-target siRNA (Figure 29C). The single off-target siRNAs were transfected into HeLa cells and a reduction of the luciferase activity was observed (left and right panels, siRNA off-T). However, when the siRNAs were part of complex siRNA pools (Pool 1, combined Pools 1 and 2, combined Pools 1 to 3, and combined Pools 1 to 4), the reduction of the luciferase activity was abolished. Furthermore, we analyzed the complexity requirements of the pools for off-target elimination. While the Scyl1 off target effect was already eliminated when 15 siRNAs were used, the effects of the PolG pools were slightly stronger in pools with higher complexity (left panel, compare Pool 1 with combined Pools 1 and 2).

### Experiment 6 - Simultaneous knock down of redundant gene family members

The convenient production procedure as well as the efficient knock down prompted us to ask whether we can knock down redundant gene family members using one siPool. For our analysis, we chose the human TNRC6 protein family comprising TNRC6A, B and C. These proteins are downstream factors of Ago proteins and are essential for miRNA-guided gene silencing. We generated complex siRNA pools against the individual TNRC6 proteins (Figure 35C, siPool A, B and C) and also combined them to one siPool (siPool ABC).

The sequence for the siRNAs were chosen by selecting for siRNAs with a T residue in position 1 and G or C residues in position 19. Overall GC content was between 7 and 12 GC residues of the 19mer antisense strand sequence. Furthermore positions 2,10 and 18 of the antisense strand were preferred to be A/T, A and G/C respectively.. SiRNAs were designed against the coding sequence as well as the 3'UTR of TNRC6 A, B and C. For each pool of 30 siRNAs, siRNA sequences were selected to have a maximum of 7 nucleotide overlap.

The chosen target sequence elements were as follows:

**Table 11 - Pool A**

| | 19 bp core sequence without 3' overhang for Pool A | | | |
|---|---|---|---|---|
| siRN A# | sense | SEQ ID No.: | Reverse complement antisense | SEQ ID No.: |
| 1 | GCAGGGATTTAGTGCAAGA | 352 | TCTTGCACTAAATCCCTGC | 382 |
| 2 | GCCTCGGTATCCTCGTGAA | 353 | TTCACGAGGATACCGAGGC | 383 |
| 3 | GCAGTGCTTTAACAAATCA | 354 | TGATTTGTTAAAGCACTGC | 384 |
| 4 | GGACCTGTGTCTTCTACAA | 355 | TTGTAGAAGACACAGGTCC | 385 |
| 5 | GAGTTGGCTTCAGAATGTA | 356 | TACATTCTGAAGCCAACTC | 386 |
| 6 | GCACTGGACTTGGTTCCCA | 357 | TGGGAACCAAGTCCAGTGC | 387 |
| 7 | GGATGCTCCTGAAAGCAAA | 358 | TTTGCTTTCAGGAGCATCC | 388 |
| 8 | GGCCAGTATTAGAGAACAA | 359 | TTGTTCTCTAATACTGGCC | 389 |
| 9 | GGAAACTTGTGAATCTGAA | 360 | TTCAGATTCACAAGTTTCC | 390 |
| 10 | GGAGGCTCTTATGGTACTA | 361 | TAGTACCATAAGAGCCTCC | 391 |
| 11 | GACAAATGTTCAGGCCCTA | 362 | TAGGGCCTGAACATTTGTC | 392 |
| 12 | GGCACTAACTTTCAAGTTA | 363 | TAACTTGAAAGTTAGTGCC | 393 |
| 13 | GCAGCAAACTCCCAGAGTA | 364 | TACTCTGGGAGTTTGCTGC | 394 |
| 14 | GGCGCAAATTCTGGAGGAA | 365 | TTCCTCCAGAATTTGCGCC | 395 |
| 15 | GGAACAAACTGCCTAGCAA | 366 | TTGCTAGGCAGTTTGTTCC | 396 |
| 16 | GGATCAGGGTTCTGCCACA | 367 | TGTGGCAGAACCCTGATCC | 397 |
| 17 | GGAGAGCGATGGTAGTACA | 368 | TGTACTACCATCGCTCTCC | 398 |
| 18 | GAAGATGATTCTGCTGCTA | 369 | TAGCAGCAGAATCATCTTC | 399 |
| 19 | GGAGAAACTTCAAGGAATA | 370 | TATTCCTTGAAGTTTCTCC | 400 |
| 20 | CGTTTCCGGTTGGAACGAA | 371 | TTCGTTCCAACCGGAAACG | 401 |
| 21 | GGATAATGGTACTTCAGCA | 372 | TGCTGAAGTACCATTATCC | 402 |
| 22 | GGAACCCATTGCTGCGGCA | 373 | TGCCGCAGCAATGGGTTCC | 403 |
| 23 | GATATGCCATTGCCTGGAA | 374 | TTCCAGGCAATGGCATATC | 404 |
| 24 | GCCACCATATACAAAGAAA | 375 | TTTCTTTGTATATGGTGGC | 405 |
| 25 | CGAAGGGTCTGAGTGGCAA | 376 | TTGCCACTCAGACCCTTCG | 406 |
| 26 | GATGAAAGGTGGAAACAAA | 377 | TTTGTTTCCACCTTTCATC | 407 |
| 27 | GGAGGAATGTTACAAGACA | 378 | TGTCTTGTAACATTCCTCC | 408 |
| 28 | GGCCTCAGATTTCCAAAGA | 379 | TCTTTGGAAATCTGAGGCC | 409 |
| 29 | GCAGCAGCCTCCAGCACAA | 380 | TTGTGCTGGAGGCTGCTGC | 410 |
| 30 | GGCTTGAACTCAAACTTGA | 381 | TCAAGTTTGAGTTCAAGCC | 411 |

The above 19b core sequences result in the target sequence elements ((tar.seq.el.) in Fig. 1) for SEQ ID Nos.: 352 to 381 and the reverse complement target sequence elements ((tar.seq.el._{rc}) in Fig. 1) for SEQ ID Nos.: 382 to 411.

**Table 12 - Pool B**

| | 19 bp core sequence without 3' overhang for Pool B | | | |
|---|---|---|---|---|
| siRN A# | sense | SEQ ID No.: | Reverse complement antisense | SEQ ID No.: |
| 1 | GCATCAGATTCCAAATCTA | 412 | TAGATTTGGAATCTGATGC | 442 |
| 2 | GGAGGAGTCTGGAACACCA | 413 | TGGTGTTCCAGACTCCTCC | 443 |
| 3 | GGCAGTGCTTCCTCCCACA | 414 | TGTGGGAGGAAGCACTGCC | 444 |
| 4 | GGATGAATCCTCTTGCCAA | 415 | TTGGCAAGAGGATTCATCC | 445 |
| 5 | CCGTCCACCTAATTCCAAA | 416 | TTTGGAATTAGGTGGACGG | 446 |
| 6 | CCAGTTATCTCCTCAACAA | 417 | TTGTTGAGGAGATAACTGG | 447 |
| 7 | CCCAGACCTTCAAACCAAA | 418 | TTTGGTTTGAAGGTCTGGG | 448 |
| 8 | GGATATGGTTCTGGCTTCA | 419 | TGAAGCCAGAACCATATCC | 449 |
| 9 | GGAACCGAGTCTCGCTTTA | 420 | TAAAGCGAGACTCGGTTCC | 450 |
| 10 | GCTGCCCTCTGTAGCCACA | 421 | TGTGGCTACAGAGGGCAGC | 451 |
| 11 | GGAAGCCAATATGCACAAA | 422 | TTTGTGCATATTGGCTTCC | 452 |
| 12 | GATAGCTGGTTACCTGCCA | 423 | TGGCAGGTAACCAGCTATC | 453 |
| 13 | CCTGCCAAATCTCCACCAA | 424 | TTGGTGGAGATTTGGCAGG | 454 |
| 14 | GGAGTGCCATGGAAAGGTA | 425 | TACCTTTCCATGGCACTCC | 455 |
| 15 | GCTGCGGGATAACACCACA | 426 | TGTGGTGTTATCCCGCAGC | 456 |
| 16 | GGGTCTAATTCTTCCCTCA | 427 | TGAGGGAAGAATTAGACCC | 457 |
| 17 | CAGCAAAGTTCCCTGATTA | 428 | TAATCAGGGAACTTTGCTG | 458 |
| 18 | CAGATCCCATAGGACACAA | 429 | TTGTGTCCTATGGGATCTG | 459 |
| 19 | CCACTCATCTCTCCAACAA | 430 | TTGTTGGAGAGATGAGTGG | 460 |
| 20 | GGGTCAACCTTGAGAACGA | 431 | TCGTTCTCAAGGTTGACCC | 461 |
| 21 | GCCCACTGCTGACATTCCA | 432 | TGGAATGTCAGCAGTGGGC | 462 |
| 22 | GACATTCCATCTGAATCTA | 433 | TAGATTCAGATGGAATGTC | 463 |
| 23 | GCACTGCCCTGATCCGATA | 434 | TATCGGATCAGGGCAGTGC | 464 |
| 24 | GCACATGTGTGTGTTGGGA | 435 | TCCCAACACACACATGTGC | 465 |
| 25 | GTTTGCCACTGATGATGAA | 436 | TTCATCATCAGTGGCAAAC | 466 |
| 26 | CAGCCGCTTTCTGGCACAA | 437 | TTGTGCCAGAAAGCGGCTG | 467 |
| 27 | CCAGTCAGATCCCGTGGGA | 438 | TCCCACGGGATCTGACTGG | 468 |
| 28 | CGATCTTGCTGGCGCTTCA | 439 | TGAAGCGCCAGCAAGATCG | 469 |
| 29 | GGGCAGCCCTGCTCCTTTA | 440 | TAAAGGAGCAGGGCTGCCC | 470 |
| 30 | GGAGGAGGGTCGGATTCAA | 441 | TTGAATCCGACCCTCCTCC | 471 |

The above 19b core sequences result in the target sequence elements ((tar.seq.el.) in Fig. 1) for SEQ ID Nos.: 412 to 441 and the reverse complement target sequence elements ((tar.seq.el._{rc}) in Fig. 1) for SEQ ID Nos.: 442 to 471.

**Table 13 - Pool C**

| | 19 bp core sequence without 3' overhang for Pool C | | | |
|---|---|---|---|---|
| siRN A# | sense | SEQ ID No.: | Reverse complement antisense | SEQ ID No.: |
| 1 | GGCCTGTACTTGGACATGA | 472 | TCATGTCCAAGTACAGGCC | 502 |
| 2 | GAAACTTGCTGCCACAAGA | 473 | TCTTGTGGCAGCAAGTTTC | 503 |
| 3 | GAATGTGTCTTTCAGCGCA | 474 | TGCGCTGAAAGACACATTC | 504 |
| 4 | GCAGACAAATGGACTGCCA | 475 | TGGCAGTCCATTTGTCTGC | 505 |
| 5 | GGGCAGTGCTGAAGGAATA | 476 | TATTCCTTCAGCACTGCCC | 506 |
| 6 | CGTACAGCCTGGTGGTGAA | 477 | TTCACCACCAGGCTGTACG | 507 |
| 7 | GCGGCATCTTCTGGAACTA | 478 | TAGTTCCAGAAGATGCCGC | 508 |
| 8 | GAATGATCTTGACCCAAGA | 479 | TCTTGGGTCAAGATCATTC | 509 |
| 9 | CCCTAGGTCTGAAAGGAAA | 480 | TTTCCTTTCAGACCTAGGG | 510 |
| 10 | GGGTCTGGTTGGAATGACA | 481 | TGTCATTCCAACCAGACCC | 511 |
| 11 | CGGTACCGGTCAAACAGAA | 482 | TTCTGTTTGACCGGTACCG | 512 |
| 12 | GTAAACATGTGGGATAGAA | 483 | TTCTATCCCACATGTTTAC | 513 |
| 13 | GGTGGATAATGGCACAGCA | 484 | TGCTGTGCCATTATCCACC | 514 |
| 14 | GAATAATGCTGCTTCCCAA | 485 | TTGGGAAGCAGCATTATTC | 515 |
| 15 | GAAAGCACCTCCTCCTGCA | 486 | TGCAGGAGGAGGTGCTTTC | 516 |
| 16 | GATGAGGCCTGGATCATGA | 487 | TCATGATCCAGGCCTCATC | 517 |
| 17 | GAGGAGGCCTTGAAGAGTA | 488 | TACTCTTCAAGGCCTCCTC | 518 |
| 18 | GCCCGCCAATCTCCAAAGA | 489 | TCTTTGGAGATTGGCGGGC | 519 |
| 19 | GCAGCAAGTTGCGCGCACA | 490 | TGTGCGCGCAACTTGCTGC | 520 |
| 20 | CCGGTGGCTTGTCGGTGAA | 491 | TTCACCGACAAGCCACCGG | 521 |
| 21 | GCATGGTGCTATCCCTGGA | 492 | TCCAGGGATAGCACCATGC | 522 |
| 22 | GGTACGATTTAATCCAGAA | 493 | TTCTGGATTAAATCGTACC | 523 |
| 23 | CCTCAAGAGTGGAGGTAAA | 494 | TTTACCTCCACTCTTGAGG | 524 |
| 24 | GAGGCCACCTCCAGGGTTA | 495 | TAACCCTGGAGGTGGCCTC | 525 |
| 25 | GCTGGCTCGTTCTTCGAAA | 496 | TTTCGAAGAACGAGCCAGC | 526 |
| 26 | GGCCTCTTATCACATTCCA | 497 | TGGAATGTGATAAGAGGCC | 527 |
| 27 | CCACCTGAATCTGACTCAA | 498 | TTGAGTCAGATTCAGGTGG | 528 |
| 28 | GCAATGCTGTGGTCCGGTA | 499 | TACCGGACCACAGCATTGC | 529 |
| 29 | GCCCAGAAGTCTCTGCACA | 500 | TGTGCAGAGACTTCTGGGC | 530 |
| 30 | CGAGTTCGCTGGTGAAGAA | 501 | TTCTTCACCAGCGAACTCG | 531 |

The above 19b core sequences result in the target sequence elements ((tar.seq.el.) in Fig. 1) for SEQ ID Nos.: 472 to 501 and the reverse complement target sequence elements ((tar.seq.el._{rc}) in Fig. 1) for SEQ ID Nos.: 502 to 531.

This sequences were incorporated into a construct using the RNase T1 loop sequence AGTTTG as described in Example 3.

The negativ control siRNA (ctrl.) had the sense sequence 5'-UUGUCUUGCAUUCGACUAAUT-3' (SEQ ID No.: 532) and the following reverse complement antisense-sequence : 5'-UUAGUCGAAUGCAAGACAAUT-3' (SEQ ID No.:533).

The first control siRNA for TNRC6A (siRNA A1) had the sense sequence 5'-UAAUGCCAAGCGAGCUACAUT-3' (SEQ ID No.: 534) and the following reverse complement antisense-sequence : 5'-UGUAGCUCGCUUGGCAUUAUT-3' (SEQ ID No.: 535).

The second control siRNA for TNRC6A (siRNA A2) had the sense sequence 5'-UAUAGUACUGCACUGAAUAUT-3 (SEQ ID No.: 536) and the following reverse complement antisense-sequence : 5'-UAUUCAGUGCAGUACUAUAUT-3' (SEQ ID No.: 537).

The first control siRNA for TNRC6B (siRNA B1) had the sense sequence 5'-GGAGUGCCAUGGAAAGGUAUT-3' (SEQ ID No.: 538) and the following reverse complement antisense-sequence : 5'-UACCUUUCCAUGGCACUCCUT -3' (SEQ ID No.: 539).

The second control siRNA for TNRC6B (siRNA B2) had the sense sequence 5'-GGAAGUUGUUGCUAAGAAAUT-3' (SEQ ID No.: 540) and the following reverse complement antisense-sequence : 5'- UUUCUUAGCAACAACUUCCUT-3' (SEQ ID No.: 541).

The first control siRNA for TNRC6C (siRNA C1) had the sense sequence 5'-CAAUGGCGUUGGUAAUAUCUT-3' (SEQ ID No.: 542) and the following reverse complement antisense-sequence : 5'-GAUAUUACCAACGCCAUUGUT-3' (SEQ ID No.: 543).

The second control siRNA for TNRC6C (siRNA C2) had the sense sequence 5'-CAAUAUGAAUCUUGAUCAGUT-3' (SEQ ID No.: 544) and the following reverse complement antisense-sequence : 5'-CUGAUCAAGAUUCAUAUUGUT-3' (SEQ ID No.: 545).

All siPools knocked down their individual on-targets. Single siRNAs knocked down the TNRC6 genes as well but showed rather variable efficiencies (Figure 35A). Strikingly, the siPool ABC targeting all TNRC6 genes indeed reduced the mRNA levels of each family member efficiently. We next tested the consequences of TNRC6 gene knock down using a miRNA reporter assays based on a luciferase gene controlled by the HMGA2 3' UTR (Figure 35D). This 3' UTR contains seven let-7a binding sites and is repressed by the miRNA machinery (Mayr er al., (2007) Science 315, 1576-1579. Inhibition of let-7a by antisense inhibitors leads to a relief of repression (right bar) indicating that the reporter system is indeed under the control of let-7a. Knock down of TNRC6A or B either by the siPools or by individual siRNAs relieved repression and led to an increase of luciferase activity of about 1.5-1.8 fold (Figure 35B). Knock down of TNRC6C alone had only a minor effect on luciferase activity. Of note, TNRC6C expression is much weaker in the HEK 293 cells that have been used (Figure 35E). Knock down of all three family members simultaneously by the siPool ABC released repression by 2.3 to 2.5 fold, a similar range observed by the antisense inhibitor against let-7a. In summary, siPools can be used for efficient knock down of redundant gene family members and Furthermore, the siPool ABC against all TNRC6 genes is a valuable control for experiments aiming at identification of miRNA target genes.

### Experiment 7 - siPools do not cause measurable interferon responses

Since siPools and esiRNAs derive from longer dsRNA precursors and such precursors might cause an interferon response, we tested the expression of interferon response genes after siRNA transfection. We used complex siRNA pools (siPools) and esiRNAs against four different targets (PolG, Scyl1, Traf5 and Ago2) and analyzed them on an agarose gel. While siPools show distinct 21 nt long bands, all purchased esiRNAs were characterized by an RNA smear ranging from 15 to more than 40 nts (Figure 36). For interferon response experiments, we changed the cell line to MCF7 cells, which are more sensitive compared to other cell lines such as HeLa cells. All four target genes were efficiently knocked down by the siPools, while the esiRNA-mediated knock down was slightly less efficient (data not shown). We next analyzed the expression of the interferon response genes IFNB1 and OAS1 upon knock down. While siPools did not cause expression of IFNB1 or OAS1, two esiRNAs led to a strong (Scyl1, PolG) and another one (Traf5) to a medium to low interferon response (data not shown). This effect was esiRNA concentration dependent suggesting that indeed the longer RNA species within the esiRNAs cause this effect. The esiRNA against Ago2 did not cause a significant interferon response (data not shown). Similar results were obtained, when the interferon response genes IL6 and STAT1 were measured (data not shown). Together, the data suggest that due to a limited accuracy of RNase III digestion, esiRNAs contain longer by-products that cause off target effects. This is not observed for siPools.

### Experiment 8 - testing of further loop sequences

The following loop sequences were incorporated into the construct for the siRNA pool for AUKB:
Loop sequence 1: AGTTG
Loop sequence 2: AGTTTG
Loop sequence 3: AGTTAG
Loop sequence 4: AGTTTTG
Loop sequence 5: AGTTTAG
Loop sequence 6: AGTGTAG

Constructs with the loops sequence were *in vitro* transcribed and digested with RNase T1 under different conditions. The varied parameters included RNase T1 concentration (0.1 to 10 U RNase T1/µg dsRNA), incubation time (5 to 120 min), and MgCl₂ concentration (100 mM).

The following conclusions can be drawn from these experiments:
At high concentrations 10 U RNase T1/µg dsRNA vs 0.1 RNase T1/µg dsRNA and long incubation time (20 min vs 5 min) at these concentrations, siRNAs start to loose 3#-overhangs. A concentration of 0.1 to 5 U RNase T1/µg ds RNA at incubation times of 5 to 120 min seems to result in complete digest with no loss of 3'-overhangs.

Inlcusion of MgCl₂ does not alter specificity of digest, but reduces efficiency.

Regardless of loop sequences, no siRNAs shorter than 21 nt are observed at optimized digest conditions. No significant improvement is observed when increasing length from e.g. AGTTG to AGTTTG. However, when reducing number of T-G hydrogen bonds by replacing e.g. T with A, improvement of the digest is observed: Fig. 37 depicts a digest at 0.1 U RNase T1/µg dsRNA and 30 or 120 min incubation, i.e. at conditions where the digest is substantially complete. One can conclude from these data that the loop sequences AGTTTAG and AGTGTAG work better than e.g. AGTTG, AGTTTG or AGTTTTG. AGTTAG works better than AGTTTG and AGTTTAG works better than AGTTTTG.

## Claims

1. Method of preparing different double stranded RNA molecules, wherein each strand of said different double stranded molecules has a length of 15 to 30 nucleotides wherein said different double stranded RNA molecules are capable of target-specific RNA interference of at least one target gene, said method comprising at least the steps of:
a. Providing at least one first DNA molecule comprising in the 5'-3' direction as repeating units a nucleic acid sequence with the following elements:
5' - [(target-sequence-element) - (loop-sequence-element)]ₖ-3',
with k being an integer >1,
with the target-sequence-element being a continuous sequence of 15 to 30 desoxyribonucleotides, which is sense to a sequence in said at least one target gene of RNA interference, with the loop-sequence-element having the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference,
b. Providing at least one second DNA molecule comprising in the 5'-3' direction in a repetitive manner a nucleic acid sequence with the following elements:
5' - [(target-sequence-element)rc - (loop-sequence-element)]i-3',
with 1 being an integer >1 and having the same value as k in the first DNA molecule,
with the target-sequence-elementrc being a continuous sequence of 15 to 30 desoxyribonucleotides,
with the loop-sequence-element having the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference,
wherein the target-sequence-elementsrc counted from the 3' end in the repeating units of said second DNA molecule are the respective reverse complement of the target-sequence-elements counted from the 5' end in the repeating units of said first DNA molecule, and
wherein the loop-sequence-elements in the repeating units of said second DNA molecule are not reverse complements of the loop-sequence-elements in the repeating units of said first DNA molecule,
c. *In vitro* transcribing said at least one first and at least one second DNA molecules using an RNA polymerase to obtain corresponding at least one first and at least one second RNA molecules,
d. Hybridizing said at least one first and at least one second RNA molecules of step c. to obtain a double stranded RNA molecule depicted in Figure 1,
e. Digesting the double stranded RNA molecule obtained in step d. with RNase T1 capable of preferentially recognizing, cleaving and digesting the single stranded loop-sequence-elements over the hybridized double stranded sections of the double stranded RNA obtained in step d. thereby removing single stranded RNA loops,
wherein the sequences of the loop-sequence-elements are selected such that double stranded RNA molecules are obtained by recognition, cleavage and digestion of the single stranded loop-sequence elements by RNase T1 in step e., wherein each strand of said resulting double stranded molecules has a length of 15 to 30 nucleotides, wherein said resulting double stranded RNA molecules are capable of target-specific RNA interference of at least one target gene.

2. Method of preparing different double stranded RNA molecules, wherein each strand of said double stranded molecules has a length of 15 to 30 nucleotides, wherein said double stranded RNA molecules are capable of target-specific RNA interference of at least one target gene, said method comprising at least the steps of:
a. Providing at least one first DNA molecule comprising in the 5'-3' direction as repeating units a nucleic acid sequence with the following elements:
5' - [(target-sequence-element) - (loop- sequence-element)]k - (target-sequence-element) - (loop-sequence-element)ₕₚ - [(target-sequence-element)_{rc} - (loop-sequence-element)]i-3',
with k being an integer >1,
with 1 being an integer >1 and being the same as k,
with the target-sequence-element being a continuous sequence of 15 to 30 desoxyribonucleotides, which is sense to a sequence in said at least one target gene of RNA interference,
with the loop-sequence-element having the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference,
wherein the (loop sequence element)ₕₚ is of sufficient length to allow for a hairpin structure enabling a self-hybdrization pattern,
with the target-sequence-elementrc being a continuous sequence of 15 to 30 desoxyribonucleotides,
wherein the target-sequence-elementsrc counted from the 3' end are the respective reverse complement of the target-sequence-elements counted from the 5' end,
wherein the loop-sequence-elements following the (loop sequence element)ₕₚ are not reverse complements of the loop-sequence-elements preceeding the in the repeating units of said second DNA molecule,
b. *In vitro* transcribing said at least one first DNA molecules using an RNA polymerase to obtain corresponding at least one first RNA molecule, which upon hybridization provides the general structure depicted in Figure 2,
c. Digesting the double stranded RNA molecule obtained in step b. with RNase T1 capable of preferentially recognizing, cleaving and digesting the single stranded loop-sequence-elements over the hybridized double stranded sections of the double stranded RNA obtained in step b. thereby removing single stranded RNA loops,
wherein the sequences of the loop-sequence-elements are selected such that double stranded RNA molecules are obtained by recognition, cleavage and digestion of the single stranded loop-sequence elements by RNase T1 in step c., wherein each strand of said resulting double stranded molecules has a length of 15 to 30 nucleotides and wherein said resulting double stranded RNA molecules are capable of target-specific RNA interference of at least one target gene.

3. Method according to any of claims 1 or 2, wherein the number of target-sequence-elements is at least 2, at least 3, at least 4, at least 5, at least 6, at least 7, at least 8, at least 9, at least 10, at least 11, at least 12, at least 13, at least 14 or at least 15, wherein the sequence of said target-sequence-elements of the at least one first DNA molecule as depicted in Fig. 1 or of the at least one DNA molecule as depicted in Fig. 2 is sense to sequences of said at least one target gene of RNA interference, wherein the sequences of said target-sequence-elementsrc of the at least one second DNA molecule as depicted in Fig. 1 or of the at least one DNA molecule as depicted in Fig. 2 are the reverse complements of the sequences of the target-sequence-elements of the at least one first DNA molecule as depicted in Fig. 1 or of the at least one DNA molecule as depicted in Fig. 2, which they hybridize to, wherein the loop-sequence elements of the at least one first DNA molecules are not reverse complements of each other, and, in case of the at least one DNA molecule as depicted in Fig. 2 the (loop sequence element)ₕₚ is of sufficient length to allow for a hairpin structure enabling a self-hybdrization pattern as depicted in Fig. 2.

4. Method according to any of claims 1, 2, or 3, wherein each strand of said different double stranded molecules has a length of 17 to 25 nucleotides.

5. Method according to any of claims 1, 2, 3, or 4, wherein each strand of said different double stranded molecules has a 3'-overhang of 2 nucleotides.

6. A combination or a kit of at least two DNA molecules, which upon in vitro transcription, hybridization and digestion with RNase T1 according to claim 1 are capable of providing double stranded RNA molecules, wherein each strand of said different double stranded molecules has a length of 15 to 30 nucleotides and wherein said double stranded RNA molecules are capable of target-specific RNA interference of at least one target gene, wherein said at least two DNA molecules have the sequence elements necessary to obtain an RNA molecule of the general structure depicted in Fig. 1 after in vitro transcription and hybridization,
wherein the loop-sequence-element have the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference, such that double stranded RNA molecules can be obtained after cleavage and digestion with RNase T1 capable of preferentially recognizing and cleaving the single stranded loop-sequence-elements over the hybridized double stranded sections of the double stranded RNA thereby removing single stranded RNA loops.

7. A combination or a kit of at least one DNA molecule, which upon in vitro transcription, hybridization and digestion with RNase T1 according to claim 2 is capable of providing double stranded RNA molecules, wherein each strand of said double stranded molecules has a length of 15 to 30 nucleotides and wherein said different double stranded RNA molecules are capable of target-specific RNA interference of at least one target gene,
wherein said at least one DNA molecule has the sequence elements necessary to obtain an RNA molecule of the general structure of Fig. 2 after in vitro transcription and hybridization,
wherein the loop-sequence-element have the sequence 5'-(X)ₘ-G-(Y)ₙ-G-3',
with X being A and m being an integer of 1,
with Y being A or T and n being an integer of 1, 2, or 3, and
with X and Y being selected such that there is no hybridization of two molecules consisting just of the loop-sequence-element and wherein the loop-sequence element does not match a sense or antisense sequence in said at least one target gene of RNA interference, such that double stranded RNA molecules are obtained after digestion with RNase T1 capable of preferentially recognizing, cleaving and digesting the single stranded loop-sequence-elements over the hybridized double stranded sections of the double stranded RNA thereby removing single stranded RNA loops.

8. The combination or the kit according to claim 6 or 7, further comprising:
a. optionally an RNA polymerase,
b. optionally a buffer for in vitro transcription,
c. optionally a buffer for hybridization,
d. optionally RNase T1, and
e. optionally written instructions.

9. Use of a method of any of claims 1 to 5 or of a combination or a kit of any claims 6 to 8 for producing siRNA pools.

## Patentansprüche

1. Verfahren zur Herstellung unterschiedlicher doppelsträngiger RNA-Moleküle, wobei jeder Strang der besagten unterschiedlichen doppelsträngigen Moleküle eine Länge von 15 bis 30 Nukleotiden hat, wobei besagte unterschiedliche doppelsträngige RNA-Moleküle zur zielspezifischen RNA-Interferenz von mindestens einem Zielgen befähigt sind, wobei besagtes Verfahren mindestens die Schritte umfasst:
a. Bereitstellen mindestens eines ersten DNA-Moleküls, das in 5'-3' Richtung als sich wiederholende Einheiten eine Nukleinsäuresequenz mit den folgenden Elementen umfasst:
5' - [(Zielsequenzelement) - (Loop-Sequenzelement)]k - 3',
wobei k eine ganze Zahl > 1 ist,
wobei das Zielsequenzelement eine kontinuierliche Sequenz von 15 bis 30 Desoxyribonukleotiden ist, die "sense" zu einer Sequenz in besagtem mindestens einem Zielgen der RNA-Interferenz ist, wobei das Loop-Sequenzelement die Sequenz 5'-(X)m-G-(Y)n-G-3' hat,
wobei X A und m eine ganze Zahl 1 ist,
wobei Y A oder T ist und n eine ganze Zahl 1, 2 oder 3 ist, und
wobei X und Y so ausgewählt sind, dass keine Hybridisierung von zwei Molekülen stattfindet, die nur aus dem Loop-Sequenzelement bestehen und wobei das Loop-Sequenzelement nicht zu einer Sense- oder Antisense-Sequenz in besagtem mindestens einem Zielgen der RNA-Interferenz passt,
b. Bereitstellen von mindestens einem zweiten DNA-Molekül, das in 5'-3' Richtung in einer sich wiederholenden Weise eine Nukleinsäuresequenz mit den folgenden Elementen umfasst:
5' - [(Zielsequenzelement)rc - (Loop-Sequenzelement)]l-3',
wobei 1 eine ganze Zahl > 1 ist und denselben Wert wie k im ersten DNA-Molekül hat,
wobei das Zielsequenzelementrc eine kontinuierliche Sequenz von 15 bis 30 Desoxyribonukleotiden ist,
wobei das Loop-Sequenzelement die Sequenz 5'-(X)m-G-(Y)n-G-3' hat, wobei X A und m eine ganze Zahl 1 ist,
wobei Y A oder T ist und n eine ganze Zahl 1, 2 oder 3 ist, und
wobei X und Y so ausgewählt sind, dass keine Hybridisierung von zwei Molekülen stattfindet, die nur aus dem Loop-Sequenzelement bestehen und wobei das Loop-Sequenzelement nicht zu einer Sense- oder Antisense-Sequenz in besagtem mindestens einem Zielgen der RNA-Interferenz passt, wobei die Zielsequenzelementerc, gezählt vom 3'-Ende in den sich wiederholenden Einheiten des besagten zweiten DNA-Moleküls die entsprechenden umgekehrten Gegenstücke der Zielsequenzelemente gezählt vom 5'-Ende in den sich wiederholenden Einheiten des besagten ersten DNA-Moleküls sind und
wobei die Loop-Sequenzelemente in den sich wiederholenden Einheiten des besagten zweiten DNA-Moleküls nicht die entsprechenden umgekehrten Gegenstücke der Loop-Sequenzelemente in den sich wiederholenden Einheiten des besagten ersten DNA-Moleküls sind,
c. In-vitro-Transkribieren von besagtem mindestens einem ersten und mindestens einem zweiten DNA-Molekül unter Verwendung einer RNA-Polymerase, um mindestens ein entsprechendes erstes und mindestens ein zweites RNA-Molekül zu erhalten,
d. Hybridisieren des mindestens einen ersten und mindestens einen zweiten RNA-Moleküls aus Schritt c. um ein doppelsträngiges RNA-Molekül wie in Abb. 1 dargestellt zu erhalten,
e. Verdauen des in Schritt d. erhaltenen doppelsträngigen RNA-Moleküls mit RNase T1, die fähig ist die einzelsträngigen Loop-Sequenzelemente gegenüber den hybridisierten doppelsträngigen Abschnitten der in Schritt d. erhaltenen doppelsträngigen RNA bevorzugt zu erkennen, zu spalten und zu verdauen wodurch einzelsträngige RNA-Loops entfernt werden,
wobei die Sequenzen der Loop-Sequenzelemente so ausgewählt sind, dass doppelsträngige RNA-Moleküle durch Erkennung, Spaltung und Verdau von den einzelsträngigen Loop-Sequenzelementen durch Rnase T1 aus Schritt e. erhalten werden, wobei jeder Strang der besagten resultierenden doppelsträngigen Moleküle eine Länge von 15 bis 30 Nukleotiden hat und wobei besagte resultierende doppelsträngige RNA-Moleküle zur zielspezifischen RNA-Interferenz von mindestens einem Zielgen fähig sind.

2. Verfahren zur Herstellung unterschiedlicher doppelsträngiger RNA-Moleküle, wobei jeder Strang der besagten doppelsträngigen Moleküle eine Länge von 15 bis 30 Nukleotiden hat, wobei besagte doppelsträngige RNA-Moleküle zur zielspezifischen RNA-Interferenz von mindestens einem Zielgen befähigt sind, wobei besagtes Verfahren mindestens die Schritte umfasst:
a. Bereitstellen mindestens eines ersten DNA-Moleküls, das in 5'-3' Richtung als sich wiederholende Einheiten eine Nukleinsäuresequenz mit den folgenden Elementen umfasst:
5'- [(Zielsequenzelement) - (Loop-Sequenzelement)]k -(Zielsequenzelement) - (Loop-Sequenzelement)hp - [(Zielsequenzelement)rc - (Loop-Sequenzelement)]l-3',
wobei k eine ganze Zahl > 1 ist,
wobei 1 eine ganze Zahl > 1 ist und denselben Wert wie k hat,
wobei das Zielsequenzelement eine kontinuierliche Sequenz von 15 bis 30 Desoxyribonukleotiden ist, die "sense" zu einer Sequenz in besagtem mindestens einem Zielgen der RNA-Interferenz ist,
wobei das Loop-Sequenzelement die Sequenz 5'-(X)m-G-(Y)n-G-3' hat, wobei X A und m eine ganze Zahl 1 ist,
wobei Y A oder T ist und n eine ganze Zahl 1, 2 oder 3 ist, und
wobei X und Y so ausgewählt sind, dass keine Hybridisierung von zwei Molekülen stattfindet, die nur aus dem Loop-Sequenzelement bestehen und wobei das Loop-Sequenzelement nicht zu einer Sense- oder Antisense-Sequenz in besagtem mindestens einem Zielgen der RNA-Interferenz passt, wobei das (Loop-Sequenzelement)hp von ausreichender Länge ist um eine Haarnadelstruktur zu ergeben, die ein Selbsthybridisierungsmuster ermöglicht,
wobei das Zielsequenzelementrc eine kontinuierliche Sequenz von 15 bis 30 Desoxyribonukleotiden ist,
wobei die Zielsequenzelementerc, gezählt vom 3'-Ende die entsprechenden umgekehrten Gegenstücke der Zielsequenzelemente gezählt vom 5'-Ende sind,
wobei die Loop-Sequenzelemente, die dem (Loop-Sequenzelement)hp folgen nicht die entsprechenden umgekehrten Gegenstücke der Loop-Sequenzelemente vor den sich wiederholenden Einheiten des besagten zweiten DNA-Moleküls sind,
b. In-vitro-Transkribieren von besagten mindestens einem ersten DNA-Molekül unter Verwendung einer RNA-Polymerase, um mindestens ein entsprechendes erstes RNA-Molekül zu erhalten, das nach Hybridisierung die generelle Struktur wie in Abb. 2 dargestellt aufweist,
c. Verdauen des in Schritt b. erhaltenen doppelsträngigen RNA-Moleküls mit RNase T1, die fähig ist die einzelsträngigen Loop-Sequenzelemente gegenüber den hybridisierten doppelsträngigen Abschnitten der doppelsträngigen in Schritt b. erhaltenen RNA bevorzugt zu erkennen, zu spalten und zu verdauen wodurch einzelsträngige RNA-Loops entfernt werden,
wobei die Sequenzen der Loop-Sequenzelemente so ausgewählt sind, dass doppelsträngige RNA-Moleküle durch Erkennung, Spaltung und Verdau von den einzelsträngigen Loop-Sequenzelementen durch Rnase T1 aus Schritt c. erhalten werden, wobei jeder Strang der besagten resultierenden doppelsträngigen Moleküle eine Länge von 15 bis 30 Nukleotiden hat und wobei besagte resultierende doppelsträngige RNA-Moleküle zur zielspezifischen RNA-Interferenz von mindestens einem Zielgen fähig sind.

3. Verfahren gemäß einem der Ansprüche 1 oder 2, wobei die Anzahl der Zielsequenzelemente mindestens 2, mindestens 3, mindestens 4, mindestens 5, mindestens 6, mindestens 7, mindestens 8, mindestens 9, mindestens 10, mindestens 11, mindestens 12, mindestens 13, mindestens 14 oder mindestens 15 ist, wobei die Sequenz von besagten Zielsequenzelementen des mindestens einen ersten DNA-Moleküls, wie in Abb. 1 dargestellt, oder des mindestens einen DNA-Moleküls, wie in Abb. 2 dargestellt, "sense" zu den Sequenzen des besagten mindestens einen Zielgens der RNA-Interferenz ist, wobei die Sequenzen von den besagten Zielsequenzelementenrc des mindestens einen zweiten DNA-Moleküls, wie in Abb. 1 dargestellt, oder des mindestens einen DNA-Moleküls, wie in Abb. 2 dargestellt, die umgekehrten Gegenstücke der Sequenzen der Zielsequenzelemente von dem mindestens einen ersten DNA-Molekül, wie in Abb. 1 dargestellt, oder dem mindestens einen DNA-Molekül, wie in Abb. 2 dargestellt, sind, an welche sie hybridisieren, wobei die Loop-Sequenzelemente des mindestens einen ersten DNA-Moleküls nicht die umgekehrte Gegenstücken voneinander sind, und im Falle von dem mindestens einen DNA-Molekül, wie in Abb. 2 dargestellt, das (Loop-Sequenzelement)hp von mindestens ausreichender Länge ist um eine Haarnadelstruktur zu ergeben, die ein Selbsthybridisierungsmuster ermöglicht, wie in Abb. 2 dargestellt.

4. Verfahren gemäß einem der Ansprüche 1, 2 oder 3, wobei jeder Strang von besagten unterschiedlichen doppelsträngigen Molekülen eine Länge von 17 bis 25 Nukleotiden hat.

5. Verfahren gemäß einem der Ansprüche 1, 2, 3 oder 4, wobei jeder Strang von besagten unterschiedlichen doppelsträngigen Molekülen einen 3'-Überhang von 2 Nukleotiden hat.

6. Eine Kombination oder ein Kit von mindestens zwei DNA-Molekülen, welche nach in vitro Transkription, Hybridisierung und Verdau mit RNase T1 gemäß Anspruch 1 fähig sind doppelsträngige RNA-Moleküle bereitzustellen, wobei jeder Strang von besagten unterschiedlichen doppelsträngigen Molekülen eine Länge von 15 bis 30 Nukleotiden hat und wobei besagte doppelsträngige RNA-Moleküle zur zielspezifischen RNA-Interferenz von mindestens einem Zielgen fähig sind, wobei besagte mindestens zwei DNA-Moleküle die notwendigen Sequenzelemente aufweisen um nach in vitro Transkription und Hybridisierung ein RNA Molekül zu erhalten, das die generelle Struktur wie in Abb. 1 dargestellt aufweist,
wobei das Loop-Sequenzelement die Sequenz 5'-(X)m-G-(Y)n-G-3' hat,
wobei X A und m eine ganze Zahl 1 ist,
wobei Y A oder T ist und n eine ganze Zahl 1, 2 oder 3 ist, und
wobei X und Y so ausgewählt sind, dass keine Hybridisierung von zwei Molekülen stattfindet, die nur aus dem Loop-Sequenzelement bestehen und wobei das Loop-Sequenzelement nicht zu einer Sense- oder Antisense-Sequenz in besagtem mindestens einem Zielgen der RNA-Interferenz passt, so dass doppelsträngige RNA-Moleküle nach Spaltung und Verdau mit RNase T1 erhalten werden, die fähig ist bevorzugt die einzelsträngigen Loop-Sequenzelemente gegenüber doppelsträngigen Abshnitten der doppelsträngigen RNA zu erkennen und zu spalten, wodurch einzelsträngige RNA-Loops entfernt werden.

7. Eine Kombination oder ein Kit von mindestens einem DNA-Molekül, welches nach in vitro Transkription, Hybridisierung und Verdau mit RNase T1 gemäß Anspruch 2 fähig ist doppelsträngige RNA-Moleküle bereitzustellen, wobei jeder Strang von besagten doppelsträngigen Molekülen eine Länge von 15 bis 30 Nukleotiden hat und wobei besagte unterschiedliche doppelsträngige RNA-Moleküle zur zielspezifischen RNA-Interferenz von mindestens einem Zielgen fähig sind, wobei das besagte mindestens eine DNA-Molekül die notwendigen Sequenzelemente aufweist um nach in vitro Transkription und Hybridisierung ein RNA Molekül zu erhalten, das die generelle Struktur wie in Abb. 2 dargestellt, aufweist,
wobei das Loop-Sequenzelement die Sequenz 5'-(X)m-G-(Y)n-G-3' hat,
wobei X A und m eine ganze Zahl 1 ist,
wobei Y A oder T ist und n eine ganze Zahl 1, 2 oder 3 ist, und
wobei X und Y so ausgewählt sind, dass keine Hybridisierung von zwei Molekülen stattfindet, die nur aus dem Loop-Sequenzelement bestehen und wobei das Loop-Sequenzelement nicht zu einer Sense- oder Antisense-Sequenz in besagtem mindestens einem Zielgen der RNA-Interferenz passt, so dass doppelsträngige RNA-Moleküle nach Verdau mit RNase T1 erhalten werden, die fähig ist bevorzugt die einzelsträngigen Loop-Sequenzelemente gegenüber hybridisierten doppelsträngigen Abschnitten der doppelsträngigen RNA zu erkennen, zu spalten und zu verdauen, wodurch einzelsträngige RNA-Loops entfernt werden.

8. Die Kombination oder das Kit gemäß den Ansprüchen 6 oder 7, weiterhin umfassend:
a. optional eine RNA-Polymerase,
b. optional einen Puffer für die In-vitro-Transkription,
c. optional einen Puffer zur Hybridisierung,
d. optional RNase T1 und
e. optional schriftliche Anweisungen.

9. Verwendung eines Verfahrens gemäß einem der Ansprüche 1 bis 5 oder einer Kombination oder eines Kits gemäß einem der Ansprüche 6 bis 8 zur Herstellung von siRNA-Pools.

## Revendications

1. Procédé de préparation de molécules d'ARN double brin différentes, dans lequel chaque brin desdites molécules double brin différentes a une longueur de 15 à 30 nucléotides dans lequel lesdites molécules d'ARN double brin différentes sont capable d'interférence d'ARN spécifique de cible d'au moins un gène cible, ledit procédé comprenant au moins les étapes de :
a. fourniture d'au moins une première molécule d'ADN comprenant dans la direction 5'-3' comme motifs répétés une séquence d'acide nucléique avec les éléments suivants :
5'-[(élément de séquence cible)- (élément de séquence en boucle)]ₖ-3',
k étant un nombre entier > 1,
l'élément de séquence cible étant une séquence continue de 15 à 30 désoxyribonucléotides, qui est sens à une séquence dans ledit au moins un gène cible d'interférence d'ARN, l'élément de séquence en boucle ayant la séquence 5'-(X)ₘ-G-(Y)ₙ-G-3',
X étant A et m étant un nombre entier de 1,
Y étant A ou T et n étant un nombre entier de 1, 2 ou 3, et
X et Y étant choisis de telle manière qu'il n'y a aucune hybridation de deux molécules constituées juste de l'élément de séquence en boucle et dans lequel l'élément de séquence en boucle ne correspond pas à une séquence sens ou antisens dans ledit au moins un gène cible d'interférence d'ARN,
b. fourniture d'au moins une seconde molécule d'ADN comprenant dans la direction 5'-3' de manière répétée une séquence d'acide nucléique avec les éléments suivants :
5'-[(élément de séquence cible)_{rc} - (élément de séquence en boucle)ₗ-3',
l étant un nombre entier > 1 et ayant la même valeur que k dans la première molécule d'ADN,
l'élément de séquence ciblerc étant une séquence continue de 15 à 30 désoxyribonucléotides,
l'élément de séquence en boucle ayant la séquence 5'-(X)ₘ-G-(Y)ₙ-G-3',
X étant A et m étant un nombre entier de 1,
Y étant A ou T et n étant un nombre entier de 1, 2 ou 3, et
X et Y étant choisis de telle manière qu'il n'y a pas d'hybridation de deux molécules constituées juste de l'élément de séquence en boucle et dans lequel l'élément de séquence en boucle ne correspond pas à une séquence sens ou antisens dans ledit au moins un gène cible d'interférence d'ARN,
dans lequel les éléments de séquence cible_{rc} comptés à partir de l'extrémité 3' dans les motifs répétés de ladite seconde molécule d'ADN sont le complément inverse respectif des éléments de séquence cible comptés à partir de l'extrémité 5' dans les motifs répétés de ladite première molécule d'ADN, et
dans lequel les éléments de séquence en boucle dans les motifs répétés de ladite seconde molécule d'ADN ne sont pas les compléments inverses des éléments de séquence en boucle dans les motifs répétés de ladite première molécule d'ADN,
c. transcription *in vitro* de ladite au moins première et de ladite au moins seconde molécules d'ADN en utilisant une ARN polymérase pour obtenir les séquences correspondantes à au moins une première et au moins une seconde molécules d'ARN,
d. hybridation d'au moins une première et d'au moins une seconde molécules d'ARN de l'étape c. pour obtenir une molécule d'ARN double brin décrite sur la figure 1,
e. digestion de la molécule d'ARN double brin obtenue dans l'étape d. avec l'ARNase T1 capable de reconnaître préférentiellement, de cliver et de digérer les éléments de séquence en boucle simple brin obtenus dans l'étape d. enlevant ainsi les boucles d'ARN simple brin,
dans lequel les séquences des éléments de séquence en boucle sont choisis de telle manière que les molécules d'ARN double brin sont obtenues par reconnaissance, clivage et digestion des éléments de séquence en boucle simple brin par l'ARNase T1 dans l'étape e., dans lequel chaque brin desdites molécules double brin résultantes a une longueur de 15 à 30 nucléotides, dans lequel lesdites molécules d'ARN double brin résultantes sont capables d'interférence d'ARN spécifique de cible d'au moins un gène cible.

2. Procédé de préparation de molécules d'ARN double brin différentes, dans lequel chaque brin desdites molécules double brin a une longueur de 15 à 30 nucléotides, dans lequel lesdites molécules d'ARN double brin sont capables d'interférence d'ARN spécifique de cible d'au moins un gène cible, ledit procédé comprenant au moins les étapes de :
a. fourniture d'au moins une première molécule d'ADN comprenant dans la direction 5'-3' comme motifs répétés une séquence d'acide nucléique avec les éléments suivants :
5'-[(élément de séquence cible) - (élément de séquence en boucle)]ₖ -(élément de séquence cible) -(élément de séquence en boucle)ₕₚ - [(élément de séquence cible)_{rc} - (élément de séquence en boucle)]ₗ-3',
k étant un nombre entier > 1,
l étant un nombre entier > 1 et étant identique à k,
l'élément de séquence cible étant une séquence continue de 15 à 30 désoxyribonucléotides, qui est sens à une séquence dans ledit au moins un gène cible d'interférence d'ARN,
l'élément de séquence en boucle ayant la séquence 5'-(X)ₘ-G-(Y)ₙ-G-3',
X étant A et m un nombre entier de 1,
Y étant A ou T et n étant un nombre entier de 1, 2 ou 3, et
X et Y étant choisis de telle manière qu'il n'y a aucune hybridation de deux molécules constituées juste de l'élément de séquence en boucle et dans lequel l'élément de séquence en boucle ne correspond pas à une séquence sens ou antisens dans ledit au moins un gène cible d'interférence d'ARN,
dans lequel (l'élément de séquence en boucle)ₕₚ est de longueur suffisante pour permettre une structure en épingle à cheveux permettant un motif d'auto-hybridation,
l'élément de séquence_{rc} étant une séquence continue de 15 à 30 désoxyribonucléotides,
dans lequel les éléments de séquence cible_{rc} comptés à partir de l'extrémité 3' sont le complément inverse respectif des éléments de séquence cible comptés à partir de l'extrémité 5',
dans lequel les éléments de séquence en boucle suivant (l'élément de séquence en boucle)hp ne sont pas les compléments inverses des éléments de séquence en boucle précédents dans les motifs répétés de la seconde molécule d'ADN,
b. transcription *in vitro* d'au moins les premières molécules d'ADN en utilisant une ARN polymérase pour obtenir la au moins une première molécule d'ARN correspondante, après quoi l'hybridation fournit la structure générale décrite sur la figure 2,
c. digestion de la molécule d'ARN double brin obtenue dans l'étape b. avec l'ARNase T1 capable de reconnaître préférentiellement, de cliver et de digérer les éléments de séquence en boucle simple brin sur les sections double brin hybridées de l'ARN double brin obtenu dans l'étape b. enlevant ainsi les boucles d'ARN simple brin,
dans lequel les séquences des éléments de séquence en boucle sont choisies de telle manière que les molécules d'ARN double brin sont obtenues par reconnaissance, clivage et digestion des éléments de séquence en boucle simple brin par l'ARNase T1 dans l'étape c., dans lequel chaque brin desdites molécules double brin résultantes a une longueur de 15 à 30 nucléotides et dans lequel lesdites molécules d'ARN double brin résultantes sont capables d'interférence d'ARN spécifique de cible d'au moins un gène cible.

3. Procédé selon l'une quelconque des revendications 1 ou 2, dans lequel le nombre d'éléments de séquence cible est d'au moins 2, d'au moins 3, d'au moins 4, d'au moins 5, d'au moins 6, d'au moins 7, d'au moins 8, d'au moins 9, d'au moins 10, d'au moins 11, d'au moins 12, d'au moins 13, d'au moins 14 ou d'au moins 15, dans lequel la séquence desdits éléments de séquence cible de l'au moins une première molécule d'ADN telle que décrite sur la figure 1 ou de l'au moins une molécule d'ADN telle que décrite sur la figure 2 est sens aux séquences dudit au moins un gène cible d'interférence d'ARN, dans lequel les séquences desdits éléments de séquence cible_{rc} de l'au moins seconde molécule d'ADN telle que décrite sur la figure 1 ou de l'au moins une molécule d'ADN décrite telle que sur la figure 2 sont les compléments inverses des séquences des éléments de séquence cible de l'au moins une première molécule d'ADN telle que décrite sur la figure 1 ou de l'au moins une molécule d'ADN telle que décrite sur la figure 2, auxquelles elles s'hybrident, dans lequel les éléments de séquence en boucle des au moins une premières molécules d'ADN ne sont pas des compléments inverses l'un de l'autre, et, dans le cas de l'au moins une molécule d'ADN telle que décrite sur la figure 2 l'(élément de séquence en boucle)ₕₚ est de longueur suffisante pour permettre à une structure en épingle à cheveux de permettre un motif d'auto-hybridation tel que décrit sur la figure 2.

4. Procédé selon l'une quelconque des revendications 1, 2 ou 3, dans lequel chaque brin desdites molécules double brin différentes a une longueur de 17 à 25 nucléotides.

5. Procédé selon l'une quelconque des revendications 1, 2, 3 ou 4, dans lequel chaque brin desdites molécules double brin différentes a une avancée en 3' de 2 nucléotides.

6. Combinaison ou kit d'au moins deux molécules d'ADN, qui après transcription *in vitro,* hybridation et digestion avec l'ARNase T1 selon la revendication 1 sont capables de fournir des molécules d'ARN double brin, dans lequel chaque brin desdites molécules double brin différentes a une longueur de 15 à 30 nucléotides et dans lequel lesdites molécules d'ARN double brin sont capables d'interférence d'ARN spécifique de cible d'au moins un gène cible, dans lequel lesdites au moins deux molécules d'ADN ont les éléments de séquence nécessaires pour obtenir une molécule d'ARN de la structure générale décrite sur la figure 1 après transcription *in vitro* et hybridation,
dans lequel l'élément de séquence en boucle a la séquence 5'-(X)ₘ-G-(Y)ₙ-G-3',
X étant A et m un nombre entier de 1,
Y étant A ou T et n étant un nombre entier de 1, 2 ou 3, et
X et Y étant choisis de telle manière qu'il n'y a aucune hybridation de deux molécules constituées juste de l'élément de séquence en boucle et dans lequel l'élément de séquence en boucle ne correspond pas à une séquence sens ou antisens dans ledit au moins un gène cible d'interférence d'ARN, de telle manière que les molécules d'ARN double brin peuvent être obtenues après clivage et digestion avec l'ARNase T1 capable de reconnaître préférentiellement et de cliver les éléments de séquence en boucle simple brin sur les sections double brin hybridées de l'ARN double brin enlevant ainsi les boucles d'ARN simple brin.

7. Combinaison ou kit d'au moins une molécule d'ADN, qui après transcription *in vitro,* hybridation et digestion avec l'ARNase T1 selon la revendication 2 est capable de fournir des molécules d'ARN double brin, dans lequel chaque brin desdites molécules double brin a une longueur de 15 à 30 nucléotides et dans lequel lesdites molécules d'ARN double brin différentes sont capables d'interférence d'ARN spécifique de cible d'au moins un gène cible,
dans lequel ladite au moins une molécule d'ADN a les éléments de séquence nécessaires pour obtenir une molécule d'ARN de la structure générale de la figure 2 après transcription *in vitro* et hybridation,
dans lequel l'élément de séquence en boucle a la séquence 5'-(X)m-G-(Y)n-G-3'
X étant A et m étant un nombre entier de 1,
Y étant A ou T et n étant un nombre entier de 1, 2 ou 3 et
X et Y étant choisis de telle manière qu'il n'y a aucune hybridation de deux molécules consistant juste en l'élément de séquence en boucle et dans lequel l'élément de séquence en boucle ne correspond pas à une séquence sens ou antisens dans ledit au moins un gène cible d'interférence d'ARN, de telle manière que les molécules d'ARN double brin sont obtenues après digestion avec l'ARNse T1 capable de reconnaître préférentiellement, de cliver et de digérer les éléments de séquence en boucle simple brin sur les sections double brin hybridées de l'ARN double brin enlevant ainsi les boules d'ARN simple brin.

8. Combinaison ou kit selon la revendication 6 ou 7, comprenant en outre :
a. éventuellement une ARN polymérase,
b. éventuellement un tampon pour la transcription in vitro,
c. éventuellement un tampon pour l'hybridation,
d. éventuellement l'ARNase T1, et
e. éventuellement des instructions écrites.

9. Utilisation d'un procédé selon l'une quelconque des revendications 1 à 5 ou d'une combinaison ou d'un kit selon l'une quelconque des revendications 6 à 8 pour produire des pools d'ARNsi.


### SEQUENCE LISTING

<110> INTANA BIOSCIENCE GMBH, UNIVERSITÄT REGENSBURG
<120> High complexity siRNA pools
<130> I-8479-WO
<160> 546
<170> PatentIn version 3.5
<210> 1
   <211> 21
   <212> DNA
   <213> Artificial Sequence
<220>
   <223> siRNA, sense, complete sequence
<400> 1
   aaggcaaguu uggaaacgut t 21
<210> 2
   <211> 21
   <212> DNA
   <213> Artificial Sequence
<220>
   <223> siRNA, sense, complete sequence
<400> 2
   gaugcucuaa uguacugcct t 21
<210> 3
   <211> 21
   <212> DNA
   <213> Artificial Sequence
<220>
   <223> siRNA, sense, complete sequence
<400> 3
   gaagagcugc acauuugact t 21
<210> 4
   <211> 21
   <212> DNA
   <213> Artificial Sequence
<220>
   <223> siRNA, sense, complete sequence
<400> 4
   ucuuaacgcg gcacuucact t 21
<210> 5
   <211> 21
   <212> DNA
   <213> Artificial Sequence
<220>
   <223> siRNA, sense, complete sequence
<400> 5
   ucgucaaggu ggaccuaaat t 21
<210> 6
   <211> 21
   <212> DNA
   <213> Artificial Sequence
<220>
   <223> siRNA, sense, complete sequence
<400> 6
   ccaaacugcu caggcauaat t 21
<210> 7
   <211> 21
   <212> DNA
   <213> Artificial Sequence
<220>
   <223> siRNA, sense, complete sequence
<400> 7
   ggugauggag aauagcagut t 21
<210> 8
   <211> 21
   <212> DNA
   <213> Artificial Sequence
<220>
   <223> siRNA, sense, complete sequence
<400> 8
   ccugcgucuc uacaacuaut t 21
<210> 9
   <211> 21
   <212> DNA
   <213> Artificial Sequence
<220>
   <223> siRNA, sense, complete sequence
<400> 9
   gucccagaua gagaaggagt t 21
<210> 10
   <211> 21
   <212> DNA
   <213> Artificial Sequence
<220>
   <223> siRNA, sense, complete sequence
<400> 10
   gguccucuuc aagucccagt t 21
<210> 11
   <211> 21
   <212> DNA
   <213> Artificial Sequence
<220>
   <223> siRNA, sense, complete sequence
<400> 11
   ccaacauccu gcgucucuat t 21
<210> 12
   <211> 21
   <212> DNA
   <213> Artificial Sequence
<220>
   <223> siRNA, sense, complete sequence
<400> 12
   gacaaugugu ggcacccugt t 21
<210> 13
   <211> 21
   <212> DNA
   <213> Artificial Sequence
<220>
   <223> siRNA, sense, complete sequence
<400> 13
   gcagagagau cgaaauccat t 21
<210> 14
   <211> 21
   <212> DNA
   <213> Artificial Sequence
<220>
   <223> siRNA, sense, complete sequence
<400> 14
   gccagaaaau cugcucuuat t 21
<210> 15
   <211> 21
   <212> DNA
   <213> Artificial Sequence
<220>
   <223> siRNA, reverse complement antisense, complete sequence
<400> 15
   acguuuccaa acuugccuut g 21
<210> 16
   <211> 21
   <212> DNA
   <213> Artificial Sequence
<220>
   <223> siRNA, reverse complement antisense, complete sequence
<400> 16
   ggcaguacau uagagcauct g 21
<210> 17
   <211> 21
   <212> DNA
   <213> Artificial Sequence
<220>
   <223> siRNA, reverse complement antisense, complete sequence
<400> 17
   gucaaaugug cagcucuuct g 21
<210> 18
   <211> 21
   <212> DNA
   <213> Artificial Sequence
<220>
   <223> siRNA, reverse complement antisense, complete sequence
<400> 18
   gugaagugcc gcguuaagat g 21
<210> 19
   <211> 21
   <212> DNA
   <213> Artificial Sequence
<220>
   <223> siRNA, reverse complement antisense, complete sequence
<400> 19
   uuuaggucca ccuugacgat g 21
<210> 20
   <211> 21
   <212> DNA
   <213> Artificial Sequence
<220>
   <223> siRNA, reverse complement antisense, complete sequence
<400> 20
   uuaugccuga gcaguuugga g 21
<210> 21
   <211> 21
   <212> DNA
   <213> Artificial Sequence
<220>
   <223> siRNA, reverse complement antisense, complete sequence
<400> 21
   acugcuauuc uccaucacct t 21
<210> 22
   <211> 21
   <212> DNA
   <213> Artificial Sequence
<220>
   <223> siRNA, reverse complement antisense, complete sequence
<400> 22
   auaguuguag agacgcagga t 21
<210> 23
   <211> 21
   <212> DNA
   <213> Artificial Sequence
<220>
   <223> siRNA, reverse complement antisense, complete sequence
<400> 23
   cuccuucucu aucugggact t 21
<210> 24
   <211> 21
   <212> DNA
   <213> Artificial Sequence
<220>
   <223> siRNA, reverse complement antisense, complete sequence
<400> 24
   cugggacuug aagaggacct t 21
<210> 25
   <211> 21
   <212> DNA
   <213> Artificial Sequence
<220>
   <223> siRNA, reverse complement antisense, complete sequence
<400> 25
   uagagacgca ggauguuggg a 21
<210> 26
   <211> 21
   <212> DNA
   <213> Artificial Sequence
<220>
   <223> siRNA, reverse complement antisense, complete sequence
<400> 26
   cagggugcca cacauuguct t 21
<210> 27
   <211> 21
   <212> DNA
   <213> Artificial Sequence
<220>
   <223> siRNA, reverse complement antisense, complete sequence
<400> 27
   uggauuucga ucucucugcg c 21
<210> 28
   <211> 21
   <212> DNA
   <213> Artificial Sequence
<220>
   <223> siRNA, reverse complement antisense, complete sequence
<400> 28
   uaagagcaga uuuucuggct t 21
<210> 29
   <211> 19
   <212> DNA
   <213> Artificial Sequence
<220>
   <223> siRNA, sense, 19 bp core sequence without 3' overhang
<400> 29
   aaggcaaguu uggaaacgu 19
<210> 30
   <211> 19
   <212> DNA
   <213> Artificial Sequence
<220>
   <223> siRNA, sense, 19 bp core sequence without 3' overhang
<400> 30
   gaugcucuaa uguacugcc 19
<210> 31
   <211> 19
   <212> DNA
   <213> Artificial Sequence
<220>
   <223> siRNA, sense, 19 bp core sequence without 3' overhang
<400> 31
   gaagagcugc acauuugac 19
<210> 32
   <211> 19
   <212> DNA
   <213> Artificial Sequence
<220>
   <223> siRNA, sense, 19 bp core sequence without 3' overhang
<400> 32
   ucuuaacgcg gcacuucac 19
<210> 33
   <211> 19
   <212> DNA
   <213> Artificial Sequence
<220>
   <223> siRNA, sense, 19 bp core sequence without 3' overhang
<400> 33
   ucgucaaggu ggaccuaaa 19
<210> 34
   <211> 19
   <212> DNA
   <213> Artificial Sequence
<220>
   <223> siRNA, sense, 19 bp core sequence without 3' overhang
<400> 34
   ccaaacugcu caggcauaa 19
<210> 35
   <211> 19
   <212> DNA
   <213> Artificial Sequence
<220>
   <223> siRNA, sense, 19 bp core sequence without 3' overhang
<400> 35
   ggugauggag aauagcagu 19
<210> 36
   <211> 19
   <212> DNA
   <213> Artificial Sequence
<220>
   <223> siRNA, sense, 19 bp core sequence without 3' overhang
<400> 36
   ccugcgucuc uacaacuau 19
<210> 37
   <211> 19
   <212> DNA
   <213> Artificial Sequence
<220>
   <223> siRNA, sense, 19 bp core sequence without 3' overhang
<400> 37
   gucccagaua gagaaggag 19
<210> 38
   <211> 19
   <212> DNA
   <213> Artificial Sequence
<220>
   <223> siRNA, sense, 19 bp core sequence without 3' overhang
<400> 38
   gguccucuuc aagucccag 19
<210> 39
   <211> 19
   <212> DNA
   <213> Artificial Sequence
<220>
   <223> siRNA, sense, 19 bp core sequence without 3' overhang
<400> 39
   ccaacauccu gcgucucua 19
<210> 40
   <211> 19
   <212> DNA
   <213> Artificial Sequence
<220>
   <223> siRNA, sense, 19 bp core sequence without 3' overhang
<400> 40
   gacaaugugu ggcacccug 19
<210> 41
   <211> 19
   <212> DNA
   <213> Artificial Sequence
<220>
   <223> siRNA, sense, 19 bp core sequence without 3' overhang
<400> 41
   gcagagagau cgaaaucca 19
<210> 42
   <211> 19
   <212> DNA
   <213> Artificial Sequence
<220>
   <223> siRNA, sense, 19 bp core sequence without 3' overhang
<400> 42
   gccagaaaau cugcucuua 19
<210> 43
   <211> 19
   <212> DNA
   <213> Artificial Sequence
<220>
   <223> siRNA, reverse complement antisense, 19 bp core sequence without 3' overhang
<400> 43
   acguuuccaa acuugccuu 19
<210> 44
   <211> 19
   <212> DNA
   <213> Artificial Sequence
<220>
   <223> siRNA, reverse complement antisense, 19 bp core sequence without 3' overhang
<400> 44
   ggcaguacau uagagcauc 19
<210> 45
   <211> 19
   <212> DNA
   <213> Artificial Sequence
<220>
   <223> siRNA, reverse complement antisense, 19 bp core sequence without 3' overhang
<400> 45
   gucaaaugug cagcucuuc 19
<210> 46
   <211> 19
   <212> DNA
   <213> Artificial Sequence
<220>
   <223> siRNA, reverse complement antisense, 19 bp core sequence without 3' overhang
<400> 46
   gugaagugcc gcguuaaga 19
<210> 47
   <211> 19
   <212> DNA
   <213> Artificial Sequence
<220>
   <223> siRNA, reverse complement antisense, 19 bp core sequence without 3' overhang
<400> 47
   uuuaggucca ccuugacga 19
<210> 48
   <211> 19
   <212> DNA
   <213> Artificial Sequence
<220>
   <223> siRNA, reverse complement antisense, 19 bp core sequence without 3' overhang
<400> 48
   uuaugccuga gcaguuugg 19
<210> 49
   <211> 19
   <212> DNA
   <213> Artificial Sequence
<220>
   <223> siRNA, reverse complement antisense, 19 bp core sequence without 3' overhang
<400> 49
   acugcuauuc uccaucacc 19
<210> 50
   <211> 19
   <212> DNA
   <213> Artificial Sequence
<220>
   <223> siRNA, reverse complement antisense, 19 bp core sequence without 3' overhang
<400> 50
   auaguuguag agacgcagg 19
<210> 51
   <211> 19
   <212> DNA
   <213> Artificial Sequence
<220>
   <223> siRNA, reverse complement antisense, 19 bp core sequence without 3' overhang
<400> 51
   cuccuucucu aucugggac 19
<210> 52
   <211> 19
   <212> DNA
   <213> Artificial Sequence
<220>
   <223> siRNA, reverse complement antisense, 19 bp core sequence without 3' overhang
<400> 52
   cugggacuug aagaggacc 19
<210> 53
   <211> 19
   <212> DNA
   <213> Artificial Sequence
<220>
   <223> siRNA, reverse complement antisense, 19 bp core sequence without 3' overhang
<400> 53
   uagagacgca ggauguugg 19
<210> 54
   <211> 19
   <212> DNA
   <213> Artificial Sequence
<220>
   <223> siRNA, reverse complement antisense, 19 bp core sequence without 3' overhang
<400> 54
   cagggugcca cacauuguc 19
<210> 55
   <211> 19
   <212> DNA
   <213> Artificial Sequence
<220>
   <223> siRNA, reverse complement antisense, 19 bp core sequence without 3' overhang
<400> 55
   uggauuucga ucucucugc 19
<210> 56
   <211> 19
   <212> DNA
   <213> Artificial Sequence
<220>
   <223> siRNA, reverse complement antisense, 19 bp core sequence without 3' overhang
<400> 56
   uaagagcaga uuuucuggc 19
<210> 57
   <211> 1314
   <212> DNA
   <213> Homo sapiens
<400> 57
   cggggcggga gatttgaaaa gtccttggcc agggcgcggc gtggcagatt cagttgtttg      60
   cgggcggccg ggagagtagc agtgccttgg accccagctc tcctccccct ttctctctaa     120
   ggatggccca gaaggagaac tcctacccct ggccctacgg ccgacagacg gctccatctg     180
   gcctgagcac cctgccccag cgagtcctcc ggaaagagcc tgtcacccca tctgcacttg     240
   tcctcatgag ccgctccaat gtccagccca cagctgcccc tggccagaag gtgatggaga     300
   atagcagtgg gacacccgac atcttaacgc ggcacttcac aattgatgac tttgagattg     360
   ggcgtcctct gggcaaaggc aagtttggaa acgtgtactt ggctcgggag aagaaaagcc     420
   atttcatcgt ggcgctcaag gtcctcttca agtcccagat agagaaggag ggcgtggagc     480
   atcagctgcg cagagagatc gaaatccagg cccacctgca ccatcccaac atcctgcgtc     540
   tctacaacta tttttatgac cggaggagga tctacttgat tctagagtat gccccccgcg      600
   gggagctcta caaggagctg cagaagagct gcacatttga cgagcagcga acagccacga     660
   tcatggagga gttggcagat gctctaatgt actgccatgg gaagaaggtg attcacagag     720
   acataaagcc agaaaatctg ctcttagggc tcaagggaga gctgaagatt gctgacttcg     780
   gctggtctgt gcatgcgccc tccctgagga ggaagacaat gtgtggcacc ctggactacc     840
   tgcccccaga gatgattgag gggcgcatgc acaatgagaa ggtggatctg tggtgcattg     900
   gagtgctttg ctatgagctg ctggtgggga acccaccctt tgagagtgca tcacacaacg     960
   agacctatcg ccgcatcgtc aaggtggacc taaagttccc cgcttccgtg cccatgggag    1020
   cccaggacct catctccaaa ctgctcaggc ataacccctc ggaacggctg cccctggccc    1080
   aggtctcagc ccacccttgg gtccgggcca actctcggag ggtgctgcct ccctctgccc    1140
   ttcaatctgt cgcctgatgg tccctgtcat tcactcgggt gcgtgtgttt gtatgtctgt    1200
   gtatgtatag gggaaagaag ggatccctaa ctgttccctt atctgttttc tacctcctcc    1260
   tttgtttaat aaaggctgaa gctttttgta ctcatgaaaa aaaaaaaaaa aaaa          1314
<210> 58
   <211> 344
   <212> PRT
   <213> Homo sapiens
<400> 58
   Met Ala Gln Lys Glu Asn Ser Tyr Pro Trp Pro Tyr Gly Arg Gln Thr
   1               5                   10                  15
   Ala Pro Ser Gly Leu Ser Thr Leu Pro Gln Arg Val Leu Arg Lys Glu
               20                  25                  30
   Pro Val Thr Pro Ser Ala Leu Val Leu Met Ser Arg Ser Asn Val Gln
           35                  40                  45
   Pro Thr Ala Ala Pro Gly Gln Lys Val Met Glu Asn Ser Ser Gly Thr
       50                  55                  60
   Pro Asp Ile Leu Thr Arg His Phe Thr Ile Asp Asp Phe Glu Ile Gly
   65                  70                  75                  80
   Arg Pro Leu Gly Lys Gly Lys Phe Gly Asn Val Tyr Leu Ala Arg Glu
                   85                  90                  95
   Lys Lys Ser His Phe Ile Val Ala Leu Lys Val Leu Phe Lys Ser Gln
               100                 105                 110
   Ile Glu Lys Glu Gly Val Glu His Gln Leu Arg Arg Glu Ile Glu Ile
           115                 120                 125
   Gln Ala His Leu His His Pro Asn Ile Leu Arg Leu Tyr Asn Tyr Phe
       130                 135                 140
   Tyr Asp Arg Arg Arg Ile Tyr Leu Ile Leu Glu Tyr Ala Pro Arg Gly
   145                 150                 155                 160
   Glu Leu Tyr Lys Glu Leu Gln Lys Ser Cys Thr Phe Asp Glu Gln Arg
                   165                 170                 175
   Thr Ala Thr Ile Met Glu Glu Leu Ala Asp Ala Leu Met Tyr Cys His
               180                 185                 190
   Gly Lys Lys Val Ile His Arg Asp Ile Lys Pro Glu Asn Leu Leu Leu
           195                 200                 205
   Gly Leu Lys Gly Glu Leu Lys Ile Ala Asp Phe Gly Trp Ser Val His
       210                 215                 220
   Ala Pro Ser Leu Arg Arg Lys Thr Met Cys Gly Thr Leu Asp Tyr Leu
   225                 230                 235                 240
   Pro Pro Glu Met Ile Glu Gly Arg Met His Asn Glu Lys Val Asp Leu
                   245                 250                 255
   Trp Cys Ile Gly Val Leu Cys Tyr Glu Leu Leu Val Gly Asn Pro Pro
               260                 265                 270
   Phe Glu Ser Ala Ser His Asn Glu Thr Tyr Arg Arg Ile Val Lys Val
           275                 280                 285
   Asp Leu Lys Phe Pro Ala Ser Val Pro Met Gly Ala Gln Asp Leu Ile
       290                 295                 300
   Ser Lys Leu Leu Arg His Asn Pro Ser Glu Arg Leu Pro Leu Ala Gln
   305                 310                 315                 320
   Val Ser Ala His Pro Trp Val Arg Ala Asn Ser Arg Arg Val Leu Pro
                   325                 330                 335
   Pro Ser Ala Leu Gln Ser Val Ala
               340
<210> 59
   <211> 1224
   <212> DNA
   <213> Homo sapiens
<400> 59
   cggggcggga gatttgaaaa gtccttggcc agggcgcggc gtggcagatt cagttgtttg      60
   cgggcggccg ggagagtagc agtgccttgg accccaggct ccatctggcc tgagcaccct     120
   gccccagcga gtcctccgga aagagcctgt caccccatct gcacttgtcc tcatgagccg     180
   ctccaatgtc cagcccacag ctgcccctgg ccagaaggtg atggagaata gcagtgggac     240
   acccgacatc ttaacgcggc acttcacaat tgatgacttt gagattgggc gtcctctggg     300
   caaaggcaag tttggaaacg tgtacttggc tcgggagaag aaaagccatt tcatcgtggc     360
   gctcaaggtc ctcttcaagt cccagataga gaaggagggc gtggagcatc agctgcgcag     420
   agagatcgaa atccaggccc acctgcacca tcccaacatc ctgcgtctct acaactattt     480
   ttatgaccgg aggaggatct acttgattct agagtatgcc ccccgcgggg agctctacaa     540
   ggagctgcag aagagctgca catttgacga gcagcgaaca gccacgatca tggaggagtt     600
   ggcagatgct ctaatgtact gccatgggaa gaaggtgatt cacagagaca taaagccaga     660
   aaatctgctc ttagggctca agggagagct gaagattgct gacttcggct ggtctgtgca     720
   tgcgccctcc ctgaggagga agacaatgtg tggcaccctg gactacctgc ccccagagat    780
   gattgagggg cgcatgcaca atgagaaggt ggatctgtgg tgcattggag tgctttgcta     840
   tgagctgctg gtggggaacc caccctttga gagtgcatca cacaacgaga cctatcgccg     900
   catcgtcaag gtggacctaa agttccccgc ttccgtgccc atgggagccc aggacctcat     960
   ctccaaactg ctcaggcata acccctcgga acggctgccc ctggcccagg tctcagccca    1020
   cccttgggtc cgggccaact ctcggagggt gctgcctccc tctgcccttc aatctgtcgc    1080
   ctgatggtcc ctgtcattca ctcgggtgcg tgtgtttgta tgtctgtgta tgtatagggg    1140
   aaagaaggga tccctaactg ttcccttatc tgttttctac ctcctccttt gtttaataaa    1200
   ggctgaagct tttgtactcc atga                                            1224
<210> 60
   <211> 303
   <212> PRT
   <213> Homo sapiens
<400> 60
   Met Ser Arg Ser Asn Val Gln Pro Thr Ala Ala Pro Gly Gln Lys Val
   1               5                   10                  15
   Met Glu Asn Ser Ser Gly Thr Pro Asp Ile Leu Thr Arg His Phe Thr
               20                  25                  30
   Ile Asp Asp Phe Glu Ile Gly Arg Pro Leu Gly Lys Gly Lys Phe Gly
           35                  40                  45
   Asn Val Tyr Leu Ala Arg Glu Lys Lys Ser His Phe Ile Val Ala Leu
       50                  55                  60
   Lys Val Leu Phe Lys Ser Gln Ile Glu Lys Glu Gly Val Glu His Gln
   65                  70                  75                  80
   Leu Arg Arg Glu Ile Glu Ile Gln Ala His Leu His His Pro Asn Ile
                   85                  90                  95
   Leu Arg Leu Tyr Asn Tyr Phe Tyr Asp Arg Arg Arg Ile Tyr Leu Ile
               100                 105                 110
   Leu Glu Tyr Ala Pro Arg Gly Glu Leu Tyr Lys Glu Leu Gln Lys Ser
           115                 120                 125
   Cys Thr Phe Asp Glu Gln Arg Thr Ala Thr Ile Met Glu Glu Leu Ala
       130                 135                 140
   Asp Ala Leu Met Tyr Cys His Gly Lys Lys Val Ile His Arg Asp Ile
   145                 150                 155                 160
   Lys Pro Glu Asn Leu Leu Leu Gly Leu Lys Gly Glu Leu Lys Ile Ala
                   165                 170                 175
   Asp Phe Gly Trp Ser Val His Ala Pro Ser Leu Arg Arg Lys Thr Met
               180                 185                 190
   Cys Gly Thr Leu Asp Tyr Leu Pro Pro Glu Met Ile Glu Gly Arg Met
           195                 200                 205
   His Asn Glu Lys Val Asp Leu Trp Cys Ile Gly Val Leu Cys Tyr Glu
       210                 215                 220
   Leu Leu Val Gly Asn Pro Pro Phe Glu Ser Ala Ser His Asn Glu Thr
   225                 230                 235                 240
   Tyr Arg Arg Ile Val Lys Val Asp Leu Lys Phe Pro Ala Ser Val Pro
                   245                 250                 255
   Met Gly Ala Gln Asp Leu Ile Ser Lys Leu Leu Arg His Asn Pro Ser
               260                 265                 270
   Glu Arg Leu Pro Leu Ala Gln Val Ser Ala His Pro Trp Val Arg Ala
           275                 280                 285
   Asn Ser Arg Arg Val Leu Pro Pro Ser Ala Leu Gln Ser Val Ala
       290                 295                 300
<210> 61
   <211> 368
   <212> DNA
   <213> Artificial Sequence
<220>
   <223> sense template
<400> 61
   aagctttaat acgactcact ataggagttg gccagaaaat ctgctcttaa gttggcagag      60
   agatcgaaat ccaagttgga caatgtgtgg caccctgagt tgccaacatc ctgcgtctct     120
   aagttgggtc ctcttcaagt cccagagttg gtcccagata gagaaggaga gttgcctgcg     180
   tctctacaac tatagttggg tgatggagaa tagcagtagt tgccaaactg ctcaggcata     240
   aagttgtcgt caaggtggac ctaaaagttg tcttaacgcg gcacttcaca gttggaagag     300
   ctgcacattt gacagttgga tgctctaatg tactgccagt tgaaggcaag tttggaaacg     360
   tagaattc                                                              368
<210> 62
   <211> 368
   <212> DNA
   <213> Artificial Sequence
<220>
   <223> antisense template
<400> 62
   aagctttaat acgactcact ataggagttg acgtttccaa acttgcctta gttgggcagt      60
   acattagagc atcagttggt caaatgtgca gctcttcagt tggtgaagtg ccgcgttaag     120
   aagttgttta ggtccacctt gacgaagttg ttatgcctga gcagtttgga gttgactgct     180
   attctccatc accagttgat agttgtagag acgcaggagt tgctccttct ctatctggga     240
   cagttgctgg gacttgaaga ggaccagttg tagagacgca ggatgttgga gttgcagggt     300
   gccacacatt gtcagttgtg gatttcgatc tctctgcagt tgtaagagca gattttctgg     360
   cagaattc                                                              368
<210> 63
   <211> 21
   <212> DNA
   <213> Artificial Sequence
<220>
   <223> siRNA ID s495, sense
<400> 63
   ggugauggag aauagcagut t 21
<210> 64
   <211> 21
   <212> DNA
   <213> Artificial Sequence
<220>
   <223> siRNA ID s495, antisense
<400> 64
   acugcuauuc uccaucacct t 21
<210> 65
   <211> 21
   <212> DNA
   <213> Artificial Sequence
<220>
   <223> siRNA, sense, first negative control
<400> 65
   aguacugcuu acgauacggt t 21
<210> 66
   <211> 21
   <212> DNA
   <213> Artificial Sequence
<220>
   <223> siRNA, antisense, first negative control
<400> 66
   ccguaucgua agcaguacut t 21
<210> 67
   <211> 21
   <212> DNA
   <213> Artificial Sequence
<220>
   <223> siRNA, sense, second negative control
<400> 67
   uucuccgaac gugucacgut t 21
<210> 68
   <211> 21
   <212> DNA
   <213> Artificial Sequence
<220>
   <223> siRNA, antisense, second negative control
<400> 68
   acgugacacg uucggagaat t 21
<210> 69
   <211> 19
   <212> DNA
   <213> Artificial Sequence
<220>
   <223> siRNA, sense, 19 bp core sequence without 3' overhang
<400> 69
   cgagcaaatc ttcgggcaa 19
<210> 70
   <211> 19
   <212> DNA
   <213> Artificial Sequence
<220>
   <223> siRNA, sense, 19 bp core sequence without 3' overhang
<400> 70
   gcgtcgagca cctgcagaa 19
<210> 71
   <211> 19
   <212> DNA
   <213> Artificial Sequence
<220>
   <223> siRNA, sense, 19 bp core sequence without 3' overhang
<400> 71
   gccagaagtc ccagaggaa 19
<210> 72
   <211> 19
   <212> DNA
   <213> Artificial Sequence
<220>
   <223> siRNA, sense, 19 bp core sequence without 3' overhang
<400> 72
   ctaagaaggt gaagaagga 19
<210> 73
   <211> 19
   <212> DNA
   <213> Artificial Sequence
<220>
   <223> siRNA, sense, 19 bp core sequence without 3' overhang
<400> 73
   aggaggagtt tcaacaaga 19
<210> 74
   <211> 19
   <212> DNA
   <213> Artificial Sequence
<220>
   <223> siRNA, sense, 19 bp core sequence without 3' overhang
<400> 74
   ccacagagct cctgcccaa 19
<210> 75
   <211> 19
   <212> DNA
   <213> Artificial Sequence
<220>
   <223> siRNA, sense, 19 bp core sequence without 3' overhang
<400> 75
   gcttactaat gcagtttaa 19
<210> 76
   <211> 19
   <212> DNA
   <213> Artificial Sequence
<220>
   <223> siRNA, sense, 19 bp core sequence without 3' overhang
<400> 76
   caggaagagt ttatgacca 19
<210> 77
   <211> 19
   <212> DNA
   <213> Artificial Sequence
<220>
   <223> siRNA, sense, 19 bp core sequence without 3' overhang
<400> 77
   gataattgaa ctcaccaaa 19
<210> 78
   <211> 19
   <212> DNA
   <213> Artificial Sequence
<220>
   <223> siRNA, sense, 19 bp core sequence without 3' overhang
<400> 78
   ggtgtggact acaggacaa 19
<210> 79
   <211> 19
   <212> DNA
   <213> Artificial Sequence
<220>
   <223> siRNA, sense, 19 bp core sequence without 3' overhang
<400> 79
   cattgttgct tgttgggta 19
<210> 80
   <211> 19
   <212> DNA
   <213> Artificial Sequence
<220>
   <223> siRNA, sense, 19 bp core sequence without 3' overhang
<400> 80
   gggtgaagcg ctggatatt 19
<210> 81
   <211> 19
   <212> DNA
   <213> Artificial Sequence
<220>
   <223> siRNA, sense, 19 bp core sequence without 3' overhang
<400> 81
   ctgatgcagt gccctagaa 19
<210> 82
   <211> 19
   <212> DNA
   <213> Artificial Sequence
<220>
   <223> siRNA, sense, 19 bp core sequence without 3' overhang
<400> 82
   ggaaagaatt aatgctcta 19
<210> 83
   <211> 19
   <212> DNA
   <213> Artificial Sequence
<220>
   <223> siRNA, sense, 19 bp core sequence without 3' overhang
<400> 83
   gccccaaagt tcacattaa 19
<210> 84
   <211> 19
   <212> DNA
   <213> Artificial Sequence
<220>
   <223> siRNA, reverse complement antisense, 19 bp core sequence without 3' overhang
<400> 84
   ttgcccgaag atttgctcg 19
<210> 85
   <211> 19
   <212> DNA
   <213> Artificial Sequence
<220>
   <223> siRNA, reverse complement antisense, 19 bp core sequence without 3' overhang
<400> 85
   ttctgcaggt gctcgacgc 19
<210> 86
   <211> 19
   <212> DNA
   <213> Artificial Sequence
<220>
   <223> siRNA, reverse complement antisense, 19 bp core sequence without 3' overhang
<400> 86
   ttcctctggg acttctggc 19
<210> 87
   <211> 19
   <212> DNA
   <213> Artificial Sequence
<220>
   <223> siRNA, reverse complement antisense, 19 bp core sequence without 3' overhang
<400> 87
   tccttcttca ccttcttag 19
<210> 88
   <211> 19
   <212> DNA
   <213> Artificial Sequence
<220>
   <223> siRNA, reverse complement antisense, 19 bp core sequence without 3' overhang
<400> 88
   tcttgttgaa actcctcct 19
<210> 89
   <211> 19
   <212> DNA
   <213> Artificial Sequence
<220>
   <223> siRNA, reverse complement antisense, 19 bp core sequence without 3' overhang
<400> 89
   ttgggcagga gctctgtgg 19
<210> 90
   <211> 19
   <212> DNA
   <213> Artificial Sequence
<220>
   <223> siRNA, reverse complement antisense, 19 bp core sequence without 3' overhang
<400> 90
   ttaaactgca ttagtaagc 19
<210> 91
   <211> 19
   <212> DNA
   <213> Artificial Sequence
<220>
   <223> siRNA, reverse complement antisense, 19 bp core sequence without 3' overhang
<400> 91
   tggtcataaa ctcttcctg 19
<210> 92
   <211> 19
   <212> DNA
   <213> Artificial Sequence
<220>
   <223> siRNA, reverse complement antisense, 19 bp core sequence without 3' overhang
<400> 92
   tttggtgagt tcaattatc 19
<210> 93
   <211> 19
   <212> DNA
   <213> Artificial Sequence
<220>
   <223> siRNA, reverse complement antisense, 19 bp core sequence without 3' overhang
<400> 93
   ttgtcctgta gtccacacc 19
<210> 94
   <211> 19
   <212> DNA
   <213> Artificial Sequence
<220>
   <223> siRNA, reverse complement antisense, 19 bp core sequence without 3' overhang
<400> 94
   tacccaacaa gcaacaatg 19
<210> 95
   <211> 19
   <212> DNA
   <213> Artificial Sequence
<220>
   <223> siRNA, reverse complement antisense, 19 bp core sequence without 3' overhang
<400> 95
   aatatccagc gcttcaccc 19
<210> 96
   <211> 19
   <212> DNA
   <213> Artificial Sequence
<220>
   <223> siRNA, reverse complement antisense, 19 bp core sequence without 3' overhang
<400> 96
   ttctagggca ctgcatcag 19
<210> 97
   <211> 19
   <212> DNA
   <213> Artificial Sequence
<220>
   <223> siRNA, reverse complement antisense, 19 bp core sequence without 3' overhang
<400> 97
   tagagcatta attctttcc 19
<210> 98
   <211> 19
   <212> DNA
   <213> Artificial Sequence
<220>
   <223> siRNA, reverse complement antisense, 19 bp core sequence without 3' overhang
<400> 98
   ttaatgtgaa ctttggggc 19
<210> 99
   <211> 19
   <212> DNA
   <213> Artificial Sequence
<220>
   <223> siRNA, sense, 19 bp core sequence without 3' overhang
<400> 99
   cggcacaacc cattggaca 19
<210> 100
   <211> 19
   <212> DNA
   <213> Artificial Sequence
<220>
   <223> siRNA, sense, 19 bp core sequence without 3' overhang
<400> 100
   ccacaaagca aggccagaa 19
<210> 101
   <211> 19
   <212> DNA
   <213> Artificial Sequence
<220>
   <223> siRNA, sense, 19 bp core sequence without 3' overhang
<400> 101
   gagtcagaaa tgttcaata 19
<210> 102
   <211> 19
   <212> DNA
   <213> Artificial Sequence
<220>
   <223> siRNA, sense, 19 bp core sequence without 3' overhang
<400> 102
   ccatgaagga cattcgtga 19
<210> 103
   <211> 19
   <212> DNA
   <213> Artificial Sequence
<220>
   <223> siRNA, sense, 19 bp core sequence without 3' overhang
<400> 103
   gagagaggta caaagaaga 19
<210> 104
   <211> 19
   <212> DNA
   <213> Artificial Sequence
<220>
   <223> siRNA, sense, 19 bp core sequence without 3' overhang
<400> 104
   gaagaaggaa ccagccaca 19
<210> 105
   <211> 19
   <212> DNA
   <213> Artificial Sequence
<220>
   <223> siRNA, sense, 19 bp core sequence without 3' overhang
<400> 105
   ccatatggca aacggtaga 19
<210> 106
   <211> 19
   <212> DNA
   <213> Artificial Sequence
<220>
   <223> siRNA, sense, 19 bp core sequence without 3' overhang
<400> 106
   cggtagaaga actggatta 19
<210> 107
   <211> 19
   <212> DNA
   <213> Artificial Sequence
<220>
   <223> siRNA, sense, 19 bp core sequence without 3' overhang
<400> 107
   caaggaagtc acagtggaa 19
<210> 108
   <211> 19
   <212> DNA
   <213> Artificial Sequence
<220>
   <223> siRNA, sense, 19 bp core sequence without 3' overhang
<400> 108
   aagattcctt ctaactgaa 19
<210> 109
   <211> 19
   <212> DNA
   <213> Artificial Sequence
<220>
   <223> siRNA, sense, 19 bp core sequence without 3' overhang
<400> 109
   gaattcagtg ggttcagaa 19
<210> 110
   <211> 19
   <212> DNA
   <213> Artificial Sequence
<220>
   <223> siRNA, sense, 19 bp core sequence without 3' overhang
<400> 110
   gcagaagccc caaagttca 19
<210> 111
   <211> 19
   <212> DNA
   <213> Artificial Sequence
<220>
   <223> siRNA, sense, 19 bp core sequence without 3' overhang
<400> 111
   gctctgatgc agtgcccta 19
<210> 112
   <211> 19
   <212> DNA
   <213> Artificial Sequence
<220>
   <223> siRNA, sense, 19 bp core sequence without 3' overhang
<400> 112
   aattaatgct ctaacgtga 19
<210> 113
   <211> 19
   <212> DNA
   <213> Artificial Sequence
<220>
   <223> siRNA, sense, 19 bp core sequence without 3' overhang
<400> 113
   cgtgataaac ctgctccaa 19
<210> 114
   <211> 19
   <212> DNA
   <213> Artificial Sequence
<220>
   <223> siRNA, reverse complement antisense, 19 bp core sequence without 3' overhang
<400> 114
   tgtccaatgg gttgtgccg 19
<210> 115
   <211> 19
   <212> DNA
   <213> Artificial Sequence
<220>
   <223> siRNA, reverse complement antisense, 19 bp core sequence without 3' overhang
<400> 115
   ttctggcctt gctttgtgg 19
<210> 116
   <211> 19
   <212> DNA
   <213> Artificial Sequence
<220>
   <223> siRNA, reverse complement antisense, 19 bp core sequence without 3' overhang
<400> 116
   tattgaacat ttctgactc 19
<210> 117
   <211> 19
   <212> DNA
   <213> Artificial Sequence
<220>
   <223> siRNA, reverse complement antisense, 19 bp core sequence without 3' overhang
<400> 117
   tcacgaatgt ccttcatgg 19
<210> 118
   <211> 19
   <212> DNA
   <213> Artificial Sequence
<220>
   <223> siRNA, reverse complement antisense, 19 bp core sequence without 3' overhang
<400> 118
   tcttctttgt acctctctc 19
<210> 119
   <211> 19
   <212> DNA
   <213> Artificial Sequence
<220>
   <223> siRNA, reverse complement antisense, 19 bp core sequence without 3' overhang
<400> 119
   tgtggctggt tccttcttc 19
<210> 120
   <211> 19
   <212> DNA
   <213> Artificial Sequence
<220>
   <223> siRNA, reverse complement antisense, 19 bp core sequence without 3' overhang
<400> 120
   tctaccgttt gccatatgg 19
<210> 121
   <211> 19
   <212> DNA
   <213> Artificial Sequence
<220>
   <223> siRNA, reverse complement antisense, 19 bp core sequence without 3' overhang
<400> 121
   taatccagtt cttctaccg 19
<210> 122
   <211> 19
   <212> DNA
   <213> Artificial Sequence
<220>
   <223> siRNA, reverse complement antisense, 19 bp core sequence without 3' overhang
<400> 122
   ttccactgtg acttccttg 19
<210> 123
   <211> 19
   <212> DNA
   <213> Artificial Sequence
<220>
   <223> siRNA, reverse complement antisense, 19 bp core sequence without 3' overhang
<400> 123
   ttcagttaga aggaatctt 19
<210> 124
   <211> 19
   <212> DNA
   <213> Artificial Sequence
<220>
   <223> siRNA, reverse complement antisense, 19 bp core sequence without 3' overhang
<400> 124
   ttctgaaccc actgaattc 19
<210> 125
   <211> 19
   <212> DNA
   <213> Artificial Sequence
<220>
   <223> siRNA, reverse complement antisense, 19 bp core sequence without 3' overhang
<400> 125
   tgaactttgg ggcttctgc 19
<210> 126
   <211> 19
   <212> DNA
   <213> Artificial Sequence
<220>
   <223> siRNA, reverse complement antisense, 19 bp core sequence without 3' overhang
<400> 126
   tagggcactg catcagagc 19
<210> 127
   <211> 19
   <212> DNA
   <213> Artificial Sequence
<220>
   <223> siRNA, reverse complement antisense, 19 bp core sequence without 3' overhang
<400> 127
   tcacgttaga gcattaatt 19
<210> 128
   <211> 19
   <212> DNA
   <213> Artificial Sequence
<220>
   <223> siRNA, reverse complement antisense, 19 bp core sequence without 3' overhang
<400> 128
   ttggagcagg tttatcacg 19
<210> 129
   <211> 19
   <212> DNA
   <213> Artificial Sequence
<220>
   <223> siRNA, sense, 19 bp core sequence without 3' overhang
<400> 129
   gcagaggtgc acagacttt 19
<210> 130
   <211> 19
   <212> DNA
   <213> Artificial Sequence
<220>
   <223> siRNA, sense, 19 bp core sequence without 3' overhang
<400> 130
   gtgagaactt ccaggacct 19
<210> 131
   <211> 19
   <212> DNA
   <213> Artificial Sequence
<220>
   <223> siRNA, sense, 19 bp core sequence without 3' overhang
<400> 131
   gagatgaaga agtcgttga 19
<210> 132
   <211> 19
   <212> DNA
   <213> Artificial Sequence
<220>
   <223> siRNA, sense, 19 bp core sequence without 3' overhang
<400> 132
   caggagagag gtacaaaga 19
<210> 133
   <211> 19
   <212> DNA
   <213> Artificial Sequence
<220>
   <223> siRNA, sense, 19 bp core sequence without 3' overhang
<400> 133
   aagctaagaa ggtgaagaa 19
<210> 134
   <211> 19
   <212> DNA
   <213> Artificial Sequence
<220>
   <223> siRNA, sense, 19 bp core sequence without 3' overhang
<400> 134
   gcagtgagga ggaggagtt 19
<210> 135
   <211> 19
   <212> DNA
   <213> Artificial Sequence
<220>
   <223> siRNA, sense, 19 bp core sequence without 3' overhang
<400> 135
   tagaagaact ggattactt 19
<210> 136
   <211> 19
   <212> DNA
   <213> Artificial Sequence
<220>
   <223> siRNA, sense, 19 bp core sequence without 3' overhang
<400> 136
   ggtaatagct gtaatgtgg 19
<210> 137
   <211> 19
   <212> DNA
   <213> Artificial Sequence
<220>
   <223> siRNA, sense, 19 bp core sequence without 3' overhang
<400> 137
   gggcatcagc cgtgagcat 19
<210> 138
   <211> 19
   <212> DNA
   <213> Artificial Sequence
<220>
   <223> siRNA, sense, 19 bp core sequence without 3' overhang
<400> 138
   tgcgcaaggt ccagagaga 19
<210> 139
   <211> 19
   <212> DNA
   <213> Artificial Sequence
<220>
   <223> siRNA, sense, 19 bp core sequence without 3' overhang
<400> 139
   agagagaaac tgcaaggaa 19
<210> 140
   <211> 19
   <212> DNA
   <213> Artificial Sequence
<220>
   <223> siRNA, sense, 19 bp core sequence without 3' overhang
<400> 140
   gcagttgaat tcagtgggt 19
<210> 141
   <211> 19
   <212> DNA
   <213> Artificial Sequence
<220>
   <223> siRNA, sense, 19 bp core sequence without 3' overhang
<400> 141
   gactacagga caaggggca 19
<210> 142
   <211> 19
   <212> DNA
   <213> Artificial Sequence
<220>
   <223> siRNA, sense, 19 bp core sequence without 3' overhang
<400> 142
   gttcacatta actcaggca 19
<210> 143
   <211> 19
   <212> DNA
   <213> Artificial Sequence
<220>
   <223> siRNA, sense, 19 bp core sequence without 3' overhang
<400> 143
   aatgctctaa cgtgataaa 19
<210> 144
   <211> 19
   <212> DNA
   <213> Artificial Sequence
<220>
   <223> siRNA, reverse complement antisense, 19 bp core sequence without 3' overhang
<400> 144
   aaagtctgtg cacctctgc 19
<210> 145
   <211> 19
   <212> DNA
   <213> Artificial Sequence
<220>
   <223> siRNA, reverse complement antisense, 19 bp core sequence without 3' overhang
<400> 145
   aggtcctgga agttctcac 19
<210> 146
   <211> 19
   <212> DNA
   <213> Artificial Sequence
<220>
   <223> siRNA, reverse complement antisense, 19 bp core sequence without 3' overhang
<400> 146
   tcaacgactt cttcatctc 19
<210> 147
   <211> 19
   <212> DNA
   <213> Artificial Sequence
<220>
   <223> siRNA, reverse complement antisense, 19 bp core sequence without 3' overhang
<400> 147
   tctttgtacc tctctcctg 19
<210> 148
   <211> 19
   <212> DNA
   <213> Artificial Sequence
<220>
   <223> siRNA, reverse complement antisense, 19 bp core sequence without 3' overhang
<400> 148
   ttcttcacct tcttagctt 19
<210> 149
   <211> 19
   <212> DNA
   <213> Artificial Sequence
<220>
   <223> siRNA, reverse complement antisense, 19 bp core sequence without 3' overhang
<400> 149
   aactcctcct cctcactgc 19
<210> 150
   <211> 19
   <212> DNA
   <213> Artificial Sequence
<220>
   <223> siRNA, reverse complement antisense, 19 bp core sequence without 3' overhang
<400> 150
   aagtaatcca gttcttcta 19
<210> 151
   <211> 19
   <212> DNA
   <213> Artificial Sequence
<220>
   <223> siRNA, reverse complement antisense, 19 bp core sequence without 3' overhang
<400> 151
   ccacattaca gctattacc 19
<210> 152
   <211> 19
   <212> DNA
   <213> Artificial Sequence
<220>
   <223> siRNA, reverse complement antisense, 19 bp core sequence without 3' overhang
<400> 152
   atgctcacgg ctgatgccc 19
<210> 153
   <211> 19
   <212> DNA
   <213> Artificial Sequence
<220>
   <223> siRNA, reverse complement antisense, 19 bp core sequence without 3' overhang
<400> 153
   tctctctgga ccttgcgca 19
<210> 154
   <211> 19
   <212> DNA
   <213> Artificial Sequence
<220>
   <223> siRNA, reverse complement antisense, 19 bp core sequence without 3' overhang
<400> 154
   ttccttgcag tttctctct 19
<210> 155
   <211> 19
   <212> DNA
   <213> Artificial Sequence
<220>
   <223> siRNA, reverse complement antisense, 19 bp core sequence without 3' overhang
<400> 155
   acccactgaa ttcaactgc 19
<210> 156
   <211> 19
   <212> DNA
   <213> Artificial Sequence
<220>
   <223> siRNA, reverse complement antisense, 19 bp core sequence without 3' overhang
<400> 156
   tgccccttgt cctgtagtc 19
<210> 157
   <211> 19
   <212> DNA
   <213> Artificial Sequence
<220>
   <223> siRNA, reverse complement antisense, 19 bp core sequence without 3' overhang
<400> 157
   tgcctgagtt aatgtgaac 19
<210> 158
   <211> 19
   <212> DNA
   <213> Artificial Sequence
<220>
   <223> siRNA, reverse complement antisense, 19 bp core sequence without 3' overhang
<400> 158
   tttatcacgt tagagcatt 19
<210> 159
   <211> 19
   <212> DNA
   <213> Artificial Sequence
<220>
   <223> siRNA, sense, 19 bp core sequence without 3' overhang
<400> 159
   gagaaggagc ctcgagaac 19
<210> 160
   <211> 19
   <212> DNA
   <213> Artificial Sequence
<220>
   <223> siRNA, sense, 19 bp core sequence without 3' overhang
<400> 160
   tgaagaagtc gttgatgga 19
<210> 161
   <211> 19
   <212> DNA
   <213> Artificial Sequence
<220>
   <223> siRNA, sense, 19 bp core sequence without 3' overhang
<400> 161
   aagaaagcta agaaggtga 19
<210> 162
   <211> 19
   <212> DNA
   <213> Artificial Sequence
<220>
   <223> siRNA, sense, 19 bp core sequence without 3' overhang
<400> 162
   gtgaggagga ggagtttca 19
<210> 163
   <211> 19
   <212> DNA
   <213> Artificial Sequence
<220>
   <223> siRNA, sense, 19 bp core sequence without 3' overhang
<400> 163
   atggcaaacg gtagaagaa 19
<210> 164
   <211> 19
   <212> DNA
   <213> Artificial Sequence
<220>
   <223> siRNA, sense, 19 bp core sequence without 3' overhang
<400> 164
   cttacaacga cgtggacat 19
<210> 165
   <211> 19
   <212> DNA
   <213> Artificial Sequence
<220>
   <223> siRNA, sense, 19 bp core sequence without 3' overhang
<400> 165
   ctgagaaggc ccagcagat 19
<210> 166
   <211> 19
   <212> DNA
   <213> Artificial Sequence
<220>
   <223> siRNA, sense, 19 bp core sequence without 3' overhang
<400> 166
   cgcaaggtcc agagagaaa 19
<210> 167
   <211> 19
   <212> DNA
   <213> Artificial Sequence
<220>
   <223> siRNA, sense, 19 bp core sequence without 3' overhang
<400> 167
   aggaagtcac agtggaaga 19
<210> 168
   <211> 19
   <212> DNA
   <213> Artificial Sequence
<220>
   <223> siRNA, sense, 19 bp core sequence without 3' overhang
<400> 168
   ggaagaagtg ggaggtggt 19
<210> 169
   <211> 19
   <212> DNA
   <213> Artificial Sequence
<220>
   <223> siRNA, sense, 19 bp core sequence without 3' overhang
<400> 169
   gctcccaaac tcaggcttt 19
<210> 170
   <211> 19
   <212> DNA
   <213> Artificial Sequence
<220>
   <223> siRNA, sense, 19 bp core sequence without 3' overhang
<400> 170
   gggcattgtt gcttgttgg 19
<210> 171
   <211> 19
   <212> DNA
   <213> Artificial Sequence
<220>
   <223> siRNA, sense, 19 bp core sequence without 3' overhang
<400> 171
   cattaactca ggcatttca 19
<210> 172
   <211> 19
   <212> DNA
   <213> Artificial Sequence
<220>
   <223> siRNA, sense, 19 bp core sequence without 3' overhang
<400> 172
   ctagaagggg aaagaatta 19
<210> 173
   <211> 19
   <212> DNA
   <213> Artificial Sequence
<220>
   <223> siRNA, sense, 19 bp core sequence without 3' overhang
<400> 173
   ttaatgctct aacgtgata 19
<210> 174
   <211> 19
   <212> DNA
   <213> Artificial Sequence
<220>
   <223> siRNA, reverse complement antisense, 19 bp core sequence without 3' overhang
<400> 174
   gttctcgagg ctccttctc 19
<210> 175
   <211> 19
   <212> DNA
   <213> Artificial Sequence
<220>
   <223> siRNA, reverse complement antisense, 19 bp core sequence without 3' overhang
<400> 175
   tccatcaacg acttcttca 19
<210> 176
   <211> 19
   <212> DNA
   <213> Artificial Sequence
<220>
   <223> siRNA, reverse complement antisense, 19 bp core sequence without 3' overhang
<400> 176
   tcaccttctt agctttctt 19
<210> 177
   <211> 19
   <212> DNA
   <213> Artificial Sequence
<220>
   <223> siRNA, reverse complement antisense, 19 bp core sequence without 3' overhang
<400> 177
   tgaaactcct cctcctcac 19
<210> 178
   <211> 19
   <212> DNA
   <213> Artificial Sequence
<220>
   <223> siRNA, reverse complement antisense, 19 bp core sequence without 3' overhang
<400> 178
   ttcttctacc gtttgccat 19
<210> 179
   <211> 19
   <212> DNA
   <213> Artificial Sequence
<220>
   <223> siRNA, reverse complement antisense, 19 bp core sequence without 3' overhang
<400> 179
   atgtccacgt cgttgtaag 19
<210> 180
   <211> 19
   <212> DNA
   <213> Artificial Sequence
<220>
   <223> siRNA, reverse complement antisense, 19 bp core sequence without 3' overhang
<400> 180
   atctgctggg ccttctcag 19
<210> 181
   <211> 19
   <212> DNA
   <213> Artificial Sequence
<220>
   <223> siRNA, reverse complement antisense, 19 bp core sequence without 3' overhang
<400> 181
   tttctctctg gaccttgcg 19
<210> 182
   <211> 19
   <212> DNA
   <213> Artificial Sequence
<220>
   <223> siRNA, reverse complement antisense, 19 bp core sequence without 3' overhang
<400> 182
   tcttccactg tgacttcct 19
<210> 183
   <211> 19
   <212> DNA
   <213> Artificial Sequence
<220>
   <223> siRNA, reverse complement antisense, 19 bp core sequence without 3' overhang
<400> 183
   accacctccc acttcttcc 19
<210> 184
   <211> 19
   <212> DNA
   <213> Artificial Sequence
<220>
   <223> siRNA, reverse complement antisense, 19 bp core sequence without 3' overhang
<400> 184
   aaagcctgag tttgggagc 19
<210> 185
   <211> 19
   <212> DNA
   <213> Artificial Sequence
<220>
   <223> siRNA, reverse complement antisense, 19 bp core sequence without 3' overhang
<400> 185
   ccaacaagca acaatgccc 19
<210> 186
   <211> 19
   <212> DNA
   <213> Artificial Sequence
<220>
   <223> siRNA, reverse complement antisense, 19 bp core sequence without 3' overhang
<400> 186
   tgaaatgcct gagttaatg 19
<210> 187
   <211> 19
   <212> DNA
   <213> Artificial Sequence
<220>
   <223> siRNA, reverse complement antisense, 19 bp core sequence without 3' overhang
<400> 187
   taattctttc cccttctag 19
<210> 188
   <211> 19
   <212> DNA
   <213> Artificial Sequence
<220>
   <223> siRNA, reverse complement antisense, 19 bp core sequence without 3' overhang
<400> 188
   tatcacgtta gagcattaa 19
<210> 189
   <211> 19
   <212> DNA
   <213> Artificial Sequence
<220>
   <223> siRNA, sense, 19 bp core sequence without 3' overhang
<400> 189
   cgttgggaat atacctcaa 19
<210> 190
   <211> 19
   <212> DNA
   <213> Artificial Sequence
<220>
   <223> siRNA, sense, 19 bp core sequence without 3' overhang
<400> 190
   gcagagtggt cagagagaa 19
<210> 191
   <211> 19
   <212> DNA
   <213> Artificial Sequence
<220>
   <223> siRNA, sense, 19 bp core sequence without 3' overhang
<400> 191
   gcaagagcct ggacgcatt 19
<210> 192
   <211> 19
   <212> DNA
   <213> Artificial Sequence
<220>
   <223> siRNA, sense, 19 bp core sequence without 3' overhang
<400> 192
   gaggatttct gtcggcaca 19
<210> 193
   <211> 19
   <212> DNA
   <213> Artificial Sequence
<220>
   <223> siRNA, sense, 19 bp core sequence without 3' overhang
<400> 193
   gagtatcagc agaagatca 19
<210> 194
   <211> 19
   <212> DNA
   <213> Artificial Sequence
<220>
   <223> siRNA, sense, 19 bp core sequence without 3' overhang
<400> 194
   gtacatggct tcctggaca 19
<210> 195
   <211> 19
   <212> DNA
   <213> Artificial Sequence
<220>
   <223> siRNA, sense, 19 bp core sequence without 3' overhang
<400> 195
   ggctacaggc caaggatga 19
<210> 196
   <211> 19
   <212> DNA
   <213> Artificial Sequence
<220>
   <223> siRNA, sense, 19 bp core sequence without 3' overhang
<400> 196
   gctctgcggt ctcactgta 19
<210> 197
   <211> 19
   <212> DNA
   <213> Artificial Sequence
<220>
   <223> siRNA, sense, 19 bp core sequence without 3' overhang
<400> 197
   ggagcttcct gtccaaatt 19
<210> 198
   <211> 19
   <212> DNA
   <213> Artificial Sequence
<220>
   <223> siRNA, sense, 19 bp core sequence without 3' overhang
<400> 198
   ggagaaggat gtccatgca 19
<210> 199
   <211> 19
   <212> DNA
   <213> Artificial Sequence
<220>
   <223> siRNA, sense, 19 bp core sequence without 3' overhang
<400> 199
   gaccacaaat cctccaaat 19
<210> 200
   <211> 19
   <212> DNA
   <213> Artificial Sequence
<220>
   <223> siRNA, sense, 19 bp core sequence without 3' overhang
<400> 200
   gcctcatcca caacaatgt 19
<210> 201
   <211> 19
   <212> DNA
   <213> Artificial Sequence
<220>
   <223> siRNA, sense, 19 bp core sequence without 3' overhang
<400> 201
   gccatctcac gtgtacata 19
<210> 202
   <211> 19
   <212> DNA
   <213> Artificial Sequence
<220>
   <223> siRNA, sense, 19 bp core sequence without 3' overhang
<400> 202
   gagccacaat aaattctat 19
<210> 203
   <211> 19
   <212> DNA
   <213> Artificial Sequence
<220>
   <223> siRNA, sense, 19 bp core sequence without 3' overhang
<400> 203
   gtcgacaggt caaggctga 19
<210> 204
   <211> 19
   <212> DNA
   <213> Artificial Sequence
<220>
   <223> siRNA, reverse complement antisense, 19 bp core sequence without 3' overhang
<400> 204
   ttgaggtata ttcccaacg 19
<210> 205
   <211> 19
   <212> DNA
   <213> Artificial Sequence
<220>
   <223> siRNA, reverse complement antisense, 19 bp core sequence without 3' overhang
<400> 205
   ttctctctga ccactctgc 19
<210> 206
   <211> 19
   <212> DNA
   <213> Artificial Sequence
<220>
   <223> siRNA, reverse complement antisense, 19 bp core sequence without 3' overhang
<400> 206
   aatgcgtcca ggctcttgc 19
<210> 207
   <211> 19
   <212> DNA
   <213> Artificial Sequence
<220>
   <223> siRNA, reverse complement antisense, 19 bp core sequence without 3' overhang
<400> 207
   tgtgccgaca gaaatcctc 19
<210> 208
   <211> 19
   <212> DNA
   <213> Artificial Sequence
<220>
   <223> siRNA, reverse complement antisense, 19 bp core sequence without 3' overhang
<400> 208
   tgatcttctg ctgatactc 19
<210> 209
   <211> 19
   <212> DNA
   <213> Artificial Sequence
<220>
   <223> siRNA, reverse complement antisense, 19 bp core sequence without 3' overhang
<400> 209
   tgtccaggaa gccatgtac 19
<210> 210
   <211> 19
   <212> DNA
   <213> Artificial Sequence
<220>
   <223> siRNA, reverse complement antisense, 19 bp core sequence without 3' overhang
<400> 210
   tcatccttgg cctgtagcc 19
<210> 211
   <211> 19
   <212> DNA
   <213> Artificial Sequence
<220>
   <223> siRNA, reverse complement antisense, 19 bp core sequence without 3' overhang
<400> 211
   tacagtgaga ccgcagagc 19
<210> 212
   <211> 19
   <212> DNA
   <213> Artificial Sequence
<220>
   <223> siRNA, reverse complement antisense, 19 bp core sequence without 3' overhang
<400> 212
   aatttggaca ggaagctcc 19
<210> 213
   <211> 19
   <212> DNA
   <213> Artificial Sequence
<220>
   <223> siRNA, reverse complement antisense, 19 bp core sequence without 3' overhang
<400> 213
   tgcatggaca tccttctcc 19
<210> 214
   <211> 19
   <212> DNA
   <213> Artificial Sequence
<220>
   <223> siRNA, reverse complement antisense, 19 bp core sequence without 3' overhang
<400> 214
   atttggagga tttgtggtc 19
<210> 215
   <211> 19
   <212> DNA
   <213> Artificial Sequence
<220>
   <223> siRNA, reverse complement antisense, 19 bp core sequence without 3' overhang
<400> 215
   acattgttgt ggatgaggc 19
<210> 216
   <211> 19
   <212> DNA
   <213> Artificial Sequence
<220>
   <223> siRNA, reverse complement antisense, 19 bp core sequence without 3' overhang
<400> 216
   tatgtacacg tgagatggc 19
<210> 217
   <211> 19
   <212> DNA
   <213> Artificial Sequence
<220>
   <223> siRNA, reverse complement antisense, 19 bp core sequence without 3' overhang
<400> 217
   atagaattta ttgtggctc 19
<210> 218
   <211> 19
   <212> DNA
   <213> Artificial Sequence
<220>
   <223> siRNA, reverse complement antisense, 19 bp core sequence without 3' overhang
<400> 218
   tcagccttga cctgtcgac 19
<210> 219
   <211> 19
   <212> DNA
   <213> Artificial Sequence
<220>
   <223> siRNA, sense, 19 bp core sequence without 3' overhang
<400> 219
   tcgatggact ggagacaga 19
<210> 220
   <211> 19
   <212> DNA
   <213> Artificial Sequence
<220>
   <223> siRNA, sense, 19 bp core sequence without 3' overhang
<400> 220
   ggcagagtgg tcagagaga 19
<210> 221
   <211> 19
   <212> DNA
   <213> Artificial Sequence
<220>
   <223> siRNA, sense, 19 bp core sequence without 3' overhang
<400> 221
   cagcagacat gtggcgctt 19
<210> 222
   <211> 19
   <212> DNA
   <213> Artificial Sequence
<220>
   <223> siRNA, sense, 19 bp core sequence without 3' overhang
<400> 222
   gtgagctggt gggagcaaa 19
<210> 223
   <211> 19
   <212> DNA
   <213> Artificial Sequence
<220>
   <223> siRNA, sense, 19 bp core sequence without 3' overhang
<400> 223
   cagcccgctt cctgcagaa 19
<210> 224
   <211> 19
   <212> DNA
   <213> Artificial Sequence
<220>
   <223> siRNA, sense, 19 bp core sequence without 3' overhang
<400> 224
   gaggagtatc agcagaaga 19
<210> 225
   <211> 19
   <212> DNA
   <213> Artificial Sequence
<220>
   <223> siRNA, sense, 19 bp core sequence without 3' overhang
<400> 225
   caaagctgaa cgaggccaa 19
<210> 226
   <211> 19
   <212> DNA
   <213> Artificial Sequence
<220>
   <223> siRNA, sense, 19 bp core sequence without 3' overhang
<400> 226
   ttgcacggct acaggccaa 19
<210> 227
   <211> 19
   <212> DNA
   <213> Artificial Sequence
<220>
   <223> siRNA, sense, 19 bp core sequence without 3' overhang
<400> 227
   cactgtagat cctgagaaa 19
<210> 228
   <211> 19
   <212> DNA
   <213> Artificial Sequence
<220>
   <223> siRNA, sense, 19 bp core sequence without 3' overhang
<400> 228
   tggaggaagt ggagaagga 19
<210> 229
   <211> 19
   <212> DNA
   <213> Artificial Sequence
<220>
   <223> siRNA, sense, 19 bp core sequence without 3' overhang
<400> 229
   agacgcagga ggaggacaa 19
<210> 230
   <211> 19
   <212> DNA
   <213> Artificial Sequence
<220>
   <223> siRNA, sense, 19 bp core sequence without 3' overhang
<400> 230
   cgactggagc agctgggaa 19
<210> 231
   <211> 19
   <212> DNA
   <213> Artificial Sequence
<220>
   <223> siRNA, sense, 19 bp core sequence without 3' overhang
<400> 231
   ccgagaggaa ggtggccaa 19
<210> 232
   <211> 19
   <212> DNA
   <213> Artificial Sequence
<220>
   <223> siRNA, sense, 19 bp core sequence without 3' overhang
<400> 232
   catctcacgt gtacataat 19
<210> 233
   <211> 19
   <212> DNA
   <213> Artificial Sequence
<220>
   <223> siRNA, sense, 19 bp core sequence without 3' overhang
<400> 233
   cataatcaga gccacaata 19
<210> 234
   <211> 19
   <212> DNA
   <213> Artificial Sequence
<220>
   <223> siRNA, reverse complement antisense, 19 bp core sequence without 3' overhang
<400> 234
   tctgtctcca gtccatcga 19
<210> 235
   <211> 19
   <212> DNA
   <213> Artificial Sequence
<220>
   <223> siRNA, reverse complement antisense, 19 bp core sequence without 3' overhang
<400> 235
   tctctctgac cactctgcc 19
<210> 236
   <211> 19
   <212> DNA
   <213> Artificial Sequence
<220>
   <223> siRNA, reverse complement antisense, 19 bp core sequence without 3' overhang
<400> 236
   aagcgccaca tgtctgctg 19
<210> 237
   <211> 19
   <212> DNA
   <213> Artificial Sequence
<220>
   <223> siRNA, reverse complement antisense, 19 bp core sequence without 3' overhang
<400> 237
   tttgctccca ccagctcac 19
<210> 238
   <211> 19
   <212> DNA
   <213> Artificial Sequence
<220>
   <223> siRNA, reverse complement antisense, 19 bp core sequence without 3' overhang
<400> 238
   ttctgcagga agcgggctg 19
<210> 239
   <211> 19
   <212> DNA
   <213> Artificial Sequence
<220>
   <223> siRNA, reverse complement antisense, 19 bp core sequence without 3' overhang
<400> 239
   tcttctgctg atactcctc 19
<210> 240
   <211> 19
   <212> DNA
   <213> Artificial Sequence
<220>
   <223> siRNA, reverse complement antisense, 19 bp core sequence without 3' overhang
<400> 240
   ttggcctcgt tcagctttg 19
<210> 241
   <211> 19
   <212> DNA
   <213> Artificial Sequence
<220>
   <223> siRNA, reverse complement antisense, 19 bp core sequence without 3' overhang
<400> 241
   ttggcctgta gccgtgcaa 19
<210> 242
   <211> 19
   <212> DNA
   <213> Artificial Sequence
<220>
   <223> siRNA, reverse complement antisense, 19 bp core sequence without 3' overhang
<400> 242
   tttctcagga tctacagtg 19
<210> 243
   <211> 19
   <212> DNA
   <213> Artificial Sequence
<220>
   <223> siRNA, reverse complement antisense, 19 bp core sequence without 3' overhang
<400> 243
   tccttctcca cttcctcca 19
<210> 244
   <211> 19
   <212> DNA
   <213> Artificial Sequence
<220>
   <223> siRNA, reverse complement antisense, 19 bp core sequence without 3' overhang
<400> 244
   ttgtcctcct cctgcgtct 19
<210> 245
   <211> 19
   <212> DNA
   <213> Artificial Sequence
<220>
   <223> siRNA, reverse complement antisense, 19 bp core sequence without 3' overhang
<400> 245
   ttcccagctg ctccagtcg 19
<210> 246
   <211> 19
   <212> DNA
   <213> Artificial Sequence
<220>
   <223> v
<400> 246
   ttggccacct tcctctcgg 19
<210> 247
   <211> 19
   <212> DNA
   <213> Artificial Sequence
<220>
   <223> siRNA, reverse complement antisense, 19 bp core sequence without 3' overhang
<400> 247
   attatgtaca cgtgagatg 19
<210> 248
   <211> 19
   <212> DNA
   <213> Artificial Sequence
<220>
   <223> siRNA, reverse complement antisense, 19 bp core sequence without 3' overhang
<400> 248
   tattgtggct ctgattatg 19
<210> 249
   <211> 19
   <212> DNA
   <213> Artificial Sequence
<220>
   <223> siRNA, sense, 19 bp core sequence without 3' overhang
<400> 249
   ccgtgtccat cttcgtcta 19
<210> 250
   <211> 19
   <212> DNA
   <213> Artificial Sequence
<220>
   <223> siRNA, sense, 19 bp core sequence without 3' overhang
<400> 250
   cttcaaaact ctacggcac 19
<210> 251
   <211> 19
   <212> DNA
   <213> Artificial Sequence
<220>
   <223> siRNA, sense, 19 bp core sequence without 3' overhang
<400> 251
   tggcttacat cgatggact 19
<210> 252
   <211> 19
   <212> DNA
   <213> Artificial Sequence
<220>
   <223> siRNA, sense, 19 bp core sequence without 3' overhang
<400> 252
   ccctcagctt cctggtcaa 19
<210> 253
   <211> 19
   <212> DNA
   <213> Artificial Sequence
<220>
   <223> siRNA, sense, 19 bp core sequence without 3' overhang
<400> 253
   gtggcagagt ggtcagaga 19
<210> 254
   <211> 19
   <212> DNA
   <213> Artificial Sequence
<220>
   <223> siRNA, sense, 19 bp core sequence without 3' overhang
<400> 254
   tcaaagagcc agccgagaa 19
<210> 255
   <211> 19
   <212> DNA
   <213> Artificial Sequence
<220>
   <223> siRNA, sense, 19 bp core sequence without 3' overhang
<400> 255
   aggagtatca gcagaagat 19
<210> 256
   <211> 19
   <212> DNA
   <213> Artificial Sequence
<220>
   <223> siRNA, sense, 19 bp core sequence without 3' overhang
<400> 256
   ctgtggtggt caagatgtt 19
<210> 257
   <211> 19
   <212> DNA
   <213> Artificial Sequence
<220>
   <223> siRNA, sense, 19 bp core sequence without 3' overhang
<400> 257
   tcaatgtgga gctgatgaa 19
<210> 258
   <211> 19
   <212> DNA
   <213> Artificial Sequence
<220>
   <223> siRNA, sense, 19 bp core sequence without 3' overhang
<400> 258
   ctgagaaatc cgtgcgaga 19
<210> 259
   <211> 19
   <212> DNA
   <213> Artificial Sequence
<220>
   <223> siRNA, sense, 19 bp core sequence without 3' overhang
<400> 259
   caggaggagg acaaggaca 19
<210> 260
   <211> 19
   <212> DNA
   <213> Artificial Sequence
<220>
   <223> siRNA, sense, 19 bp core sequence without 3' overhang
<400> 260
   tgacagatgg gacgacgaa 19
<210> 261
   <211> 19
   <212> DNA
   <213> Artificial Sequence
<220>
   <223> siRNA, sense, 19 bp core sequence without 3' overhang
<400> 261
   ccaagtgagc cgtgctagt 19
<210> 262
   <211> 19
   <212> DNA
   <213> Artificial Sequence
<220>
   <223> siRNA, sense, 19 bp core sequence without 3' overhang
<400> 262
   ccaggccatc tcacgtgta 19
<210> 263
   <211> 19
   <212> DNA
   <213> Artificial Sequence
<220>
   <223> siRNA, sense, 19 bp core sequence without 3' overhang
<400> 263
   gtacataatc agagccaca 19
<210> 264
   <211> 19
   <212> DNA
   <213> Artificial Sequence
<220>
   <223> siRNA, reverse complement antisense, 19 bp core sequence without 3' overhang
<400> 264
   tagacgaaga tggacacgg 19
<210> 265
   <211> 19
   <212> DNA
   <213> Artificial Sequence
<220>
   <223> siRNA, reverse complement antisense, 19 bp core sequence without 3' overhang
<400> 265
   gtgccgtaga gttttgaag 19
<210> 266
   <211> 19
   <212> DNA
   <213> Artificial Sequence
<220>
   <223> siRNA, reverse complement antisense, 19 bp core sequence without 3' overhang
<400> 266
   agtccatcga tgtaagcca 19
<210> 267
   <211> 19
   <212> DNA
   <213> Artificial Sequence
<220>
   <223> siRNA, reverse complement antisense, 19 bp core sequence without 3' overhang
<400> 267
   ttgaccagga agctgaggg 19
<210> 268
   <211> 19
   <212> DNA
   <213> Artificial Sequence
<220>
   <223> siRNA, reverse complement antisense, 19 bp core sequence without 3' overhang
<400> 268
   tctctgacca ctctgccac 19
<210> 269
   <211> 19
   <212> DNA
   <213> Artificial Sequence
<220>
   <223> siRNA, reverse complement antisense, 19 bp core sequence without 3' overhang
<400> 269
   ttctcggctg gctctttga 19
<210> 270
   <211> 19
   <212> DNA
   <213> Artificial Sequence
<220>
   <223> siRNA, reverse complement antisense, 19 bp core sequence without 3' overhang
<400> 270
   atcttctgct gatactcct 19
<210> 271
   <211> 19
   <212> DNA
   <213> Artificial Sequence
<220>
   <223> siRNA, reverse complement antisense, 19 bp core sequence without 3' overhang
<400> 271
   aacatcttga ccaccacag 19
<210> 272
   <211> 19
   <212> DNA
   <213> Artificial Sequence
<220>
   <223> siRNA, reverse complement antisense, 19 bp core sequence without 3' overhang
<400> 272
   ttcatcagct ccacattga 19
<210> 273
   <211> 19
   <212> DNA
   <213> Artificial Sequence
<220>
   <223> siRNA, reverse complement antisense, 19 bp core sequence without 3' overhang
<400> 273
   tctcgcacgg atttctcag 19
<210> 274
   <211> 19
   <212> DNA
   <213> Artificial Sequence
<220>
   <223> siRNA, reverse complement antisense, 19 bp core sequence without 3' overhang
<400> 274
   tgtccttgtc ctcctcctg 19
<210> 275
   <211> 19
   <212> DNA
   <213> Artificial Sequence
<220>
   <223> siRNA, reverse complement antisense, 19 bp core sequence without 3' overhang
<400> 275
   ttcgtcgtcc catctgtca 19
<210> 276
   <211> 19
   <212> DNA
   <213> Artificial Sequence
<220>
   <223> siRNA, reverse complement antisense, 19 bp core sequence without 3' overhang
<400> 276
   actagcacgg ctcacttgg 19
<210> 277
   <211> 19
   <212> DNA
   <213> Artificial Sequence
<220>
   <223> siRNA, reverse complement antisense, 19 bp core sequence without 3' overhang
<400> 277
   tacacgtgag atggcctgg 19
<210> 278
   <211> 19
   <212> DNA
   <213> Artificial Sequence
<220>
   <223> siRNA, reverse complement antisense, 19 bp core sequence without 3' overhang
<400> 278
   tgtggctctg attatgtac 19
<210> 279
   <211> 19
   <212> DNA
   <213> Artificial Sequence
<220>
   <223> siRNA, sense, 19 bp core sequence without 3' overhang
<400> 279
   catcgatgga ctggagaca 19
<210> 280
   <211> 19
   <212> DNA
   <213> Artificial Sequence
<220>
   <223> siRNA, sense, 19 bp core sequence without 3' overhang
<400> 280
   tgaaggagct ggagatctc 19
<210> 281
   <211> 19
   <212> DNA
   <213> Artificial Sequence
<220>
   <223> siRNA, sense, 19 bp core sequence without 3' overhang
<400> 281
   gctacaccag atcgtgaaa 19
<210> 282
   <211> 19
   <212> DNA
   <213> Artificial Sequence
<220>
   <223> siRNA, sense, 19 bp core sequence without 3' overhang
<400> 282
   gcagcctcat ccacaacaa 19
<210> 283
   <211> 19
   <212> DNA
   <213> Artificial Sequence
<220>
   <223> siRNA, sense, 19 bp core sequence without 3' overhang
<400> 283
   ctggtggctt catgagcaa 19
<210> 284
   <211> 19
   <212> DNA
   <213> Artificial Sequence
<220>
   <223> siRNA, sense, 19 bp core sequence without 3' overhang
<400> 284
   acgcattccc tgaggattt 19
<210> 285
   <211> 19
   <212> DNA
   <213> Artificial Sequence
<220>
   <223> siRNA, sense, 19 bp core sequence without 3' overhang
<400> 285
   agtatcagca gaagatcat 19
<210> 286
   <211> 19
   <212> DNA
   <213> Artificial Sequence
<220>
   <223> siRNA, sense, 19 bp core sequence without 3' overhang
<400> 286
   ggctcctacc tcagtgcta 19
<210> 287
   <211> 19
   <212> DNA
   <213> Artificial Sequence
<220>
   <223> siRNA, sense, 19 bp core sequence without 3' overhang
<400> 287
   ctgtagatcc tgagaaatc 19
<210> 288
   <211> 19
   <212> DNA
   <213> Artificial Sequence
<220>
   <223> siRNA, sense, 19 bp core sequence without 3' overhang
<400> 288
   aggaagtgga gaaggatgt 19
<210> 289
   <211> 19
   <212> DNA
   <213> Artificial Sequence
<220>
   <223> siRNA, sense, 19 bp core sequence without 3' overhang
<400> 289
   ggacaaggac acagcagag 19
<210> 290
   <211> 19
   <212> DNA
   <213> Artificial Sequence
<220>
   <223> siRNA, sense, 19 bp core sequence without 3' overhang
<400> 290
   acagatggga cgacgaaga 19
<210> 291
   <211> 19
   <212> DNA
   <213> Artificial Sequence
<220>
   <223> siRNA, sense, 19 bp core sequence without 3' overhang
<400> 291
   gccccacaga tgtatttat 19
<210> 292
   <211> 19
   <212> DNA
   <213> Artificial Sequence
<220>
   <223> siRNA, sense, 19 bp core sequence without 3' overhang
<400> 292
   aggccatctc acgtgtaca 19
<210> 293
   <211> 19
   <212> DNA
   <213> Artificial Sequence
<220>
   <223> siRNA, sense, 19 bp core sequence without 3' overhang
<400> 293
   taatcagagc cacaataaa 19
<210> 294
   <211> 19
   <212> DNA
   <213> Artificial Sequence
<220>
   <223> siRNA, reverse complement antisense, 19 bp core sequence without 3' overhang
<400> 294
   tgtctccagt ccatcgatg 19
<210> 295
   <211> 19
   <212> DNA
   <213> Artificial Sequence
<220>
   <223> siRNA, reverse complement antisense, 19 bp core sequence without 3' overhang
<400> 295
   gagatctcca gctccttca 19
<210> 296
   <211> 19
   <212> DNA
   <213> Artificial Sequence
<220>
   <223> siRNA, reverse complement antisense, 19 bp core sequence without 3' overhang
<400> 296
   tttcacgatc tggtgtagc 19
<210> 297
   <211> 19
   <212> DNA
   <213> Artificial Sequence
<220>
   <223> siRNA, reverse complement antisense, 19 bp core sequence without 3' overhang
<400> 297
   ttgttgtgga tgaggctgc 19
<210> 298
   <211> 19
   <212> DNA
   <213> Artificial Sequence
<220>
   <223> siRNA, reverse complement antisense, 19 bp core sequence without 3' overhang
<400> 298
   ttgctcatga agccaccag 19
<210> 299
   <211> 19
   <212> DNA
   <213> Artificial Sequence
<220>
   <223> siRNA, reverse complement antisense, 19 bp core sequence without 3' overhang
<400> 299
   aaatcctcag ggaatgcgt 19
<210> 300
   <211> 19
   <212> DNA
   <213> Artificial Sequence
<220>
   <223> siRNA, reverse complement antisense, 19 bp core sequence without 3' overhang
<400> 300
   atgatcttct gctgatact 19
<210> 301
   <211> 19
   <212> DNA
   <213> Artificial Sequence
<220>
   <223> siRNA, reverse complement antisense, 19 bp core sequence without 3' overhang
<400> 301
   tagcactgag gtaggagcc 19
<210> 302
   <211> 19
   <212> DNA
   <213> Artificial Sequence
<220>
   <223> siRNA, reverse complement antisense, 19 bp core sequence without 3' overhang
<400> 302
   gatttctcag gatctacag 19
<210> 303
   <211> 19
   <212> DNA
   <213> Artificial Sequence
<220>
   <223> siRNA, reverse complement antisense, 19 bp core sequence without 3' overhang
<400> 303
   acatccttct ccacttcct 19
<210> 304
   <211> 19
   <212> DNA
   <213> Artificial Sequence
<220>
   <223> siRNA, reverse complement antisense, 19 bp core sequence without 3' overhang
<400> 304
   ctctgctgtg tccttgtcc 19
<210> 305
   <211> 19
   <212> DNA
   <213> Artificial Sequence
<220>
   <223> siRNA, reverse complement antisense, 19 bp core sequence without 3' overhang
<400> 305
   tcttcgtcgt cccatctgt 19
<210> 306
   <211> 19
   <212> DNA
   <213> Artificial Sequence
<220>
   <223> siRNA, reverse complement antisense, 19 bp core sequence without 3' overhang
<400> 306
   ataaatacat ctgtggggc 19
<210> 307
   <211> 19
   <212> DNA
   <213> Artificial Sequence
<220>
   <223> siRNA, reverse complement antisense, 19 bp core sequence without 3' overhang
<400> 307
   tgtacacgtg agatggcct 19
<210> 308
   <211> 19
   <212> DNA
   <213> Artificial Sequence
<220>
   <223> siRNA, reverse complement antisense, 19 bp core sequence without 3' overhang
<400> 308
   tttattgtgg ctctgatta 19
<210> 309
   <211> 6
   <212> DNA
   <213> Artificial Sequence
<220>
   <223> 5 base loop sequence
<400> 309
   agtttg 6
<210> 310
   <211> 19
   <212> DNA
   <213> Artificial Sequence
<220>
   <223> minimal T7 RNA polymerase promoter sequence
<400> 310
   taatacgact cactatagg 19
<210> 311
   <211> 6
   <212> DNA
   <213> Artificial Sequence
<220>
   <223> HindIII restriction site
<400> 311
   aagctt 6
<210> 312
   <211> 6
   <212> DNA
   <213> Artificial Sequence
<220>
   <223> EcoRI restriction site
<400> 312
   gaattc 6
<210> 313
   <211> 21
   <212> DNA
   <213> Artificial Sequence
<220>
   <223> siRNA, PolG OT, sense
<400> 313
   gggugaagcg cuggauauut t 21
<210> 314
   <211> 21
   <212> DNA
   <213> Artificial Sequence
<220>
   <223> siRNA, PolG OT, reverse complement antisense
<400> 314
   aauauccagc gcuucaccct t 21
<210> 315
   <211> 21
   <212> DNA
   <213> Artificial Sequence
<220>
   <223> siRNA, Scyl1 OT, sense
<400> 315
   gccucaucca caacaaugut t 21
<210> 316
   <211> 21
   <212> DNA
   <213> Artificial Sequence
<220>
   <223> siRNA, Scyl1 OT, reverse complement antisense
<400> 316
   acauuguugu ggaugaggct t 21
<210> 317
   <211> 21
   <212> DNA
   <213> Artificial Sequence
<220>
   <223> siRNA, negative control, sense
<400> 317
   uugucuugca uucgacuaat t 21
<210> 318
   <211> 21
   <212> DNA
   <213> Artificial Sequence
<220>
   <223> siRNA, negative control, reverse complement antisense
<400> 318
   uuagucgaau gcaagacaat t 21
<210> 319
   <211> 21
   <212> DNA
   <213> Artificial Sequence
<220>
   <223> siRNA, Mad2, sense
<400> 319
   ggaacaacug aaagauuggt t 21
<210> 320
   <211> 21
   <212> DNA
   <213> Artificial Sequence
<220>
   <223> siRNA, Mad2, reverse complement antisense
<400> 320
   ccaaucuuuc aguuguucct t 21
<210> 321
   <211> 19
   <212> DNA
   <213> Artificial Sequence
<220>
   <223> siRNA, first of the smart pools for PolG, sense
<400> 321
   gguaucggcu gucggauga 19
<210> 322
   <211> 19
   <212> DNA
   <213> Artificial Sequence
<220>
   <223> siRNA, first of the smart pools for PolG, reverse complement antisense
<400> 322
   ucauccgaca gccgauacc 19
<210> 323
   <211> 19
   <212> DNA
   <213> Artificial Sequence
<220>
   <223> siRNA, second of the smart pools for PolG, sense
<400> 323
   agugggaccu gcaagaauu 19
<210> 324
   <211> 19
   <212> DNA
   <213> Artificial Sequence
<220>
   <223> siRNA, second of the smart pools for PolG, reverse complement antisense
<400> 324
   aauucuugca ggucccacu 19
<210> 325
   <211> 19
   <212> DNA
   <213> Artificial Sequence
<220>
   <223> siRNA, third of the smart pools for PolG, sense
<400> 325
   ucacaaggau gguaauagc 19
<210> 326
   <211> 19
   <212> DNA
   <213> Artificial Sequence
<220>
   <223> siRNA, third of the smart pools for PolG, reverse complement antisense
<400> 326
   gcuauuacca uccuuguga 19
<210> 327
   <211> 19
   <212> DNA
   <213> Artificial Sequence
<220>
   <223> siRNA, fourth of the smart pools for PolG, sense
<400> 327
   gcuuacuaau gcaguuuaa 19
<210> 328
   <211> 19
   <212> DNA
   <213> Artificial Sequence
<220>
   <223> siRNA, fourth of the smart pools for PolG, reverse complement antisense
<400> 328
   uuaaacugca uuaguaagc 19
<210> 329
   <211> 255
   <212> DNA
   <213> Artificial Sequence
<220>
   <223> dsRNA of a fragment of human POLG
<400> 329
   ggaagaagtg ggaggtggtt gctgaacggg catggaaggg gggcacagag tcagaaatgt       60
   tcaataagct tgagagcatt gctacgtctg acataccacg taccccggtg ctgggctgct      120
   gcatcagccg agccctggag ccctcggctg tccaggaaga gtttatgacc agccgtgtga      180
   attgggtggt acagagctct gctgttgact acttacacct catgcttgtg gccatgaagt      240
   ggctgtttga agagt                                                        255
<210> 330
   <211> 426
   <212> DNA
   <213> Artificial Sequence
<220>
   <223> dsRNA of a fragment of human SCYL1
<400> 330
   cagccgagaa gcaaaaattc ttccaggagc tgagcaagag cctggacgca ttccctgagg       60
   atttctgtcg gcacaaggtg ctgccccagc tgctgaccgc cttcgagttc ggcaatgctg      120
   gggccgttgt cctcacgccc ctcttcaagg tgggcaagtt cctgagcgct gaggagtatc      180
   agcagaagat catccctgtg gtggtcaaga tgttctcatc cactgaccgg gccatgcgca      240
   tccgcctcct gcagcagatg gagcagttca tccagtacct tgacgagcca acagtcaaca      300
   cccagatctt cccccacgtc gtacatggct tcctggacac caaccctgcc atccgggagc      360
   agacggtcaa gtccatgctg ctcctggccc caaagctgaa cgaggccaac ctcaatgtgg      420
   agctga                                                                  426
<210> 331
   <211> 20
   <212> DNA
   <213> Artificial Sequence
<220>
   <223> primer, POLG forward
<400> 331
   ttccaggacc tgatgcagta 20
<210> 332
   <211> 20
   <212> DNA
   <213> Artificial Sequence
<220>
   <223> primer, POLG reverse
<400> 332
   acaggcaggt aggagacacc 20
<210> 333
   <211> 20
   <212> DNA
   <213> Artificial Sequence
<220>
   <223> primer, SCYL1 forward
<400> 333
   ctggaggaag tggagaagga 20
<210> 334
   <211> 20
   <212> DNA
   <213> Artificial Sequence
<220>
   <223> primer, SCYL1 reverse
<400> 334
   tcagcttgga ggtgagtgag 20
<210> 335
   <211> 20
   <212> DNA
   <213> Artificial Sequence
<220>
   <223> primer, GAPDH forward
<400> 335
   atgggtgtga accatgagaa 20
<210> 336
   <211> 20
   <212> DNA
   <213> Artificial Sequence
<220>
   <223> primer, GAPDH reverse
<400> 336
   gtgctaagca gttggtggtg 20
<210> 337
   <211> 692
   <212> DNA
   <213> Homo sapiens
<400> 337
   ggatgacatg aggaaaataa tgtaattgta attttgaaat gtggttttcc tgaaatcaag 60
   tcatctatag ttgatatgtt ttatttcatt ggttaatttt tacatggaga aaaccaaaat 120
   gatacttact gaactgtgtg taattgttcc ttttattttt ttggtaccta tttgacttac 180
   catggagtta acatcatgaa tttattgcac attgttcaaa aggaaccagg aggttttttt 240
   gtcaacattg tgatgtatat tcctttgaag atagtaactg tagatggaaa aacttgtgct 300
   ataaagctag atgctttcct aaatcagatg ttttggtcaa gtagtttgac tcagtatagg 360
   tagggagata tttaagtata aaatacaaca aaggaagtct aaatattcag aatctttgtt 420
   aaggtcctga aagtaactca taatctataa acaatgaaat attgctgtat agctcctttt 480
   gaccttcatt tcatgtatag ttttccctat tgaatcagtt tccaattatt tgactttaat 540
   ttatgtaact tgaacctatg aagcaatgga tatttgtact gtttaatgtt ctgtgataca 600
   gaactcttaa aaatgttttt tcatgtgttt tataaaatca agttttaagt gaaagtgagg 660
   aaataaagtt aagtttgttt taaatttgtc tt 692
<210> 338
   <211> 20
   <212> DNA
   <213> Artificial Sequence
<220>
   <223> primer, Mad2 forward
<400> 338
   agatgacagt gcacccagag 20
<210> 339
   <211> 20
   <212> DNA
   <213> Artificial Sequence
<220>
   <223> primer, Mad2 reverse
<400> 339
   tccaacagtg gcagaaatgt 20
<210> 340
   <211> 21
   <212> DNA
   <213> Artificial Sequence
<220>
   <223> siRNA, first of the smart pools for Scyl1, sense
<400> 340
   uuucucagga ucuacaguga g 21
<210> 341
   <211> 21
   <212> DNA
   <213> Artificial Sequence
<220>
   <223> siRNA, second of the smart pools for Scyl1, sense
<400> 341
   uugagguaua uucccaacgg g 21
<210> 342
   <211> 21
   <212> DNA
   <213> Artificial Sequence
<220>
   <223> siRNA, third of the smart pools for Scyl1, sense
<400> 342
   uugguuucua caaagcgguu g 21
<210> 343
   <211> 21
   <212> DNA
   <213> Artificial Sequence
<220>
   <223> siRNA, fourth of the smart pools for Scyl1, sense
<400> 343
   uuguacaaua aauacaucug u 21
<210> 344
   <211> 20
   <212> DNA
   <213> Artificial Sequence
<220>
   <223> primer, PolG forward
<400> 344
   ttccaggacc tgatgcagta 20
<210> 345
   <211> 20
   <212> DNA
   <213> Artificial Sequence
<220>
   <223> primer, PolG reverse
<400> 345
   acaggcaggt aggagacacc 20
<210> 346
   <211> 30
   <212> DNA
   <213> Artificial Sequence
<220>
   <223> primer, Mad2 forward
<400> 346
   gatcgagctc ggatgacatg aggaaaataa 30
<210> 347
   <211> 35
   <212> DNA
   <213> Artificial Sequence
<220>
   <223> primer, Mad2 reverse
<400> 347
   gatcgtttaa acaagacaaa tttaaaacaa actta 35
<210> 348
   <211> 19
   <212> DNA
   <213> Artificial Sequence
<220>
   <223> PolG Pool #1 siRNA off-T guide
<400> 348
   gggtgaagcg ctggatatt 19
<210> 349
   <211> 19
   <212> DNA
   <213> Artificial Sequence
<220>
   <223> PolG Pool #1 siRNA off-T passenger
<400> 349
   aatatccagc gcttcaccc 19
<210> 350
   <211> 19
   <212> DNA
   <213> Artificial Sequence
<220>
   <223> Scyl1 Pool #1 siRNA off-T guide
<400> 350
   gcctcatcca caacaatgt 19
<210> 351
   <211> 19
   <212> DNA
   <213> Artificial Sequence
<220>
   <223> Scyl1 Pool #1 siRNA off-T passenger
<400> 351
   acattgttgt ggatgaggc 19
<210> 352
   <211> 19
   <212> DNA
   <213> Artificial Sequence
<220>
   <223> siRNA, sense, 19 bp core sequence without 3' overhang
<400> 352
   gcagggattt agtgcaaga 19
<210> 353
   <211> 19
   <212> DNA
   <213> Artificial Sequence
<220>
   <223> siRNA, sense, 19 bp core sequence without 3' overhang
<400> 353
   gcctcggtat cctcgtgaa 19
<210> 354
   <211> 19
   <212> DNA
   <213> Artificial Sequence
<220>
   <223> siRNA, sense, 19 bp core sequence without 3' overhang
<400> 354
   gcagtgcttt aacaaatca 19
<210> 355
   <211> 19
   <212> DNA
   <213> Artificial Sequence
<220>
   <223> siRNA, sense, 19 bp core sequence without 3' overhang
<400> 355
   ggacctgtgt cttctacaa 19
<210> 356
   <211> 19
   <212> DNA
   <213> Artificial Sequence
<220>
   <223> siRNA, sense, 19 bp core sequence without 3' overhang
<400> 356
   gagttggctt cagaatgta 19
<210> 357
   <211> 19
   <212> DNA
   <213> Artificial Sequence
<220>
   <223> siRNA, sense, 19 bp core sequence without 3' overhang
<400> 357
   gcactggact tggttccca 19
<210> 358
   <211> 19
   <212> DNA
   <213> Artificial Sequence
<220>
   <223> siRNA, sense, 19 bp core sequence without 3' overhang
<400> 358
   ggatgctcct gaaagcaaa 19
<210> 359
   <211> 19
   <212> DNA
   <213> Artificial Sequence
<220>
   <223> siRNA, sense, 19 bp core sequence without 3' overhang
<400> 359
   ggccagtatt agagaacaa 19
<210> 360
   <211> 19
   <212> DNA
   <213> Artificial Sequence
<220>
   <223> siRNA, sense, 19 bp core sequence without 3' overhang
<400> 360
   ggaaacttgt gaatctgaa 19
<210> 361
   <211> 19
   <212> DNA
   <213> Artificial Sequence
<220>
   <223> siRNA, sense, 19 bp core sequence without 3' overhang
<400> 361
   ggaggctctt atggtacta 19
<210> 362
   <211> 19
   <212> DNA
   <213> Artificial Sequence
<220>
   <223> siRNA, sense, 19 bp core sequence without 3' overhang
<400> 362
   gacaaatgtt caggcccta 19
<210> 363
   <211> 19
   <212> DNA
   <213> Artificial Sequence
<220>
   <223> siRNA, sense, 19 bp core sequence without 3' overhang
<400> 363
   ggcactaact ttcaagtta 19
<210> 364
   <211> 19
   <212> DNA
   <213> Artificial Sequence
<220>
   <223> siRNA, sense, 19 bp core sequence without 3' overhang
<400> 364
   gcagcaaact cccagagta 19
<210> 365
   <211> 19
   <212> DNA
   <213> Artificial Sequence
<220>
   <223> siRNA, sense, 19 bp core sequence without 3' overhang
<400> 365
   ggcgcaaatt ctggaggaa 19
<210> 366
   <211> 19
   <212> DNA
   <213> Artificial Sequence
<220>
   <223> siRNA, sense, 19 bp core sequence without 3' overhang
<400> 366
   ggaacaaact gcctagcaa 19
<210> 367
   <211> 19
   <212> DNA
   <213> Artificial Sequence
<220>
   <223> siRNA, sense, 19 bp core sequence without 3' overhang
<400> 367
   ggatcagggt tctgccaca 19
<210> 368
   <211> 19
   <212> DNA
   <213> Artificial Sequence
<220>
   <223> siRNA, sense, 19 bp core sequence without 3' overhang
<400> 368
   ggagagcgat ggtagtaca 19
<210> 369
   <211> 19
   <212> DNA
   <213> Artificial Sequence
<220>
   <223> siRNA, sense, 19 bp core sequence without 3' overhang
<400> 369
   gaagatgatt ctgctgcta 19
<210> 370
   <211> 19
   <212> DNA
   <213> Artificial Sequence
<220>
   <223> siRNA, sense, 19 bp core sequence without 3' overhang
<400> 370
   ggagaaactt caaggaata 19
<210> 371
   <211> 19
   <212> DNA
   <213> Artificial Sequence
<220>
   <223> siRNA, sense, 19 bp core sequence without 3' overhang
<400> 371
   cgtttccggt tggaacgaa 19
<210> 372
   <211> 19
   <212> DNA
   <213> Artificial Sequence
<220>
   <223> siRNA, sense, 19 bp core sequence without 3' overhang
<400> 372
   ggataatggt acttcagca 19
<210> 373
   <211> 19
   <212> DNA
   <213> Artificial Sequence
<220>
   <223> siRNA, sense, 19 bp core sequence without 3' overhang
<400> 373
   ggaacccatt gctgcggca 19
<210> 374
   <211> 19
   <212> DNA
   <213> Artificial Sequence
<220>
   <223> siRNA, sense, 19 bp core sequence without 3' overhang
<400> 374
   gatatgccat tgcctggaa 19
<210> 375
   <211> 19
   <212> DNA
   <213> Artificial Sequence
<220>
   <223> siRNA, sense, 19 bp core sequence without 3' overhang
<400> 375
   gccaccatat acaaagaaa 19
<210> 376
   <211> 19
   <212> DNA
   <213> Artificial Sequence
<220>
   <223> siRNA, sense, 19 bp core sequence without 3' overhang
<400> 376
   cgaagggtct gagtggcaa 19
<210> 377
   <211> 19
   <212> DNA
   <213> Artificial Sequence
<220>
   <223> siRNA, sense, 19 bp core sequence without 3' overhang
<400> 377
   gatgaaaggt ggaaacaaa 19
<210> 378
   <211> 19
   <212> DNA
   <213> Artificial Sequence
<220>
   <223> siRNA, sense, 19 bp core sequence without 3' overhang
<400> 378
   ggaggaatgt tacaagaca 19
<210> 379
   <211> 19
   <212> DNA
   <213> Artificial Sequence
<220>
   <223> siRNA, sense, 19 bp core sequence without 3' overhang
<400> 379
   ggcctcagat ttccaaaga 19
<210> 380
   <211> 19
   <212> DNA
   <213> Artificial Sequence
<220>
   <223> siRNA, sense, 19 bp core sequence without 3' overhang
<400> 380
   gcagcagcct ccagcacaa 19
<210> 381
   <211> 19
   <212> DNA
   <213> Artificial Sequence
<220>
   <223> siRNA, sense, 19 bp core sequence without 3' overhang
<400> 381
   ggcttgaact caaacttga 19
<210> 382
   <211> 19
   <212> DNA
   <213> Artificial Sequence
<220>
   <223> siRNA, reverse complement antisense, 19 bp core sequence without 3' overhang
<400> 382
   tcttgcacta aatccctgc 19
<210> 383
   <211> 19
   <212> DNA
   <213> Artificial Sequence
<220>
   <223> siRNA, reverse complement antisense, 19 bp core sequence without 3' overhang
<400> 383
   ttcacgagga taccgaggc 19
<210> 384
   <211> 19
   <212> DNA
   <213> Artificial Sequence
<220>
   <223> siRNA, reverse complement antisense, 19 bp core sequence without 3' overhang
<400> 384
   tgatttgtta aagcactgc 19
<210> 385
   <211> 19
   <212> DNA
   <213> Artificial Sequence
<220>
   <223> siRNA, reverse complement antisense, 19 bp core sequence without 3' overhang
<400> 385
   ttgtagaaga cacaggtcc 19
<210> 386
   <211> 19
   <212> DNA
   <213> Artificial Sequence
<220>
   <223> siRNA, reverse complement antisense, 19 bp core sequence without 3' overhang
<400> 386
   tacattctga agccaactc 19
<210> 387
   <211> 19
   <212> DNA
   <213> Artificial Sequence
<220>
   <223> siRNA, reverse complement antisense, 19 bp core sequence without 3' overhang
<400> 387
   tgggaaccaa gtccagtgc 19
<210> 388
   <211> 19
   <212> DNA
   <213> Artificial Sequence
<220>
   <223> siRNA, reverse complement antisense, 19 bp core sequence without 3' overhang
<400> 388
   tttgctttca ggagcatcc 19
<210> 389
   <211> 19
   <212> DNA
   <213> Artificial Sequence
<220>
   <223> siRNA, reverse complement antisense, 19 bp core sequence without 3' overhang
<400> 389
   ttgttctcta atactggcc 19
<210> 390
   <211> 19
   <212> DNA
   <213> Artificial Sequence
<220>
   <223> siRNA, reverse complement antisense, 19 bp core sequence without 3' overhang
<400> 390
   ttcagattca caagtttcc 19
<210> 391
   <211> 19
   <212> DNA
   <213> Artificial Sequence
<220>
   <223> siRNA, reverse complement antisense, 19 bp core sequence without 3' overhang
<400> 391
   tagtaccata agagcctcc 19
<210> 392
   <211> 19
   <212> DNA
   <213> Artificial Sequence
<220>
   <223> siRNA, reverse complement antisense, 19 bp core sequence without 3' overhang
<400> 392
   tagggcctga acatttgtc 19
<210> 393
   <211> 19
   <212> DNA
   <213> Artificial Sequence
<220>
   <223> siRNA, reverse complement antisense, 19 bp core sequence without 3' overhang
<400> 393
   taacttgaaa gttagtgcc 19
<210> 394
   <211> 19
   <212> DNA
   <213> Artificial Sequence
<220>
   <223> siRNA, reverse complement antisense, 19 bp core sequence without 3' overhang
<400> 394
   tactctggga gtttgctgc 19
<210> 395
   <211> 19
   <212> DNA
   <213> Artificial Sequence
<220>
   <223> siRNA, reverse complement antisense, 19 bp core sequence without 3' overhang
<400> 395
   ttcctccaga atttgcgcc 19
<210> 396
   <211> 19
   <212> DNA
   <213> Artificial Sequence
<220>
   <223> siRNA, reverse complement antisense, 19 bp core sequence without 3' overhang
<400> 396
   ttgctaggca gtttgttcc 19
<210> 397
   <211> 19
   <212> DNA
   <213> Artificial Sequence
<220>
   <223> siRNA, reverse complement antisense, 19 bp core sequence without 3' overhang
<400> 397
   tgtggcagaa ccctgatcc 19
<210> 398
   <211> 19
   <212> DNA
   <213> Artificial Sequence
<220>
   <223> siRNA, reverse complement antisense, 19 bp core sequence without 3' overhang
<400> 398
   tgtactacca tcgctctcc 19
<210> 399
   <211> 19
   <212> DNA
   <213> Artificial Sequence
<220>
   <223> siRNA, reverse complement antisense, 19 bp core sequence without 3' overhang
<400> 399
   tagcagcaga atcatcttc 19
<210> 400
   <211> 19
   <212> DNA
   <213> Artificial Sequence
<220>
   <223> siRNA, reverse complement antisense, 19 bp core sequence without 3' overhang
<400> 400
   tattccttga agtttctcc 19
<210> 401
   <211> 19
   <212> DNA
   <213> Artificial Sequence
<220>
   <223> siRNA, reverse complement antisense, 19 bp core sequence without 3' overhang
<400> 401
   ttcgttccaa ccggaaacg 19
<210> 402
   <211> 19
   <212> DNA
   <213> Artificial Sequence
<220>
   <223> siRNA, reverse complement antisense, 19 bp core sequence without 3' overhang
<400> 402
   tgctgaagta ccattatcc 19
<210> 403
   <211> 19
   <212> DNA
   <213> Artificial Sequence
<220>
   <223> siRNA, reverse complement antisense, 19 bp core sequence without 3' overhang
<400> 403
   tgccgcagca atgggttcc 19
<210> 404
   <211> 19
   <212> DNA
   <213> Artificial Sequence
<220>
   <223> siRNA, reverse complement antisense, 19 bp core sequence without 3' overhang
<400> 404
   ttccaggcaa tggcatatc 19
<210> 405
   <211> 19
   <212> DNA
   <213> Artificial Sequence
<220>
   <223> siRNA, reverse complement antisense, 19 bp core sequence without 3' overhang
<400> 405
   tttctttgta tatggtggc 19
<210> 406
   <211> 19
   <212> DNA
   <213> Artificial Sequence
<220>
   <223> siRNA, reverse complement antisense, 19 bp core sequence without 3' overhang
<400> 406
   ttgccactca gacccttcg 19
<210> 407
   <211> 19
   <212> DNA
   <213> Artificial Sequence
<220>
   <223> siRNA, reverse complement antisense, 19 bp core sequence without 3' overhang
<400> 407
   tttgtttcca cctttcatc 19
<210> 408
   <211> 19
   <212> DNA
   <213> Artificial Sequence
<220>
   <223> siRNA, reverse complement antisense, 19 bp core sequence without 3' overhang
<400> 408
   tgtcttgtaa cattcctcc 19
<210> 409
   <211> 19
   <212> DNA
   <213> Artificial Sequence
<220>
   <223> siRNA, reverse complement antisense, 19 bp core sequence without 3' overhang
<400> 409
   tctttggaaa tctgaggcc 19
<210> 410
   <211> 19
   <212> DNA
   <213> Artificial Sequence
<220>
   <223> siRNA, reverse complement antisense, 19 bp core sequence without 3' overhang
<400> 410
   ttgtgctgga ggctgctgc 19
<210> 411
   <211> 19
   <212> DNA
   <213> Artificial Sequence
<220>
   <223> siRNA, reverse complement antisense, 19 bp core sequence without 3' overhang
<400> 411
   tcaagtttga gttcaagcc 19
<210> 412
   <211> 19
   <212> DNA
   <213> Artificial Sequence
<220>
   <223> siRNA, sense, 19 bp core sequence without 3' overhang
<400> 412
   gcatcagatt ccaaatcta 19
<210> 413
   <211> 19
   <212> DNA
   <213> Artificial Sequence
<220>
   <223> siRNA, sense, 19 bp core sequence without 3' overhang
<400> 413
   ggaggagtct ggaacacca 19
<210> 414
   <211> 19
   <212> DNA
   <213> Artificial Sequence
<220>
   <223> siRNA, sense, 19 bp core sequence without 3' overhang
<400> 414
   ggcagtgctt cctcccaca 19
<210> 415
   <211> 19
   <212> DNA
   <213> Artificial Sequence
<220>
   <223> siRNA, sense, 19 bp core sequence without 3' overhang
<400> 415
   ggatgaatcc tcttgccaa 19
<210> 416
   <211> 19
   <212> DNA
   <213> Artificial Sequence
<220>
   <223> siRNA, sense, 19 bp core sequence without 3' overhang
<400> 416
   ccgtccacct aattccaaa 19
<210> 417
   <211> 19
   <212> DNA
   <213> Artificial Sequence
<220>
   <223> siRNA, sense, 19 bp core sequence without 3' overhang
<400> 417
   ccagttatct cctcaacaa 19
<210> 418
   <211> 19
   <212> DNA
   <213> Artificial Sequence
<220>
   <223> siRNA, sense, 19 bp core sequence without 3' overhang
<400> 418
   cccagacctt caaaccaaa 19
<210> 419
   <211> 19
   <212> DNA
   <213> Artificial Sequence
<220>
   <223> siRNA, sense, 19 bp core sequence without 3' overhang
<400> 419
   ggatatggtt ctggcttca 19
<210> 420
   <211> 19
   <212> DNA
   <213> Artificial Sequence
<220>
   <223> siRNA, sense, 19 bp core sequence without 3' overhang
<400> 420
   ggaaccgagt ctcgcttta 19
<210> 421
   <211> 19
   <212> DNA
   <213> Artificial Sequence
<220>
   <223> siRNA, sense, 19 bp core sequence without 3' overhang
<400> 421
   gctgccctct gtagccaca 19
<210> 422
   <211> 19
   <212> DNA
   <213> Artificial Sequence
<220>
   <223> siRNA, sense, 19 bp core sequence without 3' overhang
<400> 422
   ggaagccaat atgcacaaa 19
<210> 423
   <211> 19
   <212> DNA
   <213> Artificial Sequence
<220>
   <223> siRNA, sense, 19 bp core sequence without 3' overhang
<400> 423
   gatagctggt tacctgcca 19
<210> 424
   <211> 19
   <212> DNA
   <213> Artificial Sequence
<220>
   <223> siRNA, sense, 19 bp core sequence without 3' overhang
<400> 424
   cctgccaaat ctccaccaa 19
<210> 425
   <211> 19
   <212> DNA
   <213> Artificial Sequence
<220>
   <223> siRNA, sense, 19 bp core sequence without 3' overhang
<400> 425
   ggagtgccat ggaaaggta 19
<210> 426
   <211> 19
   <212> DNA
   <213> Artificial Sequence
<220>
   <223> siRNA, sense, 19 bp core sequence without 3' overhang
<400> 426
   gctgcgggat aacaccaca 19
<210> 427
   <211> 19
   <212> DNA
   <213> Artificial Sequence
<220>
   <223> siRNA, sense, 19 bp core sequence without 3' overhang
<400> 427
   gggtctaatt cttccctca 19
<210> 428
   <211> 19
   <212> DNA
   <213> Artificial Sequence
<220>
   <223> siRNA, sense, 19 bp core sequence without 3' overhang
<400> 428
   cagcaaagtt ccctgatta 19
<210> 429
   <211> 19
   <212> DNA
   <213> Artificial Sequence
<220>
   <223> siRNA, sense, 19 bp core sequence without 3' overhang
<400> 429
   cagatcccat aggacacaa 19
<210> 430
   <211> 19
   <212> DNA
   <213> Artificial Sequence
<220>
   <223> siRNA, sense, 19 bp core sequence without 3' overhang
<400> 430
   ccactcatct ctccaacaa 19
<210> 431
   <211> 19
   <212> DNA
   <213> Artificial Sequence
<220>
   <223> siRNA, sense, 19 bp core sequence without 3' overhang
<400> 431
   gggtcaacct tgagaacga 19
<210> 432
   <211> 19
   <212> DNA
   <213> Artificial Sequence
<220>
   <223> siRNA, sense, 19 bp core sequence without 3' overhang
<400> 432
   gcccactgct gacattcca 19
<210> 433
   <211> 19
   <212> DNA
   <213> Artificial Sequence
<220>
   <223> siRNA, sense, 19 bp core sequence without 3' overhang
<400> 433
   gacattccat ctgaatcta 19
<210> 434
   <211> 19
   <212> DNA
   <213> Artificial Sequence
<220>
   <223> siRNA, sense, 19 bp core sequence without 3' overhang
<400> 434
   gcactgccct gatccgata 19
<210> 435
   <211> 19
   <212> DNA
   <213> Artificial Sequence
<220>
   <223> siRNA, sense, 19 bp core sequence without 3' overhang
<400> 435
   gcacatgtgt gtgttggga 19
<210> 436
   <211> 19
   <212> DNA
   <213> Artificial Sequence
<220>
   <223> siRNA, sense, 19 bp core sequence without 3' overhang
<400> 436
   gtttgccact gatgatgaa 19
<210> 437
   <211> 19
   <212> DNA
   <213> Artificial Sequence
<220>
   <223> siRNA, sense, 19 bp core sequence without 3' overhang
<400> 437
   cagccgcttt ctggcacaa 19
<210> 438
   <211> 19
   <212> DNA
   <213> Artificial Sequence
<220>
   <223> siRNA, sense, 19 bp core sequence without 3' overhang
<400> 438
   ccagtcagat cccgtggga 19
<210> 439
   <211> 19
   <212> DNA
   <213> Artificial Sequence
<220>
   <223> siRNA, sense, 19 bp core sequence without 3' overhang
<400> 439
   cgatcttgct ggcgcttca 19
<210> 440
   <211> 19
   <212> DNA
   <213> Artificial Sequence
<220>
   <223> siRNA, sense, 19 bp core sequence without 3' overhang
<400> 440
   gggcagccct gctccttta 19
<210> 441
   <211> 19
   <212> DNA
   <213> Artificial Sequence
<220>
   <223> siRNA, sense, 19 bp core sequence without 3' overhang
<400> 441
   ggaggagggt cggattcaa 19
<210> 442
   <211> 19
   <212> DNA
   <213> Artificial Sequence
<220>
   <223> siRNA, reverse complement antisense, 19 bp core sequence without 3' overhang
<400> 442
   tagatttgga atctgatgc 19
<210> 443
   <211> 19
   <212> DNA
   <213> Artificial Sequence
<220>
   <223> siRNA, reverse complement antisense, 19 bp core sequence without 3' overhang
<400> 443
   tggtgttcca gactcctcc 19
<210> 444
   <211> 19
   <212> DNA
   <213> Artificial Sequence
<220>
   <223> siRNA, reverse complement antisense, 19 bp core sequence without 3' overhang
<400> 444
   tgtgggagga agcactgcc 19
<210> 445
   <211> 19
   <212> DNA
   <213> Artificial Sequence
<220>
   <223> siRNA, reverse complement antisense, 19 bp core sequence without 3' overhang
<400> 445
   ttggcaagag gattcatcc 19
<210> 446
   <211> 19
   <212> DNA
   <213> Artificial Sequence
<220>
   <223> siRNA, reverse complement antisense, 19 bp core sequence without 3' overhang
<400> 446
   tttggaatta ggtggacgg 19
<210> 447
   <211> 19
   <212> DNA
   <213> Artificial Sequence
<220>
   <223> siRNA, reverse complement antisense, 19 bp core sequence without 3' overhang
<400> 447
   ttgttgagga gataactgg 19
<210> 448
   <211> 19
   <212> DNA
   <213> Artificial Sequence
<220>
   <223> siRNA, reverse complement antisense, 19 bp core sequence without 3' overhang
<400> 448
   tttggtttga aggtctggg 19
<210> 449
   <211> 19
   <212> DNA
   <213> Artificial Sequence
<220>
   <223> siRNA, reverse complement antisense, 19 bp core sequence without 3' overhang
<400> 449
   tgaagccaga accatatcc 19
<210> 450
   <211> 19
   <212> DNA
   <213> Artificial Sequence
<220>
   <223> siRNA, reverse complement antisense, 19 bp core sequence without 3' overhang
<400> 450
   taaagcgaga ctcggttcc 19
<210> 451
   <211> 19
   <212> DNA
   <213> Artificial Sequence
<220>
   <223> siRNA, reverse complement antisense, 19 bp core sequence without 3' overhang
<400> 451
   tgtggctaca gagggcagc 19
<210> 452
   <211> 19
   <212> DNA
   <213> Artificial Sequence
<220>
   <223> siRNA, reverse complement antisense, 19 bp core sequence without 3' overhang
<400> 452
   tttgtgcata ttggcttcc 19
<210> 453
   <211> 19
   <212> DNA
   <213> Artificial Sequence
<220>
   <223> siRNA, reverse complement antisense, 19 bp core sequence without 3' overhang
<400> 453
   tggcaggtaa ccagctatc 19
<210> 454
   <211> 19
   <212> DNA
   <213> Artificial Sequence
<220>
   <223> siRNA, reverse complement antisense, 19 bp core sequence without 3' overhang
<400> 454
   ttggtggaga tttggcagg 19
<210> 455
   <211> 19
   <212> DNA
   <213> Artificial Sequence
<220>
   <223> siRNA, reverse complement antisense, 19 bp core sequence without 3' overhang
<400> 455
   tacctttcca tggcactcc 19
<210> 456
   <211> 19
   <212> DNA
   <213> Artificial Sequence
<220>
   <223> siRNA, reverse complement antisense, 19 bp core sequence without 3' overhang
<400> 456
   tgtggtgtta tcccgcagc 19
<210> 457
   <211> 19
   <212> DNA
   <213> Artificial Sequence
<220>
   <223> siRNA, reverse complement antisense, 19 bp core sequence without 3' overhang
<400> 457
   tgagggaaga attagaccc 19
<210> 458
   <211> 19
   <212> DNA
   <213> Artificial Sequence
<220>
   <223> siRNA, reverse complement antisense, 19 bp core sequence without 3' overhang
<400> 458
   taatcaggga actttgctg 19
<210> 459
   <211> 19
   <212> DNA
   <213> Artificial Sequence
<220>
   <223> siRNA, reverse complement antisense, 19 bp core sequence without 3' overhang
<400> 459
   ttgtgtccta tgggatctg 19
<210> 460
   <211> 19
   <212> DNA
   <213> Artificial Sequence
<220>
   <223> siRNA, reverse complement antisense, 19 bp core sequence without 3' overhang
<400> 460
   ttgttggaga gatgagtgg 19
<210> 461
   <211> 19
   <212> DNA
   <213> Artificial Sequence
<220>
   <223> siRNA, reverse complement antisense, 19 bp core sequence without 3' overhang
<400> 461
   tcgttctcaa ggttgaccc 19
<210> 462
   <211> 19
   <212> DNA
   <213> Artificial Sequence
<220>
   <223> siRNA, reverse complement antisense, 19 bp core sequence without 3' overhang
<400> 462
   tggaatgtca gcagtgggc 19
<210> 463
   <211> 19
   <212> DNA
   <213> Artificial Sequence
<220>
   <223> siRNA, reverse complement antisense, 19 bp core sequence without 3' overhang
<400> 463
   tagattcaga tggaatgtc 19
<210> 464
   <211> 19
   <212> DNA
   <213> Artificial Sequence
<220>
   <223> siRNA, reverse complement antisense, 19 bp core sequence without 3' overhang
<400> 464
   tatcggatca gggcagtgc 19
<210> 465
   <211> 19
   <212> DNA
   <213> Artificial Sequence
<220>
   <223> siRNA, reverse complement antisense, 19 bp core sequence without 3' overhang
<400> 465
   tcccaacaca cacatgtgc 19
<210> 466
   <211> 19
   <212> DNA
   <213> Artificial Sequence
<220>
   <223> siRNA, reverse complement antisense, 19 bp core sequence without 3' overhang
<400> 466
   ttcatcatca gtggcaaac 19
<210> 467
   <211> 19
   <212> DNA
   <213> Artificial Sequence
<220>
   <223> siRNA, reverse complement antisense, 19 bp core sequence without 3' overhang
<400> 467
   ttgtgccaga aagcggctg 19
<210> 468
   <211> 19
   <212> DNA
   <213> Artificial Sequence
<220>
   <223> siRNA, reverse complement antisense, 19 bp core sequence without 3' overhang
<400> 468
   tcccacggga tctgactgg 19
<210> 469
   <211> 19
   <212> DNA
   <213> Artificial Sequence
<220>
   <223> siRNA, reverse complement antisense, 19 bp core sequence without 3' overhang
<400> 469
   tgaagcgcca gcaagatcg 19
<210> 470
   <211> 19
   <212> DNA
   <213> Artificial Sequence
<220>
   <223> siRNA, reverse complement antisense, 19 bp core sequence without 3' overhang
<400> 470
   taaaggagca gggctgccc 19
<210> 471
   <211> 19
   <212> DNA
   <213> Artificial Sequence
<220>
   <223> siRNA, reverse complement antisense, 19 bp core sequence without 3' overhang
<400> 471
   ttgaatccga ccctcctcc 19
<210> 472
   <211> 19
   <212> DNA
   <213> Artificial Sequence
<220>
   <223> siRNA, sense, 19 bp core sequence without 3' overhang
<400> 472
   ggcctgtact tggacatga 19
<210> 473
   <211> 19
   <212> DNA
   <213> Artificial Sequence
<220>
   <223> siRNA, sense, 19 bp core sequence without 3' overhang
<400> 473
   gaaacttgct gccacaaga 19
<210> 474
   <211> 19
   <212> DNA
   <213> Artificial Sequence
<220>
   <223> siRNA, sense, 19 bp core sequence without 3' overhang
<400> 474
   gaatgtgtct ttcagcgca 19
<210> 475
   <211> 19
   <212> DNA
   <213> Artificial Sequence
<220>
   <223> siRNA, sense, 19 bp core sequence without 3' overhang
<400> 475
   gcagacaaat ggactgcca 19
<210> 476
   <211> 19
   <212> DNA
   <213> Artificial Sequence
<220>
   <223> siRNA, sense, 19 bp core sequence without 3' overhang
<400> 476
   gggcagtgct gaaggaata 19
<210> 477
   <211> 19
   <212> DNA
   <213> Artificial Sequence
<220>
   <223> siRNA, sense, 19 bp core sequence without 3' overhang
<400> 477
   cgtacagcct ggtggtgaa 19
<210> 478
   <211> 19
   <212> DNA
   <213> Artificial Sequence
<220>
   <223> siRNA, sense, 19 bp core sequence without 3' overhang
<400> 478
   gcggcatctt ctggaacta 19
<210> 479
   <211> 19
   <212> DNA
   <213> Artificial Sequence
<220>
   <223> siRNA, sense, 19 bp core sequence without 3' overhang
<400> 479
   gaatgatctt gacccaaga 19
<210> 480
   <211> 19
   <212> DNA
   <213> Artificial Sequence
<220>
   <223> siRNA, sense, 19 bp core sequence without 3' overhang
<400> 480
   ccctaggtct gaaaggaaa 19
<210> 481
   <211> 19
   <212> DNA
   <213> Artificial Sequence
<220>
   <223> siRNA, sense, 19 bp core sequence without 3' overhang
<400> 481
   gggtctggtt ggaatgaca 19
<210> 482
   <211> 19
   <212> DNA
   <213> Artificial Sequence
<220>
   <223> siRNA, sense, 19 bp core sequence without 3' overhang
<400> 482
   cggtaccggt caaacagaa 19
<210> 483
   <211> 19
   <212> DNA
   <213> Artificial Sequence
<220>
   <223> siRNA, sense, 19 bp core sequence without 3' overhang
<400> 483
   gtaaacatgt gggatagaa 19
<210> 484
   <211> 19
   <212> DNA
   <213> Artificial Sequence
<220>
   <223> siRNA, sense, 19 bp core sequence without 3' overhang
<400> 484
   ggtggataat ggcacagca 19
<210> 485
   <211> 19
   <212> DNA
   <213> Artificial Sequence
<220>
   <223> siRNA, sense, 19 bp core sequence without 3' overhang
<400> 485
   gaataatgct gcttcccaa 19
<210> 486
   <211> 19
   <212> DNA
   <213> Artificial Sequence
<220>
   <223> siRNA, sense, 19 bp core sequence without 3' overhang
<400> 486
   gaaagcacct cctcctgca 19
<210> 487
   <211> 19
   <212> DNA
   <213> Artificial Sequence
<220>
   <223> siRNA, sense, 19 bp core sequence without 3' overhang
<400> 487
   gatgaggcct ggatcatga 19
<210> 488
   <211> 19
   <212> DNA
   <213> Artificial Sequence
<220>
   <223> siRNA, sense, 19 bp core sequence without 3' overhang
<400> 488
   gaggaggcct tgaagagta 19
<210> 489
   <211> 19
   <212> DNA
   <213> Artificial Sequence
<220>
   <223> siRNA, sense, 19 bp core sequence without 3' overhang
<400> 489
   gcccgccaat ctccaaaga 19
<210> 490
   <211> 19
   <212> DNA
   <213> Artificial Sequence
<220>
   <223> siRNA, sense, 19 bp core sequence without 3' overhang
<400> 490
   gcagcaagtt gcgcgcaca 19
<210> 491
   <211> 19
   <212> DNA
   <213> Artificial Sequence
<220>
   <223> siRNA, sense, 19 bp core sequence without 3' overhang
<400> 491
   ccggtggctt gtcggtgaa 19
<210> 492
   <211> 19
   <212> DNA
   <213> Artificial Sequence
<220>
   <223> siRNA, sense, 19 bp core sequence without 3' overhang
<400> 492
   gcatggtgct atccctgga 19
<210> 493
   <211> 19
   <212> DNA
   <213> Artificial Sequence
<220>
   <223> siRNA, sense, 19 bp core sequence without 3' overhang
<400> 493
   ggtacgattt aatccagaa 19
<210> 494
   <211> 19
   <212> DNA
   <213> Artificial Sequence
<220>
   <223> siRNA, sense, 19 bp core sequence without 3' overhang
<400> 494
   cctcaagagt ggaggtaaa 19
<210> 495
   <211> 19
   <212> DNA
   <213> Artificial Sequence
<220>
   <223> siRNA, sense, 19 bp core sequence without 3' overhang
<400> 495
   gaggccacct ccagggtta 19
<210> 496
   <211> 19
   <212> DNA
   <213> Artificial Sequence
<220>
   <223> siRNA, sense, 19 bp core sequence without 3' overhang
<400> 496
   gctggctcgt tcttcgaaa 19
<210> 497
   <211> 19
   <212> DNA
   <213> Artificial Sequence
<220>
   <223> siRNA, sense, 19 bp core sequence without 3' overhang
<400> 497
   ggcctcttat cacattcca 19
<210> 498
   <211> 19
   <212> DNA
   <213> Artificial Sequence
<220>
   <223> siRNA, sense, 19 bp core sequence without 3' overhang
<400> 498
   ccacctgaat ctgactcaa 19
<210> 499
   <211> 19
   <212> DNA
   <213> Artificial Sequence
<220>
   <223> siRNA, sense, 19 bp core sequence without 3' overhang
<400> 499
   gcaatgctgt ggtccggta 19
<210> 500
   <211> 19
   <212> DNA
   <213> Artificial Sequence
<220>
   <223> siRNA, sense, 19 bp core sequence without 3' overhang
<400> 500
   gcccagaagt ctctgcaca 19
<210> 501
   <211> 19
   <212> DNA
   <213> Artificial Sequence
<220>
   <223> siRNA, sense, 19 bp core sequence without 3' overhang
<400> 501
   cgagttcgct ggtgaagaa 19
<210> 502
   <211> 19
   <212> DNA
   <213> Artificial Sequence
<220>
   <223> siRNA, reverse complement antisense, 19 bp core sequence without 3' overhang
<400> 502
   tcatgtccaa gtacaggcc 19
<210> 503
   <211> 19
   <212> DNA
   <213> Artificial Sequence
<220>
   <223> siRNA, reverse complement antisense, 19 bp core sequence without 3' overhang
<400> 503
   tcttgtggca gcaagtttc 19
<210> 504
   <211> 19
   <212> DNA
   <213> Artificial Sequence
<220>
   <223> siRNA, reverse complement antisense, 19 bp core sequence without 3' overhang
<400> 504
   tgcgctgaaa gacacattc 19
<210> 505
   <211> 19
   <212> DNA
   <213> Artificial Sequence
<220>
   <223> siRNA, reverse complement antisense, 19 bp core sequence without 3' overhang
<400> 505
   tggcagtcca tttgtctgc 19
<210> 506
   <211> 19
   <212> DNA
   <213> Artificial Sequence
<220>
   <223> siRNA, reverse complement antisense, 19 bp core sequence without 3' overhang
<400> 506
   tattccttca gcactgccc 19
<210> 507
   <211> 19
   <212> DNA
   <213> Artificial Sequence
<220>
   <223> siRNA, reverse complement antisense, 19 bp core sequence without 3' overhang
<400> 507
   ttcaccacca ggctgtacg 19
<210> 508
   <211> 19
   <212> DNA
   <213> Artificial Sequence
<220>
   <223> siRNA, reverse complement antisense, 19 bp core sequence without 3' overhang
<400> 508
   tagttccaga agatgccgc 19
<210> 509
   <211> 19
   <212> DNA
   <213> Artificial Sequence
<220>
   <223> siRNA, reverse complement antisense, 19 bp core sequence without 3' overhang
<400> 509
   tcttgggtca agatcattc 19
<210> 510
   <211> 19
   <212> DNA
   <213> Artificial Sequence
<220>
   <223> siRNA, reverse complement antisense, 19 bp core sequence without 3' overhang
<400> 510
   tttcctttca gacctaggg 19
<210> 511
   <211> 19
   <212> DNA
   <213> Artificial Sequence
<220>
   <223> siRNA, reverse complement antisense, 19 bp core sequence without 3' overhang
<400> 511
   tgtcattcca accagaccc 19
<210> 512
   <211> 19
   <212> DNA
   <213> Artificial Sequence
<220>
   <223> siRNA, reverse complement antisense, 19 bp core sequence without 3' overhang
<400> 512
   ttctgtttga ccggtaccg 19
<210> 513
   <211> 19
   <212> DNA
   <213> Artificial Sequence
<220>
   <223> siRNA, reverse complement antisense, 19 bp core sequence without 3' overhang
<400> 513
   ttctatccca catgtttac 19
<210> 514
   <211> 19
   <212> DNA
   <213> Artificial Sequence
<220>
   <223> siRNA, reverse complement antisense, 19 bp core sequence without 3' overhang
<400> 514
   tgctgtgcca ttatccacc 19
<210> 515
   <211> 19
   <212> DNA
   <213> Artificial Sequence
<220>
   <223> siRNA, reverse complement antisense, 19 bp core sequence without 3' overhang
<400> 515
   ttgggaagca gcattattc 19
<210> 516
   <211> 19
   <212> DNA
   <213> Artificial Sequence
<220>
   <223> siRNA, reverse complement antisense, 19 bp core sequence without 3' overhang
<400> 516
   tgcaggagga ggtgctttc 19
<210> 517
   <211> 19
   <212> DNA
   <213> Artificial Sequence
<220>
   <223> siRNA, reverse complement antisense, 19 bp core sequence without 3' overhang
<400> 517
   tcatgatcca ggcctcatc 19
<210> 518
   <211> 19
   <212> DNA
   <213> Artificial Sequence
<220>
   <223> siRNA, reverse complement antisense, 19 bp core sequence without 3' overhang
<400> 518
   tactcttcaa ggcctcctc 19
<210> 519
   <211> 19
   <212> DNA
   <213> Artificial Sequence
<220>
   <223> siRNA, reverse complement antisense, 19 bp core sequence without 3' overhang
<400> 519
   tctttggaga ttggcgggc 19
<210> 520
   <211> 19
   <212> DNA
   <213> Artificial Sequence
<220>
   <223> siRNA, reverse complement antisense, 19 bp core sequence without 3' overhang
<400> 520
   tgtgcgcgca acttgctgc 19
<210> 521
   <211> 19
   <212> DNA
   <213> Artificial Sequence
<220>
   <223> siRNA, reverse complement antisense, 19 bp core sequence without 3' overhang
<400> 521
   ttcaccgaca agccaccgg 19
<210> 522
   <211> 19
   <212> DNA
   <213> Artificial Sequence
<220>
   <223> siRNA, reverse complement antisense, 19 bp core sequence without 3' overhang
<400> 522
   tccagggata gcaccatgc 19
<210> 523
   <211> 19
   <212> DNA
   <213> Artificial Sequence
<220>
   <223> siRNA, reverse complement antisense, 19 bp core sequence without 3' overhang
<400> 523
   ttctggatta aatcgtacc 19
<210> 524
   <211> 19
   <212> DNA
   <213> Artificial Sequence
<220>
   <223> siRNA, reverse complement antisense, 19 bp core sequence without 3' overhang
<400> 524
   tttacctcca ctcttgagg 19
<210> 525
   <211> 19
   <212> DNA
   <213> Artificial Sequence
<220>
   <223> siRNA, reverse complement antisense, 19 bp core sequence without 3' overhang
<400> 525
   taaccctgga ggtggcctc 19
<210> 526
   <211> 19
   <212> DNA
   <213> Artificial Sequence
<220>
   <223> siRNA, reverse complement antisense, 19 bp core sequence without 3' overhang
<400> 526
   tttcgaagaa cgagccagc 19
<210> 527
   <211> 19
   <212> DNA
   <213> Artificial Sequence
<220>
   <223> siRNA, reverse complement antisense, 19 bp core sequence without 3' overhang
<400> 527
   tggaatgtga taagaggcc 19
<210> 528
   <211> 19
   <212> DNA
   <213> Artificial Sequence
<220>
   <223> siRNA, reverse complement antisense, 19 bp core sequence without 3' overhang
<400> 528
   ttgagtcaga ttcaggtgg 19
<210> 529
   <211> 19
   <212> DNA
   <213> Artificial Sequence
<220>
   <223> siRNA, reverse complement antisense, 19 bp core sequence without 3' overhang
<400> 529
   taccggacca cagcattgc 19
<210> 530
   <211> 19
   <212> DNA
   <213> Artificial Sequence
<220>
   <223> siRNA, reverse complement antisense, 19 bp core sequence without 3' overhang
<400> 530
   tgtgcagaga cttctgggc 19
<210> 531
   <211> 19
   <212> DNA
   <213> Artificial Sequence
<220>
   <223> siRNA, reverse complement antisense, 19 bp core sequence without 3' overhang
<400> 531
   ttcttcacca gcgaactcg 19
<210> 532
   <211> 21
   <212> DNA
   <213> Artificial Sequence
<220>
   <223> siRNA, negative control (ctrl.), sense
<400> 532
   uugucuugca uucgacuaau t 21
<210> 533
   <211> 21
   <212> DNA
   <213> Artificial Sequence
<220>
   <223> siRNA, negative control (ctrl.), reverse complement antisense
<400> 533
   uuagucgaau gcaagacaau t 21
<210> 534
   <211> 21
   <212> DNA
   <213> Artificial Sequence
<220>
   <223> siRNA, first control for TNRC6A (siRNA A1), sense
<400> 534
   uaaugccaag cgagcuacau t 21
<210> 535
   <211> 21
   <212> DNA
   <213> Artificial Sequence
<220>
   <223> siRNA, first control for TNRC6A (siRNA A1), reverse complement antisense
<400> 535
   uguagcucgc uuggcauuau t 21
<210> 536
   <211> 21
   <212> DNA
   <213> Artificial Sequence
<220>
   <223> siRNA, second control for TNRC6A (siRNA A2), sense
<400> 536
   uauaguacug cacugaauau t 21
<210> 537
   <211> 21
   <212> DNA
   <213> Artificial Sequence
<220>
   <223> siRNA, second control for TNRC6A (siRNA A2), reverse complement antisense
<400> 537
   uauucagugc aguacuauau t 21
<210> 538
   <211> 21
   <212> DNA
   <213> Artificial Sequence
<220>
   <223> siRNA, first control for TNRC6B (siRNA B1), sense
<400> 538
   ggagugccau ggaaagguau t 21
<210> 539
   <211> 21
   <212> DNA
   <213> Artificial Sequence
<220>
   <223> siRNA, first control for TNRC6B (siRNA B1), reverse complement antisense
<400> 539
   uaccuuucca uggcacuccu t 21
<210> 540
   <211> 21
   <212> DNA
   <213> Artificial Sequence
<220>
   <223> siRNA, second control for TNRC6B (siRNA B2), sense
<400> 540
   ggaaguuguu gcuaagaaau t 21
<210> 541
   <211> 21
   <212> DNA
   <213> Artificial Sequence
<220>
   <223> siRNA, second control for TNRC6B (siRNA B2), reverse complement antisense
<400> 541
   uuucuuagca acaacuuccu t 21
<210> 542
   <211> 21
   <212> DNA
   <213> Artificial Sequence
<220>
   <223> siRNA, first control for TNRC6C (siRNA C1), sense
<400> 542
   caauggcguu gguaauaucu t 21
<210> 543
   <211> 21
   <212> DNA
   <213> Artificial Sequence
<220>
   <223> siRNA, first control for TNRC6C (siRNA C1), reverse complement antisense
<400> 543
   gauauuacca acgccauugu t 21
<210> 544
   <211> 21
   <212> DNA
   <213> Artificial Sequence
<220>
   <223> siRNA, second control for TNRC6C (siRNA C2), sense
<400> 544
   caauaugaau cuugaucagu t 21
<210> 545
   <211> 21
   <212> DNA
   <213> Artificial Sequence
<220>
   <223> siRNA, second control for TNRC6C (siRNA C2), reverse complement antisense
<400> 545
   cugaucaaga uucauauugu t 21
<210> 546
   <211> 130
   <212> PRT
   <213> Artificial Sequence
<220>
   <223> RNase T1
<400> 546
   Met Met Tyr Ser Lys Leu Leu Thr Leu Thr Thr Leu Leu Leu Pro Thr
   1               5                   10                  15
   Ala Leu Ala Leu Pro Ser Leu Val Glu Arg Ala Cys Asp Tyr Thr Cys
               20                  25                  30
   Gly Ser Asn Cys Tyr Ser Ser Ser Asp Val Ser Thr Ala Gln Ala Ala
           35                  40                  45
   Gly Tyr Gln Leu His Glu Asp Gly Glu Thr Val Gly Ser Asn Ser Tyr
       50                  55                  60
   Pro His Lys Tyr Asn Asn Tyr Glu Gly Phe Asp Phe Ser Val Ser Ser
   65                  70                  75                  80
   Pro Tyr Tyr Glu Trp Pro Ile Leu Ser Ser Gly Asp Val Tyr Ser Gly
                   85                  90                  95
   Gly Ser Pro Gly Ala Asp Arg Val Val Phe Asn Glu Asn Asn Gln Leu
               100                 105                 110
   Ala Gly Val Ile Thr His Thr Gly Ala Ser Gly Asn Asn Phe Val Glu
           115                 120                 125
   Cys Thr
       130